# EUROPEAN PATENT APPLICATION

(11) **EP 4 752 892 A1**
(43) Date of publication of application: **03.06.2026**
(21) Application number: 25218817.2
(22) Date of filing: 26.11.2025
(51) Int. Cl.: G16H 10/20, G16H 50/20

(54) **SYSTEMS AND METHODS FOR PROVIDING RESPONSIVE MEDICAL DATA USING TASK-SPECIFIC ORCHESTRATIONS**

(30) Priority: 27.11.2024 US 202463725890 P; 20.01.2025 US 202563747211 P
(71) Applicant: Tempus AI, Inc., Chicago, IL 60654-2282 (US)
(72) Inventor: Banaszak, Daniel Max, Chicago / IL, 60654 (US); Deng, Sarah Lu, Chicago / IL, 60654 (US); Lefkofsky, Eric P., Chicago / IL, 60654 (US)
(74) Representative: Grund, Martin

(57) **Abstract**

This application describes, amongst other things, an example method for responding to user queries. The method includes receiving, via a user interface, a user query from a user and categorizing the user query using a first task-specific orchestration. The method also includes providing information about the user query to a destination task-specific orchestration, including, in accordance with categorizing the user query in a first category, assigning a second task-specific orchestration as the destination task-specific orchestration; and, in accordance with categorizing the user query in a second category, assigning a third task-specific orchestration as the destination task-specific orchestration. The method further includes, in response to providing the information about the user query, receiving an output from the destination task-specific orchestration; and providing a response to the user query via the user interface based on the output from the destination task-specific orchestration.

## Description

### PRIORITY AND RELATED APPLICATIONS

This application claims priority to U.S. Provisional Patent Application No. 63/725,890 filed November 27, 2024, and to U.S. Provisional Patent Application No. 63/747,211 filed January 20, 2025, each of which is hereby incorporated by reference in its entirety.

### TECHNICAL FIELD

The present disclosure relates to machine-learning systems for healthcare applications, and more particularly to agent architectures that use task-specific orchestrations to respond to patient queries and provide responsive medical information.

### BACKGROUND

Healthcare systems generate vast amounts of medical data, including electronic health records, diagnostic images, laboratory results, treatment histories, and patient-reported information. This data exists across multiple sources, modalities, and formats, making it challenging for patients and healthcare providers to access, understand, and utilize it effectively. Patients often struggle to navigate complex medical information, coordinate care between multiple providers, and make informed decisions about their health.

Users of medical patient care systems often have complex questions about medical conditions, care, treatment, medications, and/or adverse effects. There are large amounts of data that could be applied to answer questions about patients' medical care. However, the users may not know which of the many potential data sources are relevant to the specific questions that they are asking or how to properly query each source.

Additionally, the burden often falls on patients to understand their health conditions and advocate for their care. For those dealing with serious or chronic conditions, the journey often involves seeing many different doctors, managing frequent appointments, and trying to make sense of various tests. This process is complex, and patients are often unable to acquire medical information from their records, coordinate between care providers, or interpret text results.

### SUMMARY

Thus, the inventors of the present application recognized a need for systems and methods that allow a user to query subject data, such as patient and medical records, using natural language and intuitive interfaces. The disclosed systems can integrate directly with various healthcare systems to upload, organize, and manage health records in one convenient place. The disclosed embodiments include systems and user interfaces for generating interactive health profiles. For example, a singular personal profile that aggregates and structures health information and can be downloaded and shared with clinicians. The disclosed embodiments also include systems and user interfaces to assist with preparing for upcoming events and appointments. For example, compiling relevant health information, documents, and key questions that can help facilitate more productive conversations. The disclosed embodiments also include systems and user interfaces for monitoring patient health. For example, tracking symptoms, sleep, medications taken, and highlighting key trends while organizing important notes and reminders in a patient health journal. The present disclosure describes a generative AI-enabled assistant that allows users to ask questions and receive real-time, personalized information and insights by searching and summarizing medical records and health information, and answering questions regarding current medical guidelines and information about care. This allows users to learn about their conditions, treatments, and clinical trial options in an easy and digestible format. In this way, the disclosed systems provide improved feedback to the user, reduce the number of inputs needed to perform the operations, provide additional control options (e.g., without cluttering the user interface with displayed controls), and perform operations automatically without requiring additional user inputs.

In accordance with some embodiments, a method includes (i) receiving, via a user interface, a user query from a user; (ii) categorizing the user query using a first task-specific orchestration, where the first task-specific orchestration is configured to categorize user queries; (iii) providing information about the user query to a destination task-specific orchestration, including: (a) in accordance with categorizing the user query in a first category, assigning a second task-specific orchestration as the destination task-specific orchestration; and (b) in accordance with categorizing the user query in a second category, assigning a third task-specific orchestration as the destination task-specific orchestration; (iv) in response to providing the information about the user query, receiving an output from the destination task-specific orchestration; and (v) providing a response to the user query via the user interface based on the output from the destination task-specific orchestration. Benefits of the example method include improving query processing efficiency through intelligent routing, reducing computational overhead by utilizing specialized orchestrations for specific query types, enhancing response accuracy through domain-specific processing, and a scalable architecture that allows for dynamic addition of new orchestrations without system redesign.

In accordance with some embodiments, a method includes (i) receiving a subject identifier; (ii) monitoring, using the subject identifier, medical information for the subject, where the medical information is updated over time; (iii) identifying an abnormality or risk for the subject based on the medical information; and (iv) in response to identifying the abnormality or risk, initiating a remedial action. Benefits of the example method include automating continuous health monitoring to reduce manual oversight requirements, early detection capabilities that enable proactive intervention before conditions worsen, real-time data processing that ensures timely identification of health risks, and automated remedial action initiation that reduces response time and improves patient outcomes.

In accordance with some embodiments, a computing system is provided, such as a cloud computing system, a server system, a personal computer system, and/or other type of electronic device. The computing system includes control circuitry and memory storing one or more sets of instructions. The one or more sets of instructions include instructions for performing any of the methods described herein. Such a computing system can provide distributed processing capabilities that enable scalable deployment across multiple environments, modular architecture that supports flexible configuration and customization of task-specific orchestrations, efficient resource utilization through specialized processing components, and robust data handling capabilities that ensure secure and reliable medical information processing.

In accordance with some embodiments, a non-transitory computer-readable storage medium is provided. The non-transitory computer-readable storage medium stores one or more sets of instructions for execution by a computing system. The one or more sets of instructions include instructions for performing any of the methods described herein.

Thus, devices and systems are disclosed with methods for receiving and responding to user prompts and queries as well as identifying and responding to abnormalities and risks. Such methods, devices, and systems may complement or replace conventional methods, devices, and systems for receiving and responding to user prompts and queries as well as identifying and responding to abnormalities and risks.

The features and advantages described in the specification are not necessarily all inclusive and, in particular, some additional features and advantages will be apparent to one of ordinary skill in the art in view of the drawings, specification, and claims provided in this disclosure. Moreover, it should be noted that the language used in the specification has been principally selected for readability and instructional purposes and has not necessarily been selected to delineate or circumscribe the subject matter described herein.

### BRIEF DESCRIPTION OF THE DRAWINGS

So that the present disclosure can be understood in greater detail, a more particular description can be had by reference to the features of various embodiments, some of which are illustrated in the appended drawings. The appended drawings, however, merely illustrate pertinent features of the present disclosure and are therefore not necessarily to be considered limiting, for the description can admit to other effective features as the person of skill in this art will appreciate upon reading this disclosure.
Figure ("FIG.") 1 is a block diagram illustrating an example platform in accordance with some embodiments.
Figures 2A and 2B are block diagrams illustrating an example client device in accordance with some embodiments.
Figure 2C illustrates examples of various logic functions that are implemented in accordance with some embodiments.
Figure 3A is a block diagram illustrating an example server system in accordance with some embodiments.
Figure 3B is a block diagram illustrating example databases in accordance with some embodiments.
Figure 4 illustrates an example architecture for deploying agents in accordance with some embodiments.
Figure 5A illustrates an example process for data importation and query processing in accordance with some embodiments.
Figure 5B illustrates an example architecture for responding to user queries in accordance with some embodiments.
Figure 5C illustrates another example architecture for responding to user queries in accordance with some embodiments.
Figures 6A-6AF illustrate example user interfaces and interactions for obtaining, analyzing, and performing actions based on subject data in accordance with some embodiments.
Figure 7 is a flow diagram illustrating an example method of responding to user queries in accordance with some embodiments.
Figure 8 is a flow diagram illustrating an example method of identifying and responding to abnormalities and risks in accordance with some embodiments.
Figures 9A-9C illustrate an example architecture for an AI assistant in accordance with some embodiments.
Figures 10A-10B illustrate an example architecture for a synthesis orchestration in accordance with some embodiments.

In accordance with common practice, the various features illustrated in the drawings are not necessarily drawn to scale, and like reference numerals can be used to denote like features throughout the specification and figures.

### DETAILED DESCRIPTION

The present disclosure describes, among other things, a platform for using task-specific orchestrations (e.g., task-specific agents) that include task-specific machine-learning models (e.g., language models, transformer models, diffusion models, and other types of models) for specific tasks and/or within specific domains as well as multi-modal models for tasks involving multiple modalities of data. The platform may include a plurality of individual task-specific orchestrations that may operate independently or in combination to return accurate and relevant information (e.g., identifying target cohorts, clinical trial information, and/or members of target populations). In some embodiments, the platform includes a plurality of modality-specific orchestrations (e.g., each configured to summarize a corresponding modality of data) and one or more multi-modal orchestration (e.g., configured to intake and analyze multiple modalities of data). In some embodiments, each orchestration (or agent) may include one or more machine-learning models, such as a language model trained and/or fine-tuned on a particular domain. The platform may also include one or more composite orchestrations (e.g., composite agents) that give instructions to, and combine results from, a plurality of task-specific orchestrations configured for different tasks.

In some embodiments, the platform acts as an operating system for implementing orchestrations to perform various clinical tasks. The platform may include one or more of the following example components. For example, a genetic sequencing component with downstream molecular bioinformatics may operate to call out relevant biomarkers in DNA, RNA, or their derivatives for a specimen (e.g., a tumor biopsy) that is sequenced and reported back to an ordering physician. As another example, a pathology imaging component may operate on cellular and/or slide level images to identify relevant biomarkers from cells within imaged specimen. As another example, a radiological imaging component may operate on larger images of the body through various radiology imaging technologies to identify the presence or longitudinal progression of tumors. Other examples include identifying various disease states using cardiology, neurology, and/or endocrinology imaging components. Each of these components may include, or communicate with, a corresponding agent to identify and/or report information relevant to a user query or request.

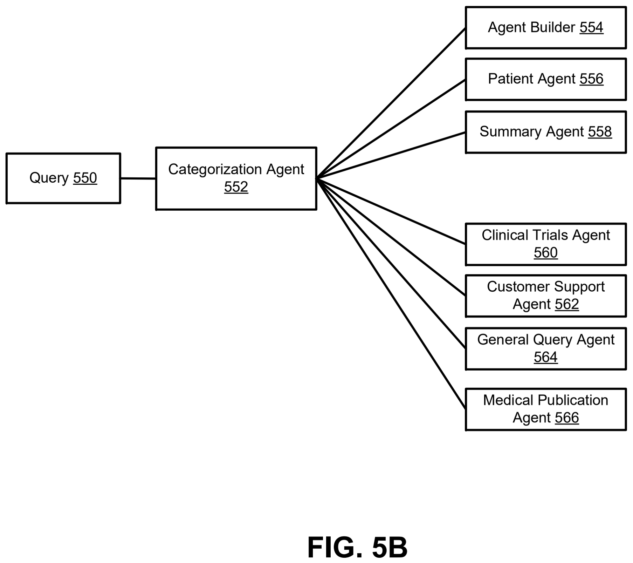

As an example, when a patient asks "What clinical trials are available for my condition?", the system's categorization orchestration may analyze the query and determine that it relates to clinical trial information. The system may then route this query to a specialized clinical trials orchestration (e.g., the clinical trials agent 560 shown in Figure 5B) rather than a general medical query orchestration. This targeted routing allows the clinical trials orchestration to access specific trial databases and generate a response tailored to the patient's medical profile, providing relevant trial options with inclusion criteria and contact information. This intelligent routing approach provides several potential advantages. For example, the system can direct queries to orchestrations specifically trained on particular data and query types, resulting in more accurate responses than a generalized system (e.g., more quickly and efficiently produced as well). The modular architecture allows users/organizations to add new specialized orchestrations for emerging domains, such as genomics or telemedicine, without disrupting existing functionality. Additionally, computational resources are used more efficiently since each orchestration only needs to maintain expertise in its specific domain rather than attempting to handle all possible domains.

In another example, the system continuously monitors a patient's electronic health records and laboratory results. In this way, when new test results show potential issues (e.g., elevated blood glucose levels that exceed normal ranges), the monitoring system may automatically identify the potential issues. The system may then initiate remedial actions such as sending an alert to the patient's healthcare provider, scheduling a follow-up appointment, and providing the patient with educational materials about issue management. This proactive approach reduces man-machine interactions, reduces the cognitive burden on patients, and enables early intervention before the condition progresses to more serious complications.

In another example, an orchestration (agent) may be configured by a user using a user interface (e.g., a console of a web or desktop application) and deployed to various environments (e.g., a research environment, an alpha environment, a beta environment, a client environment, and/or a production environment). Each environment may be linked to different sources, have different permissions, and/or have different authorized users. In some embodiments, precision medicine principles are employed in customizing the user interfaces, such as modifications based on a set of subjects (e.g., patients) associated with the user of the application. For example, the user (or an immediate family member of the user) may be one of the subjects. An environment may be defined by access to data sources and/or users. The agent configuration may be stored in a control plane. The control plane may be configured to control how data is managed, routed, and/or processed. The agents themselves may execute in the appropriate workload planes (e.g., data planes), and the workload planes may not have access to the control plane. The control plane may supervise/direct each workload plane, while the workload planes are configured to manipulate and/or transport data.

As another example, an agent builder in the control plane may be configured to push configurations into the various environments. For example, this synchronization may be fast enough that a user can configure an agent and immediately evaluate the configuration in the interactive console in a working environment. An example architecture includes two components: an agent builder in a control plane that hosts the user interface (UI) for configuring agents, and an agent host in a workload plane that hosts the UI and API for interacting with deployed agents. When an agent configuration is changed or an agent version is deployed, the agent builder may inform the agent host in each environment so that the updated agent can be deployed. For example, this may be via a pubsub message to the agent-config topic or via a simple HTTP request. In some embodiments, the agent builder utilizes a cognitive architecture that includes memory modules and action spaces. For example, the cognitive architecture organizes agents along three dimensions: their information storage (e.g., divided into working and long-term memories); their action space (e.g., divided into internal and external actions); and their decision-making procedure (e.g., structured as an interactive loop with planning and execution).

As another example, after deployment, an agent may receive a user query (e.g., requesting information about clinical trials), generate a structured application programming interface (API) call, use the generated API call to query a remote server to retrieve a relevant result, and reformat the relevant information to return to the user. In some embodiments, each action is performed by a different agent builder block component (also sometimes referred to as a builder block, block, or node). In some embodiments, the agent is configured for multiple types of tasks. In these embodiments, the agent may identify the intent of a user's query (e.g., to search for clinical trials or identify adverse events) and respond accordingly. In some embodiments, the agent is configured for only one type of task (e.g., is a task-specific agent). In some of these embodiments, the agent does not identify an intent of the user (e.g., the agent may assume the intent). In some embodiments, the agent receives the intent from a different component or system. The agent may also interface with other agents to obtain additional information for the user query (such as patient records or relevant guidelines). In some embodiments, the agent includes a pretrained language model (e.g., trained on a particular domain and/or using particular databases). In some embodiments, the agent queries an unstructured database (e.g., in addition, or alternatively, to generating the API call).

The platform, or components thereof, may be used in conjunction with any medical field (e.g., to assist physicians in the treatment of any associated disease state therein), such as on oncology, endocrinology (e.g., diabetes), neurology, mental health (e.g., depression and related pharmacogenetics), and cardiovascular disease. For example, the platform may also include a cardiology-based component (e.g., comprising one or more agents) that operates on electrocardiogram (ECG) data to identify patients having an elevated risk for cardiovascular disease. As another example, the platform may include a data curation component (e.g., comprising one or more agents) that obtains raw (e.g., unstructured) data and structures it into a common and useful format as a repository (e.g., a multimodal database) of clinical data from which other bioinformatics, analytics, agents, models, and/or components may operate. As another example, the platform may be configured to search within the clinical data to identify cohorts of related patients and/or generate insights and/or analytics. As another example, the platform may be configured to monitor an electronic health record (EHR) to identify care gaps and/or reminders to physicians to act with a respective patient. In this way, the platform may serve as a docket manager that identifies issues/events the corresponding physicians did not manually docket, e.g., to ensure patients and other subjects get timely care. The platform may also be configured to track and/or catalog relevant therapies (e.g., on label and/or off label use) for a set of disease states. The platform may also track and/or catalog relevant clinical trials (e.g., in multiple countries and/or from multiple authorities) for a set of disease states. In some embodiments, the platform is further configured to interact with patients/subjects directly.

As discussed below, the platform may include an AI-enabled assistive user interface (which may sometimes be described herein as a clinical assistant or digital assistant) that provides access to patient insights. The AI-enabled assistive user interface may use one or more of the orchestrations described herein, each of which may include ML models and/or other types of machine learning.

In some embodiments, the platform includes a hub component that allows physicians to order, track, and view test results, and export patient data. In some embodiments the hub component provides insights into genomic alterations, treatment implications, as well as clinical trial matching. The hub component may be used in conjunction with the AI-enabled clinical assistant to allow physicians to interact using conversational language including natural language inputs, follow-up questions, and remarks. The platform may also include a peer-to-peer messaging component for physicians and other medical experts to share knowledge, insight, and/or perspective on medical fields such as molecular oncology (e.g., as it pertains to patient care). The messaging component may be used in conjunction with the AI-enabled clinical assistant to engage in, and optionally learn from, the conversations on the messaging component. For example, the Al-enabled clinical assistant may be invoked in conversation to provide insights and/or data for a particular topic or conversation. The platform may also include an EHR interface component (e.g., comprising one or more agents) configured to allow physicians, and optionally other users, to view, edit, and/search an EHR. The EHR interface component may be communicatively coupled with one or more services and/or databases to obtain updated information and reports (e.g., via push notifications). The EHR interface component may be used in conjunction with the Al-enabled clinical assistant to search, edit, summarize, and/or reform an EHR. The platform may also include a research analytical component (e.g., comprising one or more agents) that provides de-identified patient/clinical data and insights. For example, the platform may provide insights derived from providing available data and/or newly-ingested data to a machine-learning model (e.g., the insights are output by the model in response to providing the data).

Reference will now be made to embodiments, examples of which are illustrated in the accompanying drawings. In the following description, numerous specific details are set forth in order to provide an understanding of the various described embodiments. However, it will be apparent to one of ordinary skill in the art that the various described embodiments may be practiced without these specific details. In other instances, well-known methods, procedures, components, circuits, and networks have not been described in detail so as not to unnecessarily obscure aspects of the embodiments.

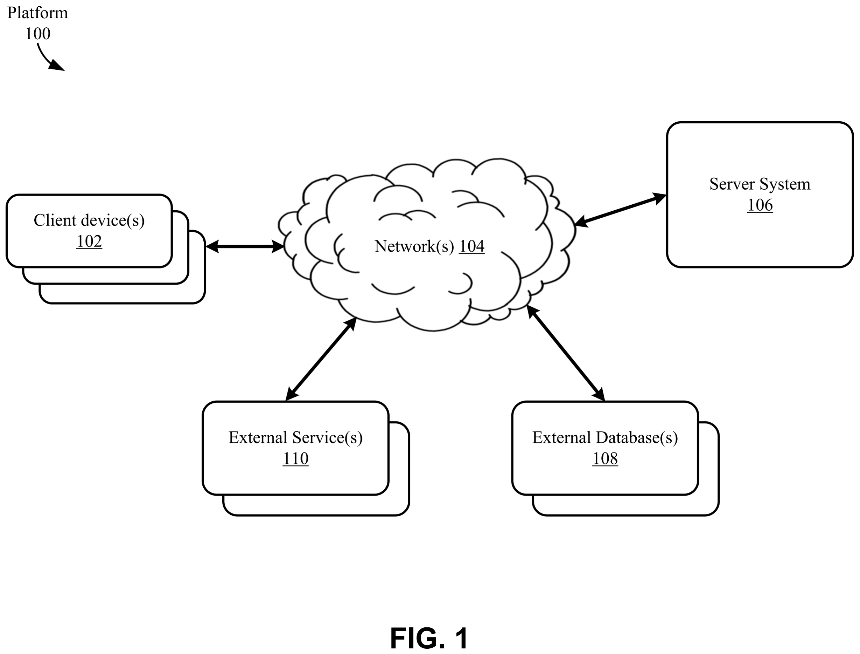

Figure 1 is a block diagram illustrating a platform 100 in accordance with some embodiments. In some embodiments, the platform 100 is an AI platform (e.g., the AI platform discussed previously). The platform 100 includes one or more client devices 102 communicatively coupled to a server system 106 via one or more networks 104. In accordance with some embodiments, the platform 100 further includes, or communicates with, one or more external services 110 and one or more external databases 108. In some embodiments, the one or more networks 104 include public communication networks, private communication networks, or a combination of both public and private communication networks. For example, the one or more networks 104 can be any network (or combination of networks) such as the Internet, other wide area networks (WAN), local area networks (LAN), virtual private networks (VPN), metropolitan area networks (MAN), peer-to-peer networks, and/or ad-hoc connections. In some embodiments, the platform 100 includes only a subset of the components shown in Figure 1. For example, the platform 100 may include only one of: a client device 102 or a server system 106.

In some embodiments, a client device 102 is associated with one or more users. In some embodiments, each user is separately authenticated (e.g., assigned distinct/unique authentication tokens). In some embodiments, a client device 102 is a personal computer, mobile electronic device, wearable computing device, laptop computer, tablet computer, mobile phone, feature phone, smart phone, a speaker, television (TV), and/or any other electronic device capable of interacting with a user (e.g., an electronic device having an I/O interface). The client device(s) 102 may communicatively couple to other components of the platform 100 wirelessly and/or through a wired connection (e.g., directly through an interface, such as an HDMI interface).

In some embodiments, the client device(s) 102 send and receive information, such as documents, queries, and/or results, through network(s) 104. For example, the client device(s) 102 may send a query or request to the server system 106, the external service(s) 110, and/or the external database(s) 108 through network(s) 104. As another example, the client device(s) 102 may receive results and other responses from the server system 106, the external service(s) 110, and/or the external database(s) 108 through network(s) 104. In some embodiments, two or more client devices 102 communicate with one another (e.g., resending and responding to queries and requests). The two or more client devices 102 may communicate via the network(s) 104 or directly (e.g., via a wired connection or through a peer-to-peer wireless connection).

In some embodiments, the server system 106 includes multiple electronic devices communicatively coupled to one another. In some embodiments, the multiple electronic devices are collocated (e.g., in a datacenter), while in other embodiments, the multiple electronic devices are geographically separated from one another. In some embodiments, the server system 106 stores and provides clinical and/or patient data. In some embodiments, the server system 106 trains, publishes, and/or utilities one or more agents and/or language models. In some embodiments, the server system 106 receives and responds to queries and requests from the client device(s) 102 using the one or more agents and/or language models. In some embodiments, the server system 106 includes multiple nodes and/or clusters configured to manage different types of tasks and/or handle requests and queries from different geographical locations.

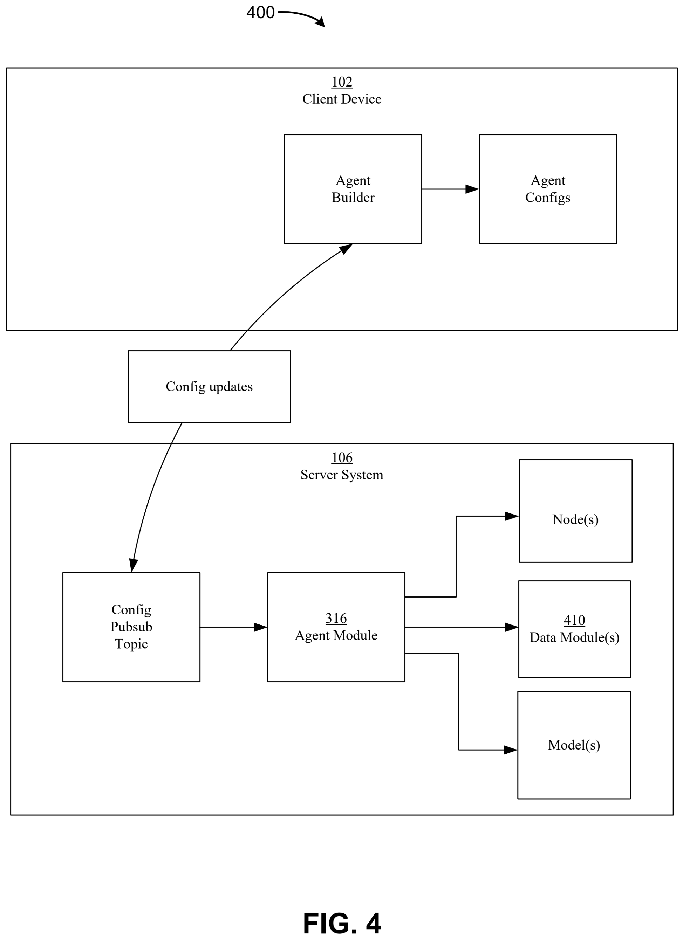

In some embodiments, the client device(s) 102 and/or the server system 106 communicate with the external service(s) 110 and/or the external database(s) 108 via an application programming interface (API). In some embodiments, the external service(s) 110 and/or the external database(s) 108 are maintained/operated by a third party to the platform 100. In some embodiments, the external service(s) 110 include agents, location services, time services, web-enabled services, and/or services that access information stored external to the platform 100. In some embodiments, the external database(s) 108 include one or more medical databases, clinical databases, subject databases, research databases, and/or general knowledge databases. In some embodiments, the external database(s) 108 comprise one or more of the databases shown in Figure 4. In some embodiments, the external database(s) 108 comprise one or more user databases (e.g., patient databases maintained by a third-party user of the platform 100).

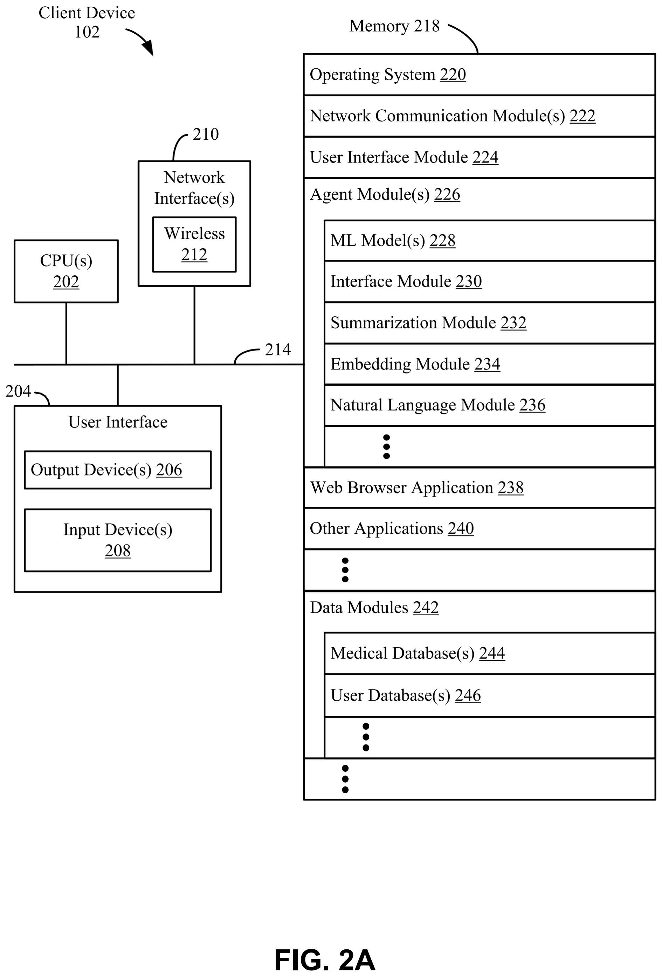

Figure 2A is a block diagram illustrating a client device 102 in accordance with some embodiments. The client device 102 includes one or more central processing units (CPUs) 202, a user interface 204, one or more network (or other communications) interfaces 210, memory 218, and one or more communication buses 214 for interconnecting these components. In some embodiments, the client device 102 includes a processor or other control circuitry (e.g., in addition, or alternatively, to the CPUs 202). For example, the client device 102 may include one or more GPUs and/or DPUs (e.g., for performing machine learning tasks). The communication buses 214 optionally include circuitry (sometimes called a chipset) that interconnects and controls communications between system components. Optionally, the client device 102 includes a location-detection component, such as a global navigation satellite system (GNSS) (e.g., GPS (global positioning system), GLONASS, Galileo, BeiDou) or other geo-location receiver, and/or location-detection software for determining the location of the client device 102.

In some embodiments, the client device 102 includes one or more sensors including, but not limited to, accelerometers, gyroscopes, compasses, magnetometer, light sensors, near field communication transceivers, barometers, humidity sensors, temperature sensors, proximity sensors, range finders, and/or other sensors/devices for sensing and measuring various environmental conditions.

The user interface 204 includes output device(s) 206 and input device(s) 208. In some embodiments, the input device(s) 208 include a keyboard, mouse, a track pad, and/or a touchscreen. In some embodiments, the user interface 204 includes a display device that includes a touch-sensitive surface, in which case the display device is a touch-sensitive display. In client devices that have a touch-sensitive display, a physical keyboard is optional (e.g., a soft keyboard may be displayed when keyboard entry is needed). In some embodiments, the output device(s) 206 include a speaker and/or a connection port for connecting to speakers, earphones, headphones, or other external listening devices. In some embodiments, the input device(s) 208 include a microphone and/or voice recognition device to capture audio (e.g., speech from a user).

In some embodiments, the one or more network interfaces 210 include wireless and/or wired interfaces for receiving data from and/or transmitting data to other client devices 102, the server system 106, and/or other devices or systems. The data communications may be conducted using any of a variety of custom or standard wireless protocols (e.g., NFC, RFID, IEEE 802.15.4, Wi-Fi, ZigBee, 6LoWPAN, Thread, Z-Wave, Bluetooth, ISA100.11a, WirelessHART, MiWi, etc.). Furthermore, the data communications may be conducted using any of a variety of custom or standard wired protocols (e.g., USB, Firewire, Ethernet, etc.). For example, the one or more network interfaces 210 may include a wireless interface 212 for enabling wireless data communications with other client devices 102, systems, and/or other wireless (e.g., Bluetooth-compatible) devices. Furthermore, in some embodiments, the wireless interface 212 (or a different communications interface of the one or more network interfaces 210) enables data communications with other WLAN-compatible devices and/or the server system 106 (via the one or more network(s) 104).

The memory 218 includes high-speed random-access memory, such as DRAM, SRAM, DDR RAM, or other random-access solid-state memory devices; and may include non-volatile memory, such as one or more magnetic disk storage devices, optical disk storage devices, flash memory devices, or other non-volatile solid-state storage devices. The memory 218 optionally includes one or more storage devices remotely located from the CPU(s) 202. The memory 218, or alternately, the non-volatile memory solid-state storage devices within the memory 218, includes a non-transitory computer-readable storage medium. In some embodiments, the memory 218 or the non-transitory computer-readable storage medium of the memory 218 stores the following programs, modules, and data structures, or a subset or superset thereof:
- an operating system 220 that includes procedures for handling various basic system services and for performing hardware-dependent tasks;
- network communication module(s) 222 for connecting the client device 102 to other computing devices connected to one or more network(s) 104 via the one or more network interface(s) 210 (wired or wireless);
- a user interface module 224 that receives commands and/or inputs from a user via the user interface 204 (e.g., from the input device(s) 208) and provides outputs via the user interface 204 (e.g., the output device(s) 206);
- agent module(s) 226 that include a set of agent building blocks and/or generated agents. In some embodiments, the agent module(s) 226 work in conjunction with an agent module at the server system 106 (e.g., the agent module(s) 316). In some embodiments, the agent module(s) 226 includes the following submodules (or sets of instructions), or a subset or superset thereof:
   ∘ model(s) 228 that engage with a user and/or perform specific tasks (e.g., in furtherance of a user request or query). In some embodiments, the model(s) 228 include one or more large language models, such as GPT-3, GPT-4, BioGPT, and PaLM-2, neural networks, transformer models, and/or other types of ML models; and
   ∘ an interface module 230 that allows the model(s) 228 to communicate with other applications, components, and devices (e.g., via an API or structured query). In some embodiments, the interface module 230 is, or includes, an agent (e.g., a task-specific orchestration, a modality-specific orchestration, or a multi-modal orchestration), an orchestration creator application, one or more orchestration libraries (e.g., orchestration marketplaces) for selecting orchestrations for performing tasks as discussed herein;
   ∘ a summarization module 232 that is configured to summarize one or more modalities of data, such as summarizing of a medical visit, annotating and/or labeling images, and/or otherwise summarizing data (e.g., in a human-readable format, such as a natural language summary);
   ∘ an embedding module 234 that is configured to generate embeddings (e.g., vectors) based on input data, such as raw input data and/or summarized input data. In some embodiments, the embedding module 234 is configured to generate modality-specific embeddings. In some embodiments, the embedding module 234 is configured to generate multi-modal embeddings (e.g., by aggregating or combining modality-specific embeddings);
   ∘ a natural language module 236 that is configured to generate natural language (e.g., conversational) outputs. In some embodiments, the natural language module 236 is configured to convert one or more ML outputs into a natural language output. In some embodiments, the natural language module 236 is configured to generate embeddings from natural language inputs (e.g., received from a user via a digital assistant interface);
- a web browser application 238 for accessing, viewing, and interacting with web sites;
- other applications 240, such as applications for word processing, calendaring, mapping, weather, stocks, time keeping, virtual digital assistant, presenting, number crunching (spreadsheets), drawing, instant messaging, e-mail, telephony, video conferencing, photo management, video management, a digital music player, a digital video player, 2D gaming, 3D (e.g., virtual reality) gaming, electronic book reader, and/or workout support; and
- one or more data modules 242 for managing the storage of and/or access to data such as medical data, clinical data, patient data, and user data. In some embodiments, the one or more data modules 242 include:
   ∘ one or more medical databases 244 for storing medical data (e.g., regarding therapies, drugs, treatments, patients, cohorts and/or diseases); and
   ∘ one or more user databases 246 for storing user data such as user preferences, user settings, and other metadata.

In some embodiments, the agent modules 226 are configured to engage with a user in an integrated, conversational manner using natural language dialog, and/or invoke external services when appropriate to obtain information or perform various actions.

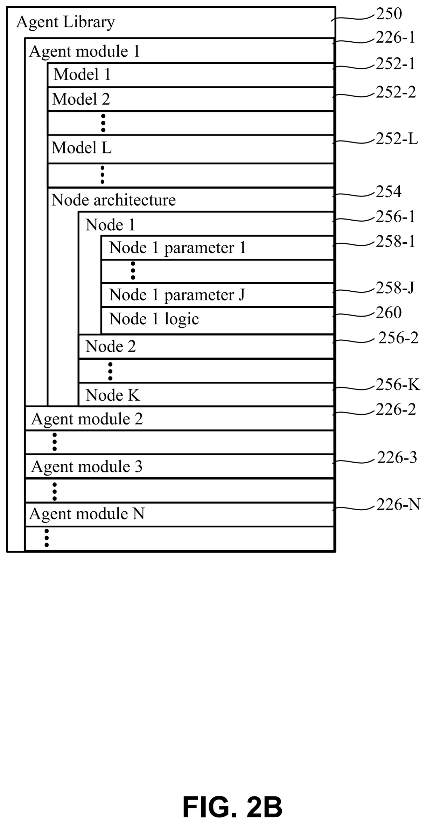

Referring to Figure 2B, in some embodiments, the platform 100 provides an agent library 250 that includes a plurality of agent modules 226 (and/or the agent module(s) 316) and a system for managing and deploying these agent modules, such as through various blocks (e.g., agent builder blocks) realized in the form of one or more nodes 256.

In some embodiments, a respective agent module 226 (or agent module 316) is associated with a defined domain of information and/or a task-specific capability, which allows for retrieving a particular agent module based on information determined from a prompt provided by a user and/or based on a selection of the agent module by the user. In some embodiments, an agent module 226-1 is configured for a first specific task (e.g., generating a summary report of a patient's medical records), a second agent module 226-2 is configured for a second specific-task (e.g., generating a set of embeddings from summary data), a third agent module 226-3 is configured for a third specific-task (e.g., generating patient care guidelines based on a patient's health profile), a fourth agent module is configured for a fourth specific-task (e.g., identifying important dates for a patient based on summary data), a fifth agent module is configured for a fifth specific-task (e.g., identifying changes in a standard of care for a disease setting), a sixth agent module is configured for a sixth specific-task (e.g., evaluating unstructured data associated with a patient to identify a cohort of similar patients), and a seventh agent module is configured for a seventh specific-task (e.g., phenotyping a subject). In some embodiments, the agent library 250 includes N agent modules, where N is a positive integer. In some embodiments, the agent library 250 is stored at one or more client devices 102 and/or the server system 106 (e.g., a first portion of the agent library 250 may be stored at a first client device 102, a second portion of the agent library 250 may be stored at a second client device 102, and a third portion of the agent library 250 may be stored at the server system 106). In some embodiments, each agent module 226 includes a client-side portion and a server-side portion (e.g., a corresponding agent module 316 at the server system 106).

In some embodiments, each agent module 226 provides range of content and functionality that an end-user can engage with and/or configure for such engagement through one or more nodes 256 associated with the agent module 226, from a simple static response to sophisticated knowledge systems that facilitate automated conversations and data analysis leading to solutions and integrated transactions with external systems. Collectively, the one or more nodes 256 form some or all of a node architecture 254 associated with the agent module 226, which defines rules for traversing between nodes. In some embodiments, each respective agent 226 has a corresponding node architecture 254, which provides a one-to-one relationship between agent modules 226 and node architectures 254. In some embodiments, a respective agent module 226 supports the generation of additional agent modules 226 that utilize one or more models 252 and/or nodes 256 of a node architecture 254 of the respective agent module 226 or a different agent module 226. In some embodiments, a respective agent module 226 supports integration with other agent modules 226 in the agent library 250.

In some embodiments, each agent module 226 provides a defined scope for engaging in a workflow. Accordingly, in some embodiments, each agent module 226 is configured to assist end users to either resolve a question and/or problem or to fulfill a specific request for retrieving information, such as through a conversational communications framework. In some embodiments, a first subset of agent modules 226 are task specific and/or modality specific, whereas a second subset of the agent modules 226 are multi-modal and/or configured to perform multiple types of tasks. Some embodiments provide an ability to create, manage, and administer agent modules 226 to make them available for use in creating, editing, or deleting agent modules 226 via a user interface, e.g., by using a user-interface-based agent module builder or the like.

Some embodiments provide a user-interface-based agent module designer to assist in the creation and editing of agent modules 226 and/or a workflow associated with a variety of agent modules 226 (the workflow is also sometimes also referred to as an assembly or orchestration). In some embodiments, this workflow is manifested as a node architecture that includes a plurality of interconnected nodes. In some embodiments, the agent module designer includes the ability to define the name of an agent module 226, create an agent module 226, edit an agent module 226, delete individual nodes 256 associated with an agent module 226, expand and/or collapse node 256 branches, the ability to see and edit the conditional logic for a node 256, and the ability to see node traversals (e.g., when one or more nodes 256 connect to a different node 256).

In some embodiments, a node 256 of an agent module 226 reflects one or more decision points within an agent module 226, such as one or more predetermined decision points. In some embodiments, an agent module 226 evaluates data (e.g., a prompt provided by a user at a client device 102, an output from a different agent module 226, etc.), such as graphical data from a client device 102 by parsing and/or evaluating the incoming data for recognized keywords, phrases, ground truth labels, etc. For example, based on detection of recognized features, an agent module 226 may process information associated with the data received from the client device 102 in a particular direction within the plurality of interconnected nodes 256, such as from a node 256-1 associated with an agent module 226-1 to a node 256-2 associated with the agent module 226-1 and/or from the node 256-1 associated with the agent module 226-1 to a node 256-K associated with the agent module 226-1. Thus, in some embodiments, the use of one or more nodes 256 associated with a respective agent module 226 in a plurality of interconnected nodes 256 is similar to walking through a decision tree, where the different nodes 256 may be associated with different agent modules 226. Each agent module 226 may evaluate information based on associated conditional logic to progress information in the plurality of interconnected nodes 256. However, the present disclosure is not limited thereto. In some embodiments, each node in the plurality of interconnected nodes 256 comprises conditional logic that can evaluate data, retrieve data, generate data, or a combination thereof, e.g., based on an evaluation of information inputted to the respective node 256. In some embodiments, each node in the plurality of interconnected nodes 256 takes some action, such as generating a message and/or sending information to another node 256 in the same agent module 226 as the respective node, or a different node 256 of another agent module 226, or the like.

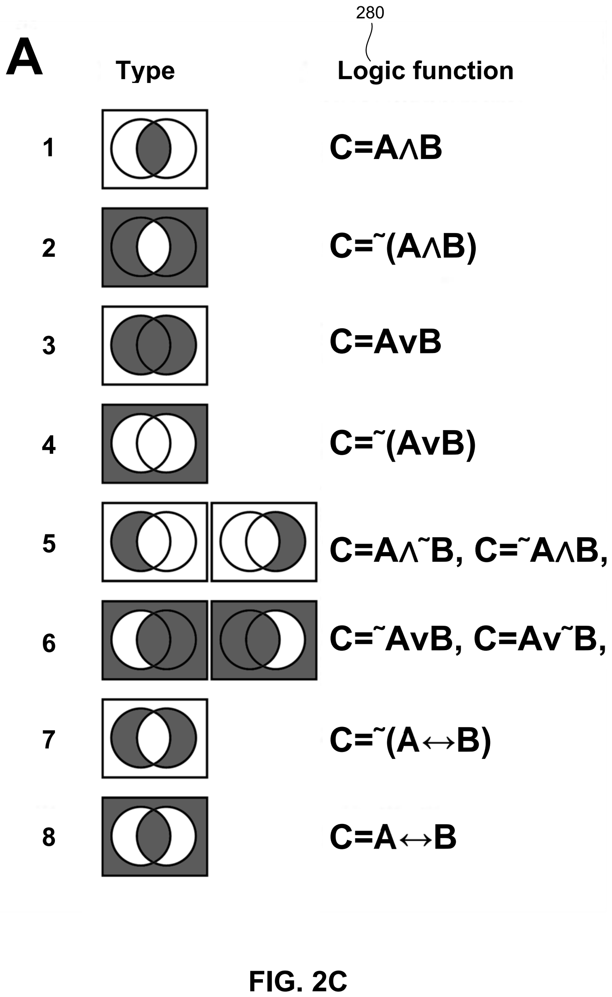

In some embodiments, a corresponding node architecture 254 associated with one or more respective agent modules 226 defines conditional logic 260, e.g., for performing a specific task (e.g., a specific clinical task). For example, each respective node 256 may include corresponding logic 260, which defines a workflow for handling one or more tasks assigned to the respective node 256. In some embodiments, the conditional logic of the node architecture 254 is executed in accordance with a first order of a first set of interconnected nodes 256 from a plurality of nodes 256 based on the corresponding logic 260 of each node 256 in the set of interconnected nodes 256. Accordingly, the logic 260 allows for granular configuration of each respective node 256 that when collectively coupled through interconnected nodes of the node architecture 254, define a conditional logic of the node architecture. For example, the logic 260 may include one or more logical operations or functions, such as AND, OR, XOR, and/or NOT operations (and/or any of the functions 280 shown in Figure 2C). As an example, logic 260 for a node 256 may require the presence of a first condition but not a second condition or third condition.

In some embodiments, the plurality of nodes includes one or more data source nodes 256 associated with a specific task of obtaining data elements from a remote data source (e.g., an external database 108). In some embodiments, the corresponding logic 260 allows for connecting to a corresponding database, e.g., by using an access token associated with the corresponding agent module 226, communicating at least a portion of the obtained data to one or more nodes 256, and/or execute one or more queries to identify/analyze such data. In some embodiments, each node architecture 254 includes at least one input node, which forms an initial terminal node in an order of nodes 256. In some embodiments, the node architecture includes a plurality of paths to traverse from an input to an output node, such as paths of branching trees. In some embodiments, each respective node 256 represents a computational process, such as a function, an input, an output, or the like, that is realized when data is applied to the node 256. Moreover, since each node is interconnected, such by an edge, to at least one other node 256, the output from one node 256 may be supplied as input to a different node 256 in order to form chains and/or orders in the node architecture 254.

In some embodiments, the memory 218 includes one or more modules not shown in Figures 2A and 2B. For example, the memory 218 may include one or more agent tools (e.g., a communication tool) that are distinct from the agent modules 226. In some embodiments, the client device 102 includes one or more standalone agents (e.g., that execute and operate at the client device 102) and/or one or more dependent agents (e.g., that operate in conjunction with a component at a remote device, such as the server system 106). In some embodiments, one or more agents are generated/trained at the server system 106 and deployed at the client device 102.

Although Figures 2A and 2B illustrate the client device 102 in accordance with some embodiments, Figures 2A and 2B are intended more as a functional description of the various features that may be present in a client device than as a structural schematic of the embodiments described herein. In practice, and as recognized by those of ordinary skill in the art, items shown separately could be combined and some items could be separated.

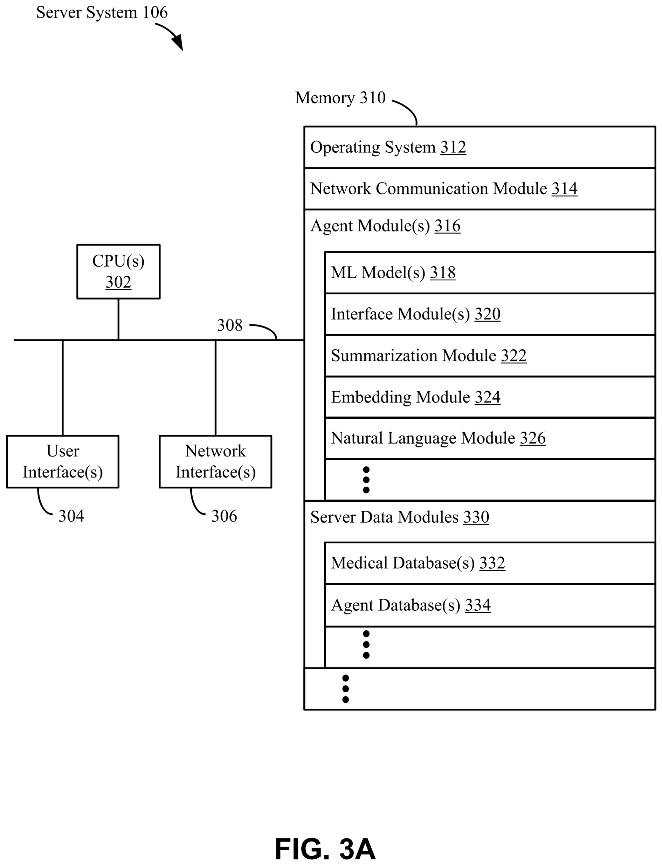

Figure 3A is a block diagram illustrating a server system 106 in accordance with some embodiments. In accordance with some embodiments, the server system 106 includes one or more CPUs 302, one or more user interfaces 304, one or more network interfaces 306, memory 310, and one or more communication buses 308 for interconnecting these components. In some embodiments, the server system 106 includes other types of control circuitry and/or processors (e.g., in addition to, or alternatively to the CPUs 302). For example, the server system 106 may include one or more GPUs or DPUs for machine learning tasks.

The memory 310 includes high-speed random-access memory, such as DRAM, SRAM, DDR RAM, or other random access solid-state memory devices; and may include non-volatile memory, such as one or more magnetic disk storage devices, optical disk storage devices, flash memory devices, or other non-volatile solid-state storage devices. The memory 310 optionally includes one or more storage devices remotely located from one or more CPUs 302. The memory 310, or, alternatively, the non-volatile solid-state memory device(s) within the memory 310, includes a non-transitory computer-readable storage medium. In some embodiments, the memory 310, or the non-transitory computer-readable storage medium of the memory 310, stores the following programs, modules and data structures, or a subset or superset thereof:
- an operating system 312 that includes procedures for handling various basic system services and for performing hardware-dependent tasks;
- a network communication module 314 that is used for connecting the server system 106 to other computing devices connected to one or more networks 104 via one or more network interfaces 306 (wired or wireless);
- agent module(s) 316 that may engage with a user (e.g., a remote user) and invoke external services when appropriate to obtain information or perform various actions (e.g., in an integrated, conversational manner using natural language dialog). In some embodiments, the agent module(s) 316 work in conjunction with the agent module(s) 226 at a client device 102. In some embodiments, the agent module(s) 316 include the following submodules (or sets of instructions), or a subset or superset thereof:
   ∘ one or more models 318 that engage with a user and/or perform specific tasks (e.g., in furtherance of a user request or query). In some embodiments, the model(s) 228 include one or more large language models, such as GPT-3, GPT-4, BioGPT, and PaLM-2, neural networks, transformer models, and/or other types of ML models; and
   ∘ one or more interface modules 320 that allow the agent module 316 to communicate with other agents, applications, components, and devices (e.g., via an API or structured query);
   ∘ a summarization module 322 that is configured to summarize one or more modalities of data, such as summarizing of a medical visit, annotating and/or labeling images, and/or otherwise summarizing data (e.g., in a human-readable format, such as a natural language summary);
   ∘ an embedding module 324 that is configured to generate embeddings (e.g., vectors) based on input data, such as raw input data and/or summarized input data. In some embodiments, the embedding module 324 is configured to generate modality-specific embeddings. In some embodiments, the embedding module 324 is configured to generate multi-modal embeddings (e.g., by aggregating or combining modality-specific embeddings); and
   ∘ a natural language module 326 that is configured to generate natural language (e.g., conversational) outputs. In some embodiments, the natural language module 326 is configured to convert one or more ML outputs into a natural language output. In some embodiments, the natural language module 326 is configured to generate embeddings from natural language inputs (e.g., received from a user via a digital assistant interface);
- one or more server data modules 330 for managing the storage of and/or access to data (e.g., clinical and user data). In some embodiments, the one or more server data modules 330 include:
   ∘ one or more medical databases 332 for storing medical data (e.g., regarding therapies, drugs, treatments, patients, cohorts, imaging, and/or diseases); and
   ∘ one or more agent databases 334 for storing agent data such as settings, training, instructions, and other metadata.

In some embodiments, the server system 106 includes web or Hypertext Transfer Protocol (HTTP) servers, File Transfer Protocol (FTP) servers, as well as web pages and applications implemented using Common Gateway Interface (CGI) script, PHP Hyper-text Preprocessor (PHP), Active Server Pages (ASP), Hyper Text Markup Language (HTML), Extensible Markup Language (XML), Java, JavaScript, Asynchronous JavaScript and XML (AJAX), XHP, Javelin, Wireless Universal Resource File (WURFL), and the like.

In some embodiments, the memory 310 includes one or more modules not shown in Figure 3A. For example, the memory 310 may include one or more agent tools (e.g., a translation tool) that are distinct from the agent module(s) 316. In some embodiments, the server system 106 includes one or more standalone agents (e.g., that execute and operate at the server system 106) and/or one or more dependent agents (e.g., that operate in conjunction with a component at a remote device, such as a client device 102). In some embodiments, the memory 310 includes an agent library (e.g., the agent library 250).

Although Figure 3A illustrates the server system 106 in accordance with some embodiments, Figure 3A is intended more as a functional description of the various features that may be present in a server system than as a structural schematic of the embodiments described herein. In practice, and as recognized by those of ordinary skill in the art, items shown separately could be combined and some items could be separated. For example, some items shown separately in Figure 3A could be implemented on single servers and single items could be implemented by one or more servers. In some embodiments, the clinical database(s) and/or the agent database(s) 334 are stored on devices that are accessed by the server system 106 (e.g., the external database(s) 108). The actual number of servers used to implement the server system 106, and how features are allocated among them, will vary from one implementation to another and, optionally, depends in part on an amount of data traffic that the server system manages during peak usage periods as well as during average usage periods.

Each of the above identified modules stored in the memory 218 and 310 corresponds to a set of instructions for performing a function described herein. The above identified modules or programs (e.g., sets of instructions) need not be implemented as separate software programs, procedures, or modules, and thus various subsets of these modules may be combined or otherwise re-arranged in various embodiments. In some embodiments, the memory 218 and 310 optionally store a subset or superset of the respective modules and data structures identified above. Furthermore, the memory 218 and 310 optionally store additional modules and data structures not described above.

As used herein, a transformer model (sometimes referred to as a transformer) is a neural network that learns context and thus meaning by tracking relationships in sequential data like the words in this sentence. Transformer models can apply attention, or self-attention, to detect how distant data elements in a series influence and depend on each other. Using embeddings (e.g., word embeddings), transformers can pre-process text as numerical representations through the encoder and understand the context of words and phrases with similar meanings as well as other relationships between words such as parts of speech. The models can then apply this knowledge of the language through the decoder to produce a unique output. Transformer models may be components of another model, such as a large language model (LLM).

An LLM is a large deep learning model that is pre-trained on large amounts of data, for example, in the size range of terabytes or even pentabytes. An LLM may have billions or trillions of parameters. LLMs typically consist of dozens or even hundreds of transformer blocks stacked on top of each other. In a classic LLM, each LLM includes an encoder block that takes a sequence and processes it into a set of context-rich embeddings, and a decoder block that takes the encoder's output and generates the output sequence. However, some LLMs include transformer blocks that only include an encoder and some LLMs include transformer blocks that only include a decoder. The transformer architecture makes use of self-attention, residual connections, and normalization. LLMs, which include stacks of transformer blocks, therefore make use of these features as well. Whereas a transformer model has in the order of millions of parameters, a large language model is characterized by having at least 1 billion parameters. As is apparent to one of skill in the art, these values exist in a continuous stream, e.g., there may be LLMs with 100 million parameters, 50 transformer blocks, or other numbers of parameters that allow for the robust performance expected of LLMs. As an example, a transformer model may have between 6 to 24 transformer blocks and an LLM may have 80 or more transformer blocks. As another example, a transformer model may be trained on domain-specific datasets that range in size between gigabytes and tens of gigabytes and an LLM may be trained on more diverse datasets that are measured in terabytes or pentabytes.

Embeddings are representations of values or objects (e.g., text, images, and/or audio) that are used by machine learning models. Thus, embeddings may represent features extracted from raw data. Embeddings may be (feature) vectors generated to capture meaningful data about each object. An embedding may be a word embedding that represents a word (or phrase) and is used in text analysis. The word embedding may be in the form of a real-valued vector that encodes the meaning of the word in such a way that the words that are closer in the vector space are expected to be similar in meaning. In the case where words and phrases are one-hot encoded, an embedding is typically dimension reduced relative to the model input. For example, consider the case where a model has a vocabulary size of 50,000 words and/or phrases. Words and phrases in model input are one-hot encoded using this vocabulary and thus the input has a dimension of 50,000. In some models in accordance with the present disclosure, such high-dimensional input is dimension reduced relative to the original one-hot input. For instance, in one particular example the embedding maps the 50,000 word/phrase vocabulary to 768 dimensions. However, there is no absolute requirement that an embedding be dimension reduced relative to the input. For instance, in some embodiments the embedding captures input context, resulting in embeddings that are not dimension reduced relative to the input.

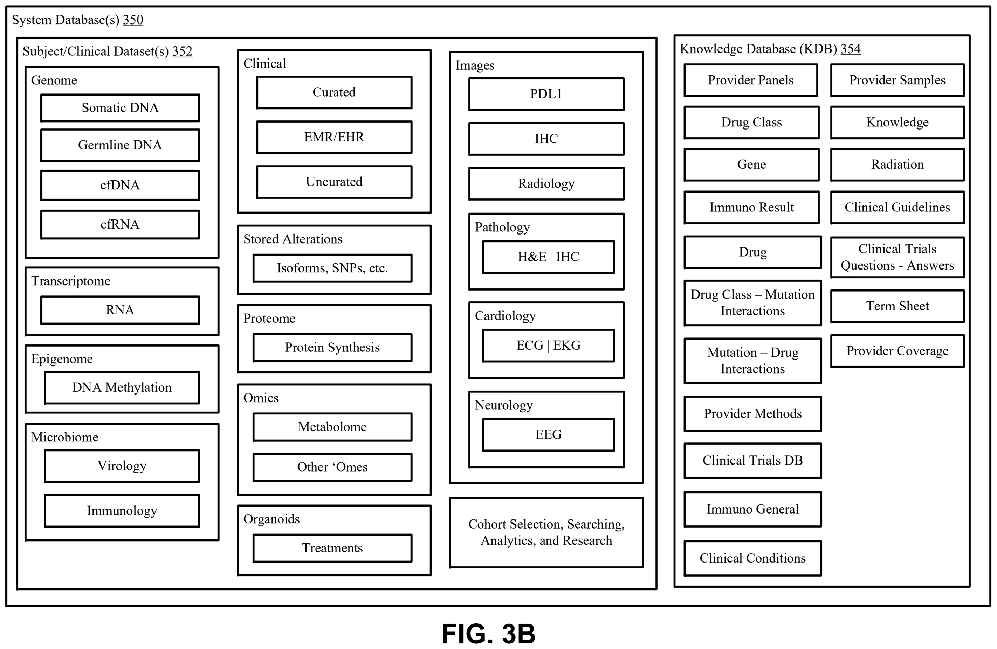

Figure 3B is a block diagram illustrating one or more system databases 350 in accordance with some embodiments. In some embodiments, at least a portion of the system database(s) 350 is stored at a client device 102 (e.g., as the medical database(s) 244), the server system 106 (e.g., as the medical database(s) 332), and/or the external database(s) 108, which advantageously allows for an edge at and/or near the client device 102, such as via the communication network. However, the present disclosure is not limited thereto. In some embodiments, a single database stores all of the information shown in Figure 4. In some embodiments, the information is stored in a set of two or more databases.

In some embodiments, the system database(s) 350 include subject and clinical datasets 352 and/or a non-subject specific knowledge database (KDB) 354. In some embodiments, the data stored in the system database(s) 350 includes a plurality of categories of data or data features, the categories of data or data features encapsulating the different data modalities such as a structured text modality, an unstructured text modality, a tabular data modality, a data visualizations modality, an image modality, an audio modality, a video modality, a biological sequence modality, a natural language modality, and a source code modality. In some embodiments, the data stored in the system database(s) 350 includes raw data (e.g., unstructured data corresponding to entire documents in original formatting). In some embodiments, the data or features stored in the system database(s) 350 includes formatted (e.g., structured data) and/or summarized data (e.g., summaries generated by one or more modality-specific summary agents). In some embodiments, the system database(s) 350 include data or features stored in an embedding format (e.g., a numerical vector format).

In some embodiments, the datasets 352 include, among other data, genome, transcriptome, epigenome, microbiome, clinical, stored alterations proteome, additional -omics, organoids, imaging and cohort and propensity data sets. For example, the cohort selection, searching, analytics, and research datasets may include data about patients and conditions, such as tumors of unknown origin (TUO) predictors, metastasis predictors, and survival analytics. As an example, the imaging datasets may include radiology imaging data, immunohistochemistry imaging data, positron emission tomography (PET) data, pathology imaging data, cardiology imaging data, neurology imaging data, and/or single-photon emission computed tomography (SPECT) imaging data. The pathology imaging data may include hematoxylin and eosin (H&E) and/or Immunohistochemistry (IHC) data. The cardiology imaging data may include electrocardiogram (ECG or EKG) data. The neurology imaging data may include electroencephalogram (EEG) data. The imaging datasets may include data regarding nodule identifiers, tracking, and/or longitudinal analytics. The imaging datasets may also include data regarding whole slide staining using hematoxylin and eosin (H&E) or immunohistochemistry (IHC) stains and/or pathology reports. The clinical data may include curated, uncurated, electronic medical record (EMR), and/or electronic health record (EHR) data. The uncurated data may include raw images of documents which can be OCRed and then fed to a model for structuring/summarizing. In some embodiments, the same model performs the OCR and structuring/summarizing, such as a LLM, transformer, neural network, or machine learning model.

In some embodiments, the clinical data includes diagnostics, imaging, biopsy information, and other disease- and condition-related data. For example, for endocrinology diagnostics, the primary test used may be a blood test to measure hormone levels in the body, which can identify various endocrine disorders by checking for imbalances in hormones such as thyroid stimulating hormone (TSH), luteinizing hormone (LH), follicle stimulating hormone (FSH), testosterone, and others depending on the suspected condition. Additional tests such as ultrasounds, CT scans, or biopsies may be performed depending on the situation, e.g., to locate abnormalities in endocrine glands like the thyroid or adrenal glands. Blood tests for endocrinology diagnostics can be used to measure various hormones in the blood, allowing diagnosis of conditions like hypothyroidism, hyperthyroidism, diabetes, and adrenal insufficiency. Imaging tests such as ultrasounds, CT scans, or MRIs can be used to visualize the endocrine glands and identify abnormalities like nodules or tumors. A fine needle aspiration (FNA) biopsy may be performed to collect a tissue sample from a suspicious area in the thyroid gland for further analysis. Thyroid function tests may be used to measure TSH, T4, and T3 levels to assess thyroid function. Cortisol level tests may be used to check for adrenal gland issues. Glucose tolerance tests may be used to diagnose diabetes by monitoring blood sugar levels, e.g., after consuming a sugary drink. Prolactin tests may be used to check for prolactin levels associated with pituitary gland disorders. Calcium and parathyroid hormone (PTH) levels may be determined to assess parathyroid gland function. For each endocrinology-related test, the data relating to the test (e.g., diagnostics, imaging, and metadata (such as timing, location, etc.)) may be stored in the clinical data, and associated with a particular subject.

As another example, for diabetes diagnostics, a doctor may use a blood test, such as the Hemoglobin A1c (A1C) test, which measures average blood sugar level over the course of two to three months. The A1C test provides a snapshot of a subject's average blood sugar over a period of time and does not require fasting. Other tests may be used, such as a fasting blood sugar test, an oral glucose tolerance test (OGTT), or a urine test, depending on the situation. The fasting blood sugar test measures a subject's blood sugar level after fasting for at least 8 hours. The OGTT involves the subject drinking a sugary liquid and then having their blood sugar levels checked at specific intervals. While not as accurate as blood tests, a urine test may be used in some situations to check for ketones, a sign of uncontrolled diabetes, particularly in type 1 diabetes. For each diabetes-related test, the data relating to the test may be stored in the clinical data, and associated with a particular subject.

As another example, to diagnose and/or assess depression a variety of tests and tools can be used, including questionnaires, physical exams, lab tests, and brain scans. For example, the Patient Health Questionnaire (PHQ-9) is a questionnaire that can help diagnose depression and assess its severity. The PHQ-2 is an initial screening tool for depression that can be used in all age groups. Other questionnaires include the Social Problem-Solving Inventory-Revised (SPSI-RTM), which is a self-report measure of social problem-solving strengths and weaknesses. The Edinburgh Postnatal Depression Scale (EPDS) is a 10-question scale that can be used to screen for depression in women who have recently given birth. In some situations, a doctor or other mental health professional may perform a physical exam and ask questions about a subject's health to diagnose/assess depression. A mental health professional may also use the criteria for depression listed in the Diagnostic and Statistical Manual of Mental Disorders (DSM-5). In some situations, lab tests are used to rule out other medical conditions that could be presenting as depression. These tests may include a complete blood count (CBC), thyroid-stimulating hormone (TSH), vitamin B-12, and the like. Additionally, a PET scan of the brain can compare brain activity during periods of depression with normal brain activity. A CT scan or MRI of the brain may be considered if organic brain syndrome or hypopituitarism is in the differential diagnosis. For each depression-related test, the data relating to the test may be stored in the clinical data, and associated with a particular subject.

As another example, there are different types of diagnostic tests that can be used to diagnose cardiovascular disease, including Electrocardiograms (ECG or EKG), longitudinal Holter monitoring, stress tests, cardiac MRIs, cardiac positron emission tomography (PET) scans, invasive coronary angiographies, echocardiograms, blood tests, x-rays, cholesterol tests, c-reactive protein tests, trimethylamine N-oxide tests, serum creatinine, and plasma ceramides tests. A doctor may use a combination of tests to diagnose a heart problem. For example, a doctor might use an echocardiogram, cardiac MRI, or a nuclear heart scan to take images of the heart during or after a stress test. For each test, the data relating to the test (including any comparisons, cross references, and conclusions based on multiple tests) may be stored in the clinical data, and associated with a particular subject.

In some embodiments, the KDB 354 includes separate sub-databases related to specific information types including, as shown, provider panels (e.g., information related to genetic panels supported by the service provider that operates the system), drug classes (e.g., drug class specific information (e.g., do drugs of a specific class work on pancreatic cancer, what drugs are considered to be included in a specific drug class, etc.)), specific genes, immuno results (e.g., information related to treatments based on specific immuno biomarker results), specific drugs, drug class-mutation interactions, mutation-drug interactions, provider methods (e.g., questions about processes performed by the service provider), clinical trials, immuno general, clinical conditions such as clinical diseases, term sheets (e.g., definitions of industry specific terms), provider coverage (e.g., information about provider tests and results), provider samples (e.g., information about types of samples that can be processed by the provider), knowledge (e.g., scripted questions and answers on various frequently asked questions that do not fall into other sub-databases), radiation (e.g., information related to suitable radiation treatments given specific cancer states), clinical guidelines (e.g., national guidelines related to classification of cancer states, accepted treatments, etc.) and clinical trials questions-answers (e.g., information related to locations and administrators of clinical trials. Organizing the KDB 354 into sub-databases may make it easier to manage those databases as information therein evolves over time and also enables addition of new sub-databases related to other defined information types. In some embodiments, the clinical datasets 352 and/or the KDB 354 is arranged in a different manner than is shown in Figure 4 (e.g., with different sub-databases and/or with a different organizational scheme).

In some embodiments, the data stored in the subject and clinical datasets 352 and/or the KDB 354 is includes raw data, annotated data, and/or summarized data. In some embodiments, the raw data is input into one or more models to generate the annotated and/or summarized data. For example, a model may receive raw data, such as sequencing results, documents, and/or images, and extract/predict status information and/or summaries. In some embodiments, one or more models (e.g., one or more agents) are used to partition, annotate, summarize, and/or structure the data received from external sources (e.g., external databases and/or third parties). In some embodiments, the data stored in the subject and clinical datasets 352 and/or the KDB 354 is classified, grouped, cross-referenced, and/or otherwise related to other data using one or more models (and/or one or more agents). For example, a cohort may be identified based on EMR/EHR information from multiple subjects/patients. In some embodiments, an intake agent is used on data that is received to perform one or more of the actions described above. In some embodiments, different intake agents (e.g., data processing/pre-processing agents) are used for different modalities of data.

Advantageously, by utilizing multiple datasets associated with different domains of subject matter and/or applying a classification system to the datasets, the knowledge database provides a storage system for data, such as medical records and clinical documentation that one or more agent modules 226 can retrieve based on a task-specific requirement associated with a respective domain or classification. Moreover, in some embodiments, the knowledge database 354 allows for storing such data with deidentifying controls in order to allow for training on and/or analysis of the stored data without risk of leaking confidential and/or privileged information.

Considering the extensive volume of text contained within a real-world data (RWD) warehouse of EHRs, it becomes impractical to process the entirety of a patient's clinical notes within the context window of a model (e.g., an LLM). In some embodiments, this challenge is addressed by implementing a retrieval-augmented generative (RAG) approach to identify relevant portions of EHR text, e.g., relevant portions of unstructured clinical notes. A RAG approach proves to be more efficient and effective than providing the model with larger context windows. In some embodiments, RAG is a two-step process that involves retrieving relevant documents from a corpus (e.g., a large corpus with thousands or millions of documents) and then feeding the retrieved documents into a model to generate an analysis and response.

In some embodiments, one or more of the agent modules of the agent library 250 use a retrieval-augmented generative (RAG) to perform operations described herein (e.g., requests to process zero-shot information). For example, the computing system may apply the RAG process to entire patient records, which allows for applying the entire patient records to a model 228 with excess computational burdens, as opposed to focusing solely on a specific type of clinical note. In some embodiments, the RAG process is used to analyze clinical mentions throughout a patient's entire record without the need for predefined sections of interest. However, the present disclosure is not limited thereto. In some embodiments, the RAG process utilizes one or more vector embeddings, such as a plurality of predetermined vector embeddings in which each predetermined vector embedding is associated with a corresponding text string, or snippet. Advantageously, this RAG approach can be more efficient and effective than providing a model (e.g., an LLM) with larger context windows.

In some embodiments, one or more of the agent modules use additional techniques to address an issue that RAG implementations can fail to obtain all of the needed information to fully answer a question (e.g., a user query). In such situations, another request (e.g., a new user query, and/or a modified version of the user query) can be automatically generated to cause more information to be obtained. An example technique includes applying a user query for information from a source dataset to a first RAG agent (e.g., one or more agent module(s) 226) to determine if there is enough information to generate an output based on the user query. The RAG agent can determine that there is enough information, that there is not enough information, or that the determination is not clear. In some embodiments, if the determination whether there is enough information is not clear, the computing system provides a query to a different task-specific orchestration (e.g., corresponding to a different agent module 226). That is, in some embodiments, the system determines that the RAG agent may not be the optimal instrumentation for resolving the user query.

In some embodiments, operations of one or more task-specific orchestrations of the system are adjusted to reduce/prevent negative consequences of retrieval-augmented generation. For example, for some inclusion/exclusion criteria for trial matches or care gap discovery, the queries have a relationship and can include a temporal question (e.g., "Is this medication administration currently administered as the first line of therapy?"). As another example, with a standard RAG retrieval approach, only documents relevant to medications may be retrieved. But the task-specific orchestration (e.g., the RAG agent) may not know if the medications were administered as part of the first or second line of therapy without the full context of the patient. In such situations, using a large context where most of the patient notes can be applied can provide the task-specific orchestration better context and more comprehensive information about the temporal relationship between events. Alternatively (e.g., to address the resource constraints of increasing the context window applied to the RAG agent), a different model (e.g., a full patient record LLM with a one-million-character context window) or agent can be used to resolve the user query in addition or alternatively to the RAG agent. For example, increasing the context window and/or performing additional operations alternative to directing a request to the RAG agent (e.g., to extract information) can increase performance of generating the output based on the user query for information. As another example, a particular subset of data may be used for verification of the data/results. For example, insurance claims data may be used to verify which medications are administered during particular times (e.g., corresponding to particular lines of treatment). In this way, insurance claims data may be used to identify transitions between different lines of treatment.

Figure 4 illustrates an example system architecture for deploying agents (e.g., agent modules 226 and/or 316) in accordance with some embodiments. The architecture 400 shown in Figure 4 includes an agent builder component in a control plane of a client device 102. The control plane may function as a supervisor of data, coordinating communication between different components and collecting data from a data plane (e.g., a working environment presented on a display of the client device 102). In some embodiments, the control plane resides above the data plane (e.g., above the working environments) and enforces rules for the data plane, which allows for partitioning the data plane to prevent unauthorized or unauthenticated control of the data plane from unsecure client devices, such as those unassociated with a portion of the data plane. However, the present disclosure is not limited thereto. In some embodiments, the agent builder hosts a user interface for configuring agent modules, such as by configuring the corresponding node architecture 254 associated with the agent module 226. In some embodiments, the agent builder component is communicatively coupled to an agent library (e.g., the agent library 250) in the control plane that stores a plurality of agent modules, such as the agent module 316, and to an agent host (e.g., via a configuration publication/subscribe (pubsub) component) in a working environment. The agent module in the working environment may be communicatively coupled to an agent library in the working environment, a document index (e.g., one or more data sources, such as knowledge database 354 and/or external databases 108), and a large language model (e.g., a model 318). In some embodiments, the agent library 250 includes a user interface and API for interacting with deployed agents.

In some embodiments, the agent builder includes a frontend and a backend. In some embodiments, the agent builder frontend includes an access component (e.g., an administrative console that may be a home user interface that a user is presented with upon providing access credentials to the application), an agent list (e.g., an agent library that may include a plurality of orchestrations to which the user has access, e.g., based on the access credentials provided to the application), an agent builder component (e.g., including a first representation of a node architecture 254 (e.g., a form-builder representation) and a second representation of the node architecture 254 (e.g., a workflow representation)), and/or a data source management component. In some embodiments, the agent builder backend includes a database layer, an API service, and/or a configuration publisher component. In some embodiments, the frontend and the backend of the agent builder are executed on separate electronic devices.

In some embodiments, the agent host includes a frontend and a backend. In some embodiments, the agent host frontend includes an access component, an agent list, an interaction console, and/or a document console. In some embodiments, the agent host backend includes a websocket for interactive user, a database layer, an API access to deployed agents, tools and/or custom chain implementation, a document loader, and/or a configuration subscription component. In some embodiments, the frontend and the backend of the agent host are executed on separate electronic devices.

In some embodiments, the agent builder component is configured to generate, deploy, and/or update one or more agent modules and/or a corresponding node architecture to one or more working environments (e.g., one or more workload planes). In some embodiments, each agent module is associated with an agent type. In some embodiments, the agent type includes a type of model and/or conditional logic, such as an implementation configuration. For example, an agent module may include a language model associated with a first node and a corresponding type-specific logic that further associates the agent module, through the first node, with a particular domain, such as a first configuration implementation for applying the prompt to the model if the prompt is associated with a first modality and a second configuration implementation if the prompt is associated with a second modality different from the first modality. In some embodiments, the logic is specified in a corresponding agent module configuration file, which advantageously allows for configuring the logic after applying various prompts to the agent module and/or using multiple client devices (e.g., end users) to configure the logic. However, the present disclosure is not limited thereto.

In some embodiments, the available agent module types include a transform agent (e.g., performing functions such as data transformations, regular expressions, and string templating), an authorization agent, a language model agent (e.g., applying inputs to a large language model), a data collection agent (e.g., RAG modules), a super-agent (e.g., aware of other agent types and their capabilities and configured to instantiate and/or delegate to the appropriate agent modules), a sequential agent (e.g., including multiple models and/or tools coupled together in a sequential fashion), a tool-using agent, a coding agent (e.g., configured to generate code in particular programming languages), and/or a categorization agent (e.g., configured to determine an intent, domain, or other categorization for user inputs). In some embodiments, the transform agent comprises one or more ML models (e.g., stored as transforms accessible by the platform). In some embodiments, the one or more ML models are stored (e.g., as disk images) for subsequent initialization/instantiation. In some embodiments, the language model agent provides and/or stores context information such as conversation history, user preferences, subject details, and the like. In some embodiments, the data collection agent is couplable to external data sources (e.g., the external service(s) 110 and/or the external database(s) 108) and configured to request and/or retrieve data from the external data sources. In some embodiments, a sequential agent includes a recursive module (e.g., repeating and/or refining outputs until predetermined criteria are met). In some embodiments, a super agent is configured to compare available agent types and recommend a particular agent type for a particular situation/purpose. In some embodiments, a coding agent is configured to generate code for new agent modules based on inputs (e.g., natural language inputs) from a user. In some embodiments, a categorization agent is a component of a routing agent. For example, the categorization agent determines an intent/domain for an input and the routing agent routes the input to a downstream component in accordance with the determined intent/domain. In some embodiments, a sequential agent is a component of a routing agent. For example, the routing agent coordinates operation (e.g., data transmission and timing) of multiple components and/or modules. In some embodiments, each agent module is generated/provided with guardrails (e.g., enforcing privacy, security, data typing, etc.). In some embodiments, an agent module is configured to recognize whether data is protected health information (PHI) and take appropriate action. For example, an agent module may disable information sharing options when providing PHI.

In some embodiments, different agent types are associated with (e.g., trained on, instructed on, and/or coupled to) different domains (e.g., different subjects, types of data, modalities of data, and/or classes of data) in a plurality of domains. For instance, in some embodiments, the plurality of domains forms an input space, which defines a universe of data associated with a variety of subject matters. In some embodiments, the input space defines an N-dimensional space of data obtained from a plurality of data sources, in which N is a positive integer, such as two, three, four, ten, etc. In some embodiments, each respective domain in the plurality of domains defines a partition classification or subset of data, such as one or more specific data sets of system databases 350 of Figure 3B. In some embodiments, different agent types are associated with (e.g., trained on, instructed on, and/or coupled to) different data modalities in a plurality of data modalities. However, the present disclosure is not limited thereto.

As a non-limiting example, consider a first input space associated with a plurality of medical records, in which each medical record in the plurality of medical records includes a plurality of text data and a plurality of graphical data associated with a corresponding patient. Accordingly, a plurality of domains collectively defined by information obtained from the plurality of medical records allows for classify the information and training a corresponding agent module on the information classified domain, such as a first domain associated with a statin drug class of Figure 3B, and a second domain associated with a glucagon-like peptide (GPL) agonist drug class.

As a non-limiting example, a first agent module may be associated with a first domain for generating a summary of a patient's textual medical record, a second agent module may be associated a second domain for responding to general medical inquiries, a third agent module may be associated with a third domain for medical visit planning and preparation, and a fourth agent module may be associated with a fourth domain for generating inferences to user queries using the data generated by the other three agent modules. In another example, a first agent module may be associated with a first domain for generating a summary of a patient's textual medical record, a second agent module may be associated with a first domain for guiding a subject, such as a patient or a medical practitioner associated with the patient, through a care plan, a third agent may be associated with a third domain for creating patient care guidelines based on a patient's health profile, a fourth agent module may be associated with a fourth domain for identifying patients requiring follow-up at a hospital, a fifth agent module may be associated with a fifth domain for identifying changes in a standard of care for a disease setting, and/or a sixth agent module may be associated with a sixth domain for evaluating data associated with a patient to identify a cohort of similar patients.

One example agent type is a database-interfacing agent module associated with one or more data source nodes 256. An example database-interfacing agent may be an adverse effects agent that has access to an FDA label database and is configured to interpret adverse effect information from the database. The configuration of the database-interfacing agent module may include a custom prompt for the model(s) of the agent module and one or more data sources that the agent database-interfacing module may access and/or use.

Another example agent type is a custom-chain agent module (e.g., a super-agent module) that takes an input prompt, analyzes the prompt (e.g., parsing the prompt into one or commands and/or a plurality of tokens), and transmits information from the parsed prompt (e.g., commands and/or tokens) to a model or other component, such as a node 256 of the custom-chain agent module or a different node 256 of a different agent module). For example, the custom-chain agent module may obtain data from different databases (e.g., external databases 108, knowledge database 354, etc.), in which the data is obtained in a variety of different formats, modalities and/or structures, such as unstructured text, structured text, tables, charts, graphical data, and/or biological data. In some embodiments, the agent module reformats, summarizes, and/or restructures the data obtained from the databases for application to a model of the custom-chain agent module and/or to a different agent module. In some embodiments, the custom-chain agent module evaluates and/or obtains a set of parameters for inputting data to the model(s) and/or agent module(s) and translates the data obtained from the databases based on the set of parameters. In some embodiments, the obtained data is restructured into a homogenous dataset (e.g., different hospitals may use different codes for the same procedure, such is homogenized by the agent module into a uniform coding). The configuration of the custom-chain agent module may include a sequence of nodes 256 associated with the custom-chain agent module and/or other nodes associated with other agent modules to be used by the custom-chain agent module and/or definitions of corresponding chain objects.

As illustrated in the above examples, an agent module may be considered a configuration of a particular agent type for a particular task through a plurality of interconnected nodes 256 that form a node architecture 254 of the agent module (e.g., represented as a database object). An agent module may be configured for dissecting complex evaluations and logics into a reasoning path through the plurality of interconnected nodes 256, which makes arriving at an accurate and precise response computationally less burdensome. In some embodiments, the agent modules are accessible via an interaction console and/or an application programming interface (API). In some embodiments, one or more parts of the agent configuration are stored in a separate versioning table (e.g., linked by agent ID). In this way, an agent configuration may be edited without affecting a deployed agent version.

In an example scenario, a user configures an agent in the console and then deploys it to one or more environments (e.g., workload planes and/or control planes). For this scenario, the agent configuration is stored in the control plane (e.g., as shown in Figure 4). As shown in Figure 4, the agents themselves execute in the appropriate working environments, and working environments do not have access to the control plane. The agent builder in the control plane may be configured to push configurations into the various environments (e.g., via the config pubsub component shown in Figure 4). In some embodiments, when an agent configuration is changed or an agent version is deployed, the agent builder informs the agent host in each environment so that the updated agent can be deployed. This may be via a pubsub message to the agent-config topic or via a simple HTTP request.

The architecture 400 allows for flexibility in supporting a variety of deployment strategies for each respective agent module. For example, some end-users, e.g., those using agent modules interactively and without engineering support, expect to operate their agent modules entirely within a production working environment. In some embodiments, the administrator, such as a creator, of an agent module is able to choose a deployment style suitable for their application, such as by restricting the agent module to one or more domains, one or more databases 108, one or more services 110, or a combination thereof. For example, a first user may wish to employ a user interface that includes one or more user interface elements described with respect to the application by directly embedding the components within a web page, and a second user may wish to interact with an API that is configured to receive user requests and provide responses in the form of data structures, which the second user may integrate into different user interface elements not associated with the application.

In some embodiments, users of an agent builder user interface in the control plane are provided with a production access token that can also make requests to the production agent host. In some embodiments, an integrated user interface is presented to a user that shows both the agent builder having a plurality of input features visualized through a representation and the interaction console without concerning the users with the differences between the control plane and the working environments. For example, for users who want to test out agent modules in a lower environment, a link may be provided to open that agent module in a new tab or frame of an application. In some embodiments, a request to authenticate is presented and an access token is obtained by the agent module for that environment. In some embodiments, the user interface includes an indication of which environment is currently active.

In some embodiments, a data module 410 (e.g., document index) as shown in Figure 4 includes one or more of: a static corpus, a dynamic corpus, an embedding model (e.g., a model 228), a chunking strategy, a storage back-end, a data classifier (e.g., public, internal, or secret), and/or a visibility setting (e.g., private, public, or restricted by role). In some embodiments, the data module maintains an index of data that may be ephemeral or permanent. In some embodiments, data elements associated with data files (e.g., documents) are evaluated via a chunking process, embeddings are generated for the chunks generated from the chunking process, and the embeddings are inserted into a database. In some embodiments, the data module 410 includes a set of retrieval parameters (e.g., for a number of documents to retrieve and/or a similarity measure). In some embodiments, the data module 410 corresponds to a set of databases (e.g., medical databases), such as the database(s) 350 in Figure 3B. In some embodiments, a parameter associated with a node 256 of a respective agent module and/or model includes selecting one or more document indices to retrieve from via the data module 410. In some embodiments, embeddings are created and siloed for future use. In some embodiments, each embedding is associated with one or more access control lists (ACLs).

Tools are a mechanism by which agent modules can integrate with other components and with the outside world. In some embodiments, tools are made available to the agent modules as agent builder blocks. Some tools may be general-purpose, and others may be custom for a particular integration. Different agent module types may have different access to tools: for example, a tools agent may be configured with a set of available tools, and the model may be configured to choose when and how to use them, rather than follow a fixed sequence of steps. In some embodiments, an agent configuration defines when and how tools are invoked. As an example, a tool may be configured with a fixed base URL so that the agent cannot make authentication requests to some other service. In some embodiments, a tool is configured to use an end-user's access token to authenticate, rather than granting an access role to the agent's machine user. In some embodiments, a tool is restricted to certain endpoints and/or methods (e.g., only GET requests) so that the tool is restricted from performing admin tasks on behalf of a user who lacks admin privileges (e.g., write permissions).

In some embodiments, a tool has parameters that are specified when configuring the agent modules and/or parameters that can be specified at invocation time by the agent module itself. An example tool is an authentication request tool configured to fetch an internal URL using a user's access token. The authentication request tool may include the following parameters: name, description, base URL, and/or input parameters (e.g., specifiable by the agent). For example, an example authentication request tool may have an order identifier as an input parameter. Another example tool is an external request tool that fetches an external URL. The parameters for the external request tool may include: name, description, base URL, and/or input parameters. Another example tool is an email tool that sends an email. The parameters for the email tool may include destination, subject, and/or body.

Other example agent modules include (i) an agent module configured to send emails summarizing which customers are facing issues with orders and/or identifying retraining opportunities, (ii) an agent module configured to generate data tables, JSON schema, and other data translations, (iii) an agent module configured to find orders within a group of clients that have particular flags and/or provide a summary by client, flag, etc. (e.g., with timestamp for order creation timing), (iv) an agent module for identifying behavioral changes in ordering habits and adjust orders accordingly (e.g., increase delays and/or cancel orders) and sending notifications, (v) an agent module for generating inclusion/exclusion criteria from a protocol document, generating structured queries (e.g., SQL queries) from a structured list, and/or generate specifications (e.g., YAML specifications) from structured lists of inclusion/exclusion criteria, and (vi) an agent module for answering questions about particular trials based on information in the protocol and/or other trial materials or documentation. As another example, a set of one or more agent modules may be configured to identify and/or evaluate adverse effects. The example agent module(s) receive a user query regarding adverse effects associated with a particular drug. In this example, the set of agent modules may parse the query in order to identify the drug name from the query and apply the drug name to one or more nodes in order to obtain a set of adverse effects associated with the drug. In this example, the set of agent modules may provide a response with a description of the set of adverse effects.

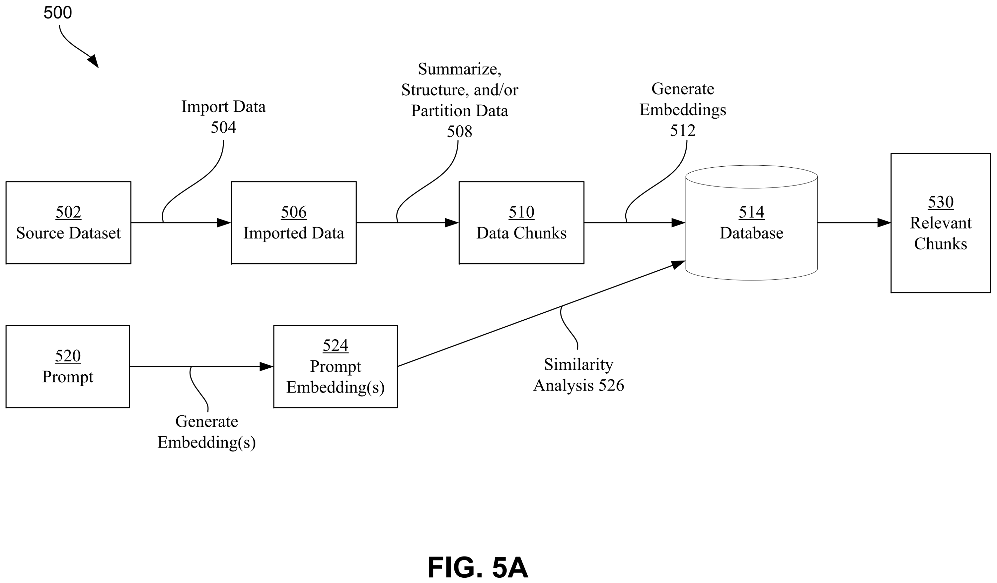

Figure 5A illustrates an example process 500 for data vectorization and query processing in accordance with some embodiments. First, a source dataset 502 is imported (504) as imported data 506. In some embodiments, the source dataset 502 includes one or more documents (e.g., one or more PDF documents), one or more images, and/or other structured or unstructured data (e.g., data tables or records). In some embodiments, the source dataset 502 is obtained from one or more databases (e.g., the external database(s) 108). In some embodiments, the source dataset 502 is identified by a user for importation into the system (e.g., the platform 100). In some embodiments, the source dataset 502 includes medical, clinical, molecular, and/or patient data.

In accordance with some embodiments, the imported data 506 is de-identified (e.g., any personally identifiable information (PII) is removed). The imported data 506 is converted (508) into data chunks 510. In some embodiments, the conversion includes summarizing the imported data 506 (e.g., using one or more machine-learning models). In some embodiments, the conversion includes converting unstructured data into structured data (e.g., using one or more machine-learning models). In some embodiments, the conversion includes partitioning the data (also sometimes called chunking or snippetizing). For example, the imported data may be converted to structured data then summarized and then the summary data may be partitioned to generate the data chunks 510. In some embodiments, the imported data 506 is summarized, e.g., with or without being converted to structured data. In some embodiments, the imported data includes visual data that is annotated and/or characterized during the conversion process. A set of (one or more) embeddings are generated (512) from the data chunks 510 and stored in a database 512 (e.g., a vector database). In some embodiments, the embeddings are used to train (e.g., fine tune) a machine-learning model (e.g., a model that is a component of a task-specific orchestration).

Figure 5A also shows a prompt 520 being received (e.g., via the platform 100). For example, the prompt may be a question about the source dataset 502. The prompt 520 is converted (520) to a set of (one or more) prompt embeddings 524. A similarity analysis 526 (e.g., a cosine similarity analysis) is performed between the prompt embedding(s) 524 and the embeddings in the database 514 (e.g., the embeddings from the data chunks 510). In this way, one or more relevant chunk(s) 530 are identified and may be returned to the user. In some embodiments, the relevant chunk(s) 530 are analyzed and/or summarized and the results of the analysis/summary are provided to the user. In some embodiments, the response to the user includes a short answer, a long answer, and/or information from the relevant data chunks 510.

As an example, a query vector may be generated and used to identify a similar vector in a vector database (e.g., the database 514). The similar vector from the query vector database (and/or the query vector) may be used to identify a second similar vector in a second vector database. The query vector and the second similar vector (and optionally the first similar vector) may be provided to a language model via a prompt. The language model outputs an answer to the query, which is, optionally reformatted, and transmitted to the user. In a specific example, the query is "what is the reason for Linda Watson's order cancelation" and the language model outputs a status reason as the answer.

In some embodiments, an agent module is configured to perform intent matching and/or parameter extraction on the user queries and requests. In some embodiments, the intent is assumed (e.g., the agent module is configured for a specific task). In some embodiments, the agent module extracts domain-specific parameters. For an example query "show patients with MSI high, TMB less than 20, which have been diagnosed with central neurocytoma in the past four months" the extracted parameters may be ["mis": "high", "tmb": "{"It" "20"}, "diagnosis": "central neurocytoma", "date_range":{...}].

In some embodiments, an agent module is configured to automatically populate a structured query (e.g., an SQL query) using a user query and transmit the structured query to a structured database. For example, the agent module may obtain a particular schema, obtain inclusion and exclusion criteria, and generate a structured query for a database based on the criteria identified from the query and the schema of the database to be searched. In some embodiments, the structured query is transmitted to another agent module or component to interact with one or more structured databases. For example, a user query of "how many patients are older than 18?" may be converted to an SQL query "SELECT COUNT(*) FROM demographic WHERE age > 18."

Figure 5B illustrates an example architecture for responding to user queries in accordance with some embodiments. In the example of Figure 5B, a query 550 (or other type of prompt) is received (e.g., via a digital assistant portal, or other type of user interface) at a system (e.g., the server system 106 or client device 102). The query 550 is analyzed by a categorization agent 552. In some embodiments, the categorization agent 552 is a task-specific orchestration. In some embodiments, the categorization agent 552 comprises one or more machine-learning (ML) models, such as a transformer model, a language model, or other type of model. In the example of Figure 5B, the categorization agent 552 categorizes the query 550, and based on the category of the query, transmits query information to a particular destination agent. In some embodiments, two or more agents are used to receive and categorize the query. In some embodiments, two or more agents are used to categorize the query and communicate with the appropriate destination agent(s). In some embodiments, based on the query categorization, the categorization agent 552 sends query information to two or more destination agents (e.g., and aggregates information outputs from the two or more destination agents).

In some embodiments, the categorization agent 552 is a super agent (e.g., configured to receive, categorize, and route user queries). In some embodiments, a different agent is used to provide a response to the query 550 based on outputs from the destination agent(s). In some embodiments, the different agent is configured to generate a natural language response based on the outputs from the destination agent(s). In some embodiments, the categorization agent 552 is configured to receive outputs from the destination agent(s) and generate a response for the user based on the received outputs.

In some embodiments, the agent builder 554 is configured to build (generate) an agent to address the query 550 (e.g., in accordance with a determination that an appropriate agent for responding to the query is not available). In some embodiments, the patient agent 556 is configured to respond to the query 550 using patient data (e.g., an EHR for the patient), such as data from the user database 244. In some embodiments, the summary agent 558 is configured to generate a summary of data obtained from one or more databases. In some embodiments, the summary agent 558 is used in conjunction with one or more of the other destination agents (e.g., to generate a summary of data output from the other destination agents).

In the example of Figure 5B, the potential destination agents include an agent builder 554, a patient agent 556, a summary agent 558, a clinical trials agent 560, a customer support agent 562, a general query agent 564, and a medical publication agent 566. In some embodiments, the potential destination agents include one or more agents not shown in Figure 5B (e.g., an adverse effects agent). In some embodiments, the potential destination agents do not include one or more of the agents shown in Figure 5B. In some embodiments, one or more of the agents are communicatively coupled to a live database (e.g., a database that continues to be updated over time). For example, the clinical trials agent 560 may be communicatively coupled to a live clinical trials database that indicates current clinical trials. As another example, the customer support agent 562 may be coupled to a live customer database (e.g., with frequently asked questions and corresponding answers). As another example, the general query agent 564 may be coupled to a live database of Internet information. As another example, the medical publication agent 566 may be coupled to a live database of medical publications.

Some embodiments employ a blended agent approach for a system of agents, with each using its own workflow based on its purpose. As shown in the example of Figure 5B, an upfront categorization agent determines the nature of a question and what data is needed to answer it, and routes to one or more subsequent agents that each generate an output. For example, all of the agents may use models that are configured to handle protected health information (PHI).

In some embodiments, an output from the architecture in Figure 5B includes a comprehensive response, in a digestible, bulleted format with appropriate medical disclaimers, web links and resources, and/or links to the documents from which relevant patient information was fetched.

In some embodiments, the destination agents include a daily companion agent configured to allow a user to take action within a corresponding application, such as logging a symptom, marking a medication taken, and updating a health profile (e.g., using natural language and without leaving the digital assistant interface). In some embodiments, the destination agents include a care coordination agent configured to take an action outside the application, such as send documents created within the application to external people (e.g., via email or text) and/or interact with patient/provider portals to schedule appointments. In some embodiments, the destination agents include a branded subject matter expert agent configured to (i) bring in source documents such as clinical guidelines, trusted web resources, published cutting-edge research, and proprietary documentation from trusted third parties and (ii) surface digestible insights in the context of a user's health status. In some embodiments, the destination agents include a report generation agent configured to create comprehensive and tailored summary reports and preparation documents for critical moments in one's health journey (e.g., initial diagnostic appointment, first day of treatment, last day of treatment). In some embodiments, the destination agents include an early warning agent configured to passively monitor patients, flag abnormalities and risks without their direct intervention, and surface warnings to patients and caregivers.

In some embodiments, the architecture shown in Figure 5B is configured to retrieve health data. For example, connect directly with various healthcare institutions to upload, organize, and manage health records, such as visit summaries, scans, and molecular tests. This may include storage and sharing of images such as CT scans, MRIs, and PET scans. In some embodiments, the architecture is configured to create a comprehensive health profile. For example, the platform creates a singular personal profile that aggregates and structures core health information that can be downloaded and shared with clinician. In some embodiments, the architecture is configured to prepare users for upcoming visits. For example, the system may compile relevant health information, documents, and key questions that can help facilitate more productive conversations. In some embodiments, the architecture is configured to monitor user health. For example, track mood, symptoms, medications, and more to highlight key trends. In some embodiments, the architecture is configured to allow users to quickly search their health record, highlight key information, receive tailored research summaries and trial options, and take actions like logging a mood with their assistant.

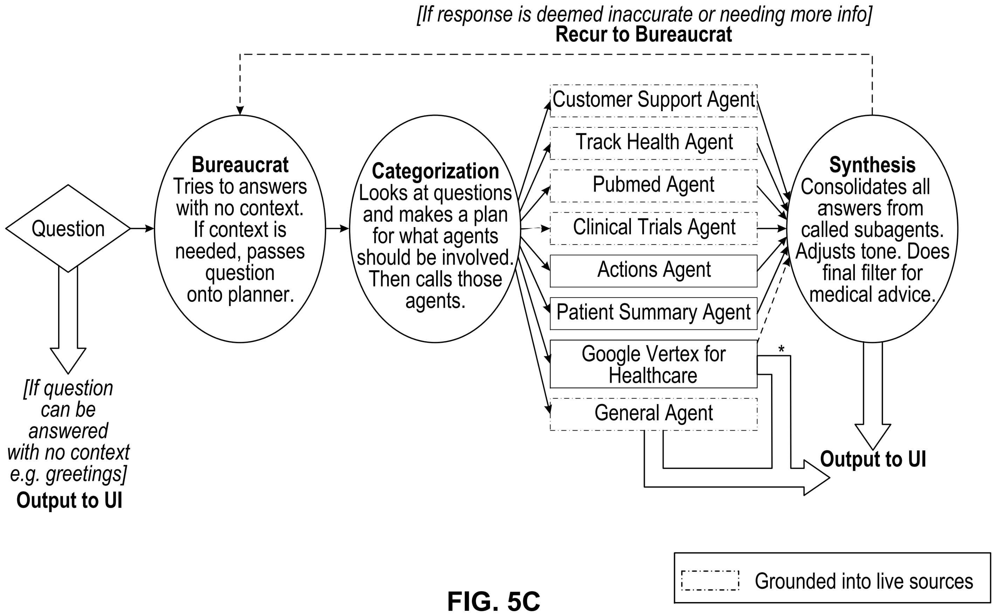

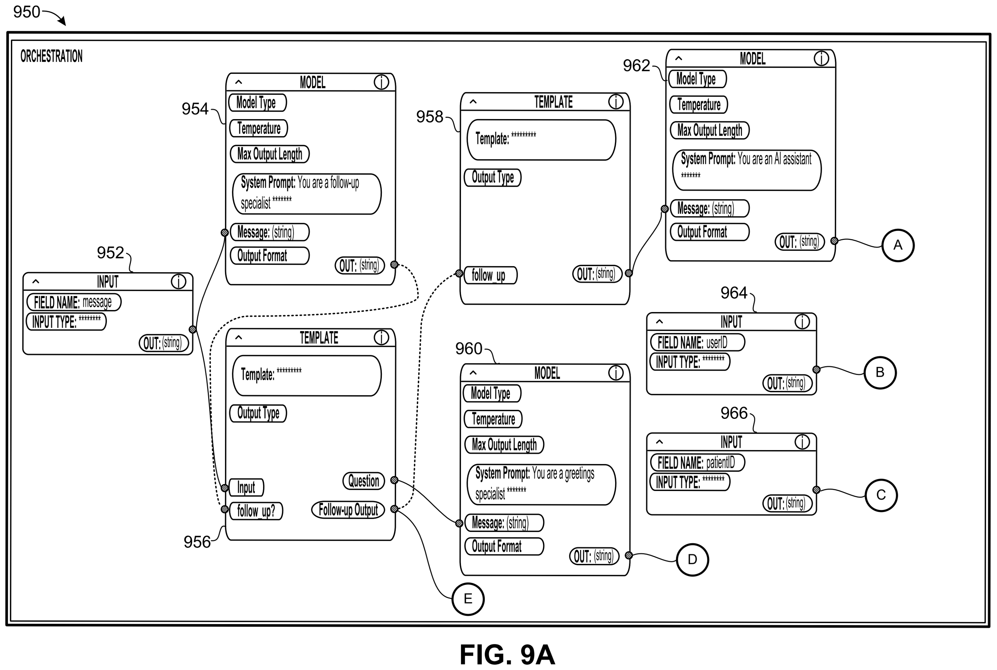

Figure 5C illustrates another example architecture for responding to user queries (e.g., an AI assistant) in accordance with some embodiments. The architecture may be referred to as a chatbot or chatbot architecture. In some embodiments, the AI assistant architecture may be configured to provide information about a user's health records, provide a timeline view and/or summaries of treatments and procedures, provide answers to general health questions, and/or other functions. In the example of Figure 5C, a user query is provided to a bureaucrat orchestration (e.g., a general inquiry agent) that is configured to respond to queries that do not require context. If context is required (or recommended), then the bureaucrat orchestration passes the query to a categorization component (e.g., a planner agent). In some embodiments, the bureaucrat orchestration determines that context is needed when a confidence level of a response without context is less than a threshold level. In some embodiments, the bureaucrat orchestration comprises a super agent or other type of master agent. In some embodiments, the bureaucrat orchestration comprises a main agent that houses the main entry and exit points for the data involved in each exchange with the AI assistant (e.g., chatbot). In some embodiments, the bureaucrat orchestration checks whether the user query is a follow-up, a greeting, or other phatic expression. In some embodiments, the bureaucrat orchestration passes non-phatic expression inputs to the categorization component. In some embodiments, the bureaucrat orchestration passes outputs from the categorization orchestration to a synthesis orchestration (e.g., for response formulation and/or formatting). In some embodiments, the bureaucrat orchestration returns the output (e.g., answer plus indication of sources) from the synthesis orchestration to a user (e.g., via an API layer). Figures 9A-9C illustrate an example architecture for the bureaucrat orchestration.

The categorization orchestration may classify an input as related to one of: (i) a user-related inquiry (e.g., general patient health and/or tracking a specific metric), (ii) clinical trials, (iii) medical research, (iv) medical advice, (v) general medical, (vi) general non-medical, (vii) customer support, and (viii) performing an action (e.g., logging a mood). In some embodiments, the categorization component is provided with a set of predefined categories (e.g., the above categories and/or other categories) and categorizes each query into one of the set of predefined categories. In some embodiments, each predefined category corresponds to a particular agent (e.g., trained on a particular subject and/or topic). In some embodiments, the categorization orchestration is an agent responsible for classifying the input and collecting the data required to best respond. In some embodiments, the categorization orchestration fetches patient health information (e.g., via one or more APIs) if required by specific category. In some embodiments, the categorization orchestration is configured to relay input and any additional information to an appropriate downstream agent (e.g., a sub-agent). In some embodiments, actions are handled in an action flow (e.g., completely handled by the API logic). In some embodiments, the categorization orchestration is configured to return the corresponding response (e.g., answer and sources) for the category to the bureaucrat component (e.g., a master agent). The categorization orchestration may be communicatively coupled to a variety of different downstream agents, such as the customer support agent, the track health agent, the pubmed agent, the clinical trials agent, the actions agent, the patient summary agent, the general agent, one or more third-party agents, and/or other agent types. The customer support agent may be configured to provide answers about what the AI assistant is able to do and assist a user with basic troubleshooting. The pubmed agent may be configured to fetch and/or update scientific research. The clinical trials agent may be configured to return relevant trials, e.g., based on patient demographics. The track health agent may be configured to answer and/or summarize questions about self-reported health metrics (e.g., medications, weight, temperature, mood, and the like). The patient summary agent may be configured to answer user-specific questions, e.g., with the context of the user's patient summary information. A healthcare agent may be configured to answer user-specific questions, e.g., that require additional context beyond the user's patient summary information. The general agent may be configured to answer general medical questions, e.g., that do not require user health information.

As mentioned previously, each agent may be trained and/or prompted to perform a particular task that relates to one of a set of predefined categories. In some embodiments, any medical response is filtered/restricted to ensure that the AI assistant architecture does not give risky medical advice. In some embodiments, an evaluation component is used to determine whether each response complies with any appropriate guidelines and/or rules. For example, if a user asks how to know if their medication is working, the AI assistant may respond that it cannot provide medical recommendations, but can give information about how to monitor medications. As another example, the evaluation component may be configured to ensure that responses are limited to relevant fields (e.g., medical fields). In some embodiments, the evaluation component is configured to determine whether a query is dangerous (e.g., related to self-harm or violence) and prohibit responses to such queries.

The synthesis orchestration may agent takes the data from the other orchestrations and/or components and generates with a well-rounded response to the user input, along with sources and, optionally, further questions that the user may pose to the chatbot. For example, the synthesis orchestration may pass on the user's input and all the data collected, to a model block (e.g., an LLM) to synthesize a well-rounded response. As another example, the synthesis orchestration may pass on the user's input and all the data collected, to a model block (e.g., the same or different model than the model used to generate the well-rounded response) to suggest possible next question(s). As another example, the synthesis orchestration may pass on the outputs of the above step (e.g., answer, sources, and/or suggested questions) to another agent (e.g., the bureaucrat orchestration, a master agent, or other component) to output to the user/client.

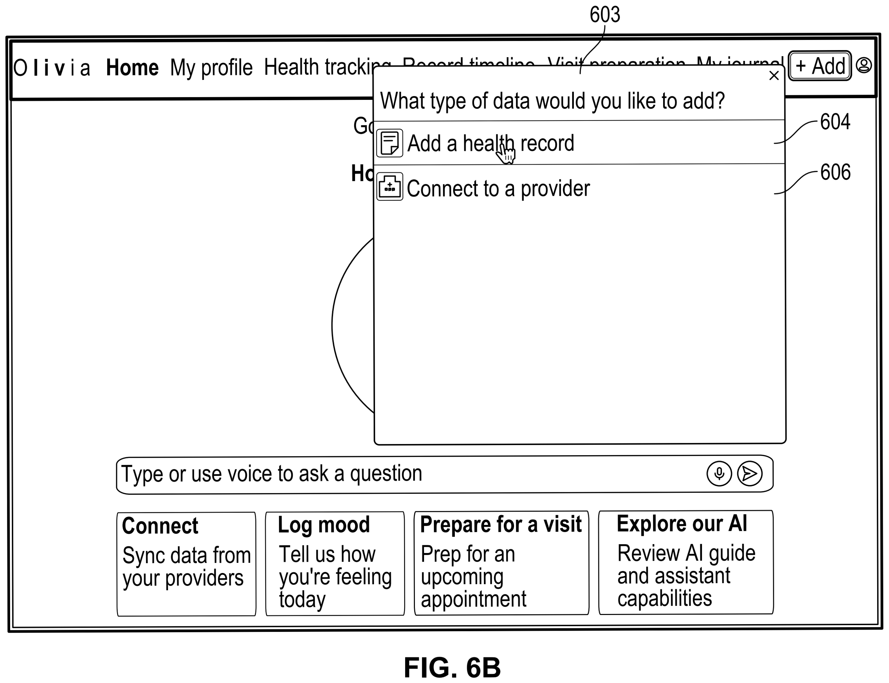

In some embodiments, the AI assistant architecture is configured to return source documents (and/or links to source documents) for each response so that the user may ensure that the response is well-supported and/or accurate. In some embodiments, the AI assistant architecture is configured to provide detailed error information and/or instructions (e.g., to assist users with understanding the breadth of approved questions). In some embodiments, the AI assistant architecture includes one or more interfaces for providing user data and/or medical data (e.g., as illustrated in Figure 6B). In some embodiments, AI assistant architecture provides a summary overview of the user (e.g., with comprehensive health summary, listing of current and/or past treatments, and/or care team members). In some embodiments, the AI assistant architecture provides a health summary such as the user's demographics, diagnoses, treatment history, and the like. In some embodiments, the AI assistant architecture provides current and past treatment and/or medication information, such as medication name, data ranges, dosages, and any related notes. In some embodiments, the AI assistant architecture is configured to communicatively couple with one or more health tracking devices (e.g., fitness bands). In some embodiments, the AI assistant architecture is configured to prepare a user for an upcoming medical visit or procedure. For example, indicating upcoming appointments, provider information, location and time information, and any necessary/recommend preparations. Example user queries topics include side effects, fasting information, diagnoses, medications, test results, appointment preparations, and/or other related topics. In some embodiments, the AI assistant architecture is configured to answer questions and/or summarize medical documents.

As shown in Figure 5C, the AI assistant architecture may include three orchestrations: a bureaucrat orchestration, a planner orchestration, and a synthesis orchestration. In addition, the AI assistant architecture may include, or be coupled to, a set of subagents. For example, the bureaucrat determines if a question is simple (e.g., greetings) or complex, requiring further information. If the latter, the question proceeds through the other blocks, fetching relevant data and synthesizing responses for tone and scanning for safety. The synthesis block may be configured to ensure a patient-friendly tone, and that medical advice is not provided.

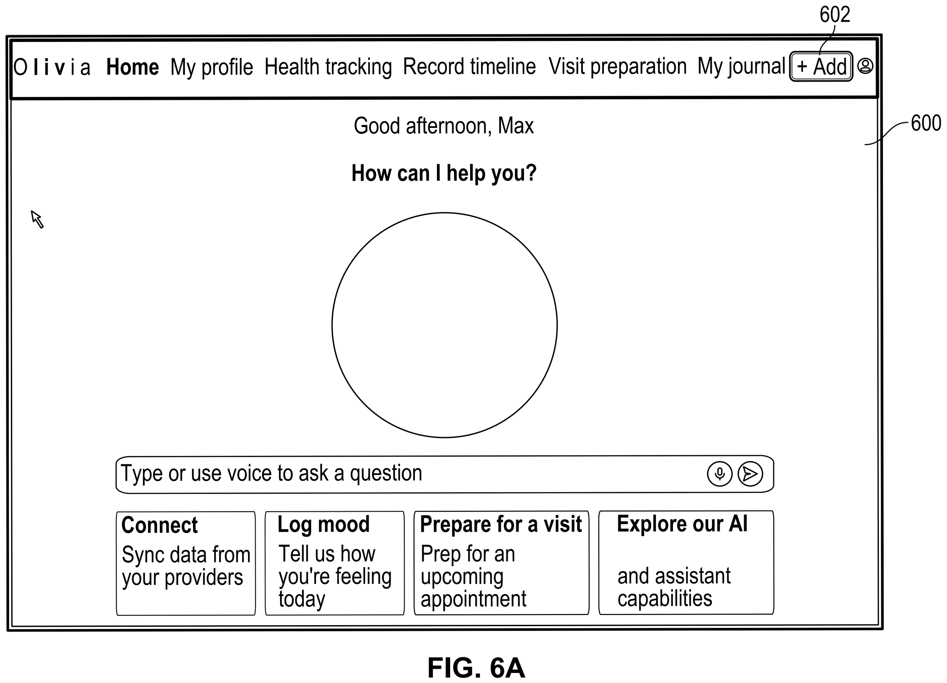

Figures 6A-6AF illustrate example user interfaces and interactions for obtaining, analyzing, and performing actions based on subject data in accordance with some embodiments. In some embodiments, the user interfaces in Figures 6A-6AF are generated by the platform 100 (e.g., and correspond to the architecture shown in Figure 5B).

Figure 6A illustrates an example user interface 600 corresponding to a digital assistant platform, e.g., employing a digital assistant to interact with a user. In some embodiments, the digital assistant is an AI digital assistant composed of a plurality of task-specific orchestrations (e.g., one or more of the agents shown in Figure 5B). The user interface 600 includes a user interface element 602 to provide information to the digital assistant platform.

Figure 6B illustrates a user interface (e.g., a menu) 603 being displayed, e.g., in response to an activation of the user interface element 602. The user interface 603 includes a user interface element 604 to add a health record (or other patient document, digital image, or digital video) to the digital assistant platform, and a user interface element 606 to connect to a provider (e.g., to obtain medical/patient documents from the provider and/or send information to the provider).

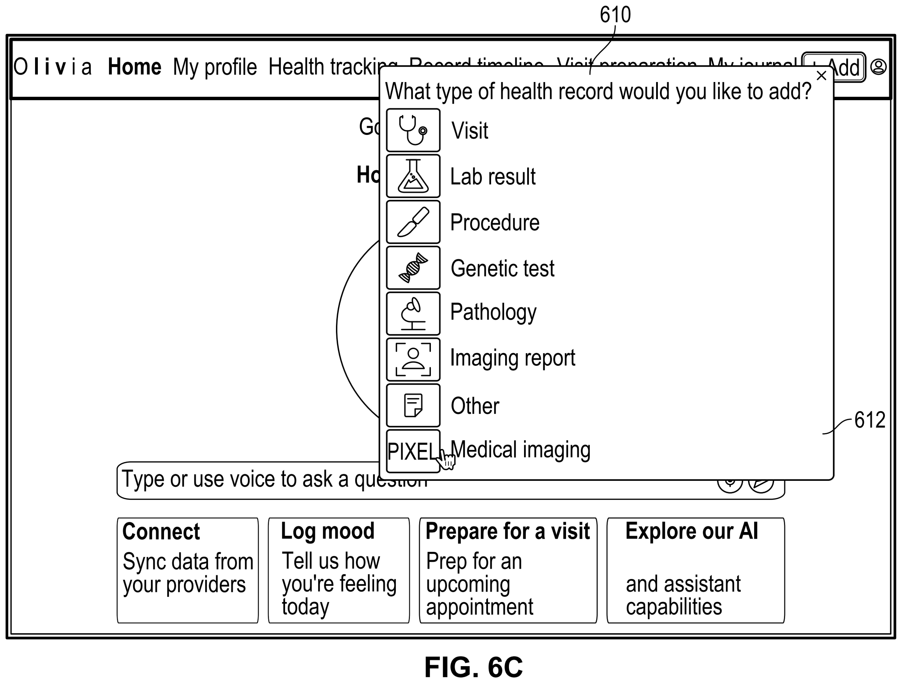

Figure 6C illustrates a user interface (e.g., a menu) 610 being displayed, e.g., in response to an activation of the user interface element 604 in Figure 6B. The user interface 610 includes selectable options corresponding to different types of health records (e.g., visit documentation, lab results, procedure documentation, genetic test data, pathology data, imaging reports, medical imaging, cardiovascular imaging, and the like). In the example of Figure 6C, a user interface element 612 corresponding to medical imaging is selected.

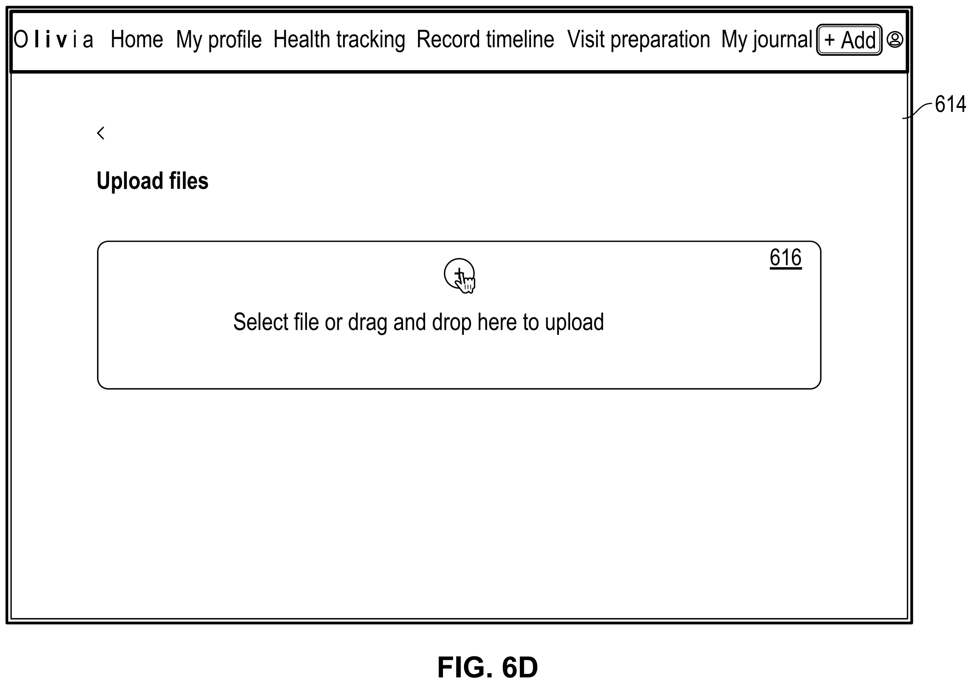

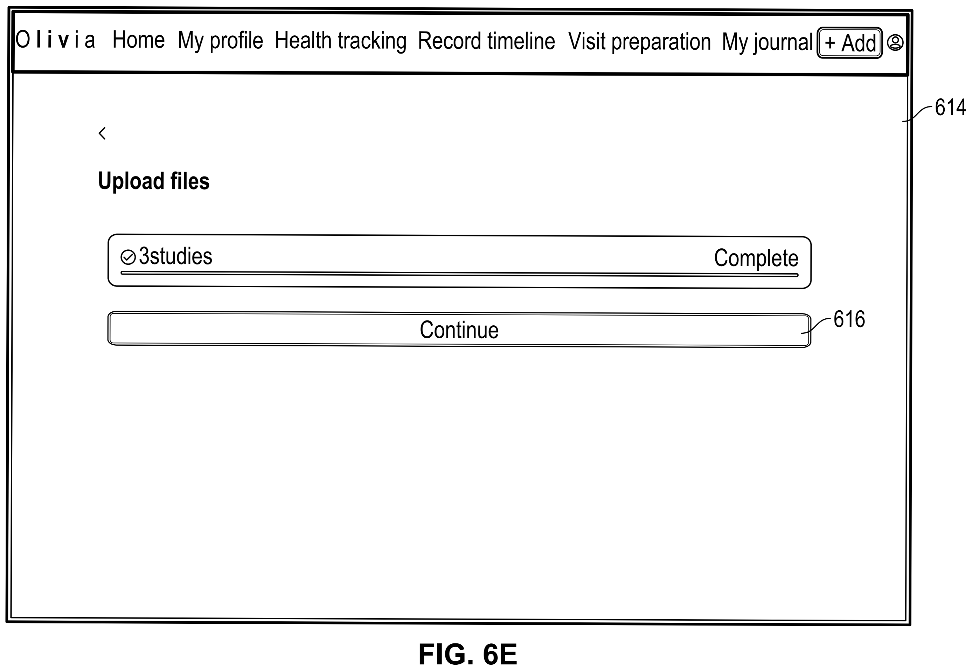

Figure 6D illustrates a user interface (e.g., a menu) 614 being displayed, e.g., in response to an activation of the user interface element 612 in Figure 6C. The user interface 614 includes a user interface element 616 to upload files (e.g., medical imaging and/or video files). Figure 6E illustrates the user interface 614 being updated as compared to Figure 6C in response to a set of one or more files (e.g., denoted "3studies") being uploaded. The user interface 615 in Figure 6E includes a user interface element 616 to proceed with importing the uploaded set of files.

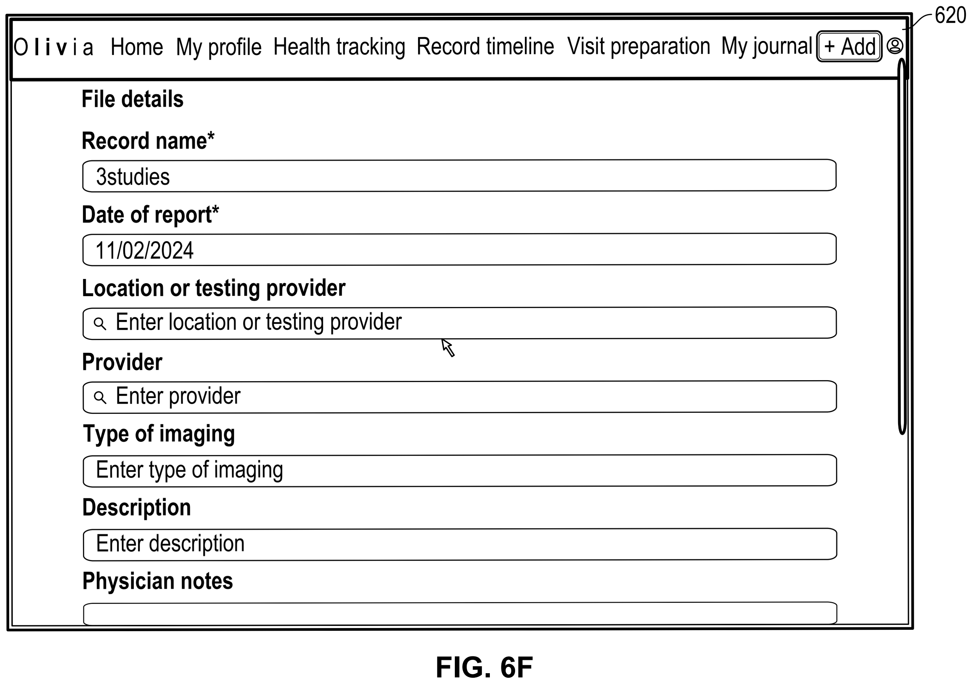

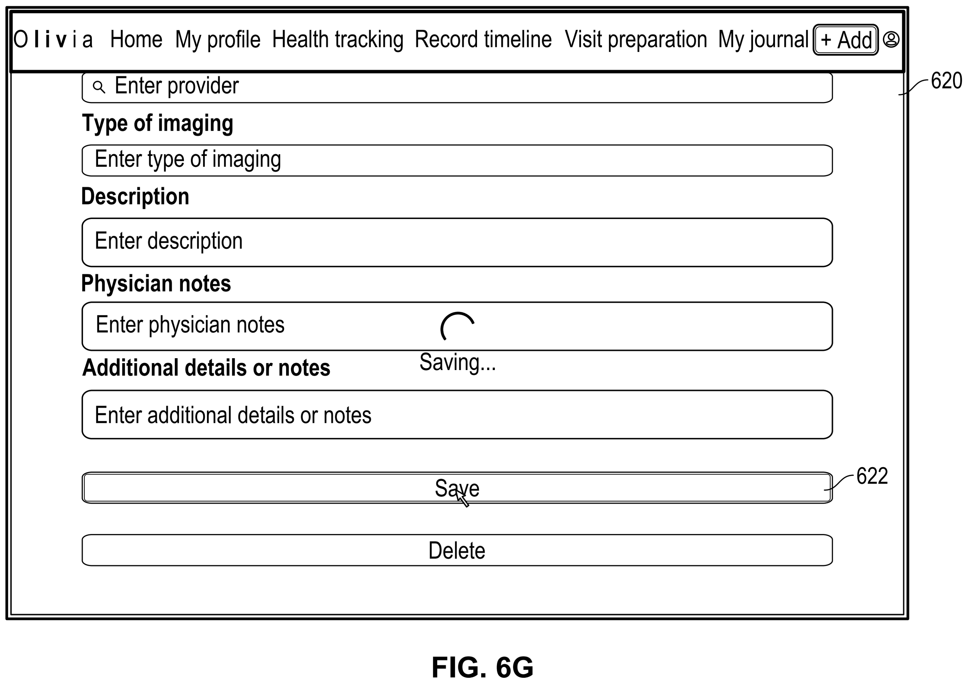

Figure 6F illustrates a portion of a user interface 620 being displayed, e.g., in response to an activation of the user interface element 616 in Figure 6E. The user interface 620 includes a set of fields for importing data about the set of files being uploaded (e.g., name, date, location, provider, etc.). In some embodiments, one or more of the fields are automatically populated (e.g., using information from a user profile of the user and/or information from the set of files (e.g., metadata for the set of files)). Figure 6G illustrates a lower portion of the user interface 620 (e.g., in response to a user scrolling downward from Figure 6F) that includes a user interface element 622 to proceed with importing the set of files (and any information input into the user interface 620). Figure 6G further shows the set of files being imported (saved) in response to selection of the user interface element 622.

In some embodiments, importing the set of files includes parsing the files to extract structured information (e.g., medical data). For example, a medical ontology may be applied to an electronic health record to extract at least one structured field value for the health record. Medical data may include numerous fields including, but not limited to, patient demographics (e.g., patient name, date of birth, gender, ethnicity, date of death, address, smoking status, diagnosis dates, personal medical history, family medical history, etc.), clinical diagnoses (e.g., date of initial diagnosis, date of metastatic diagnosis, cancer staging, tumor characterization, tissue of origin, etc.), treatments and outcomes (e.g., therapy groups, medications, surgeries, radiotherapy, imaging, adverse effects, associated outcomes, and corresponding dates, etc.), and genetic testing and laboratory information (e.g. genetic testing, performance scores, lab tests, pathology results, prognostic indicators, and corresponding dates, etc.). Each of the fields (e.g., address, cancer staging, medications, genetic testing, etc.) may also have a plurality of subfields. For example, address may have subfields for type of use (e.g., personal, business), street, city, state, zip, country, and a start or end date (i.e., date that residency at the address begins or expires). Genetic testing may have subfields for the date of genetic testing, testing provider used, test method (e.g., genetic sequencing method, gene panel), gene results (e.g., included genes, variants, expressions, etc.), tumor mutational burden, and microsatellite instability. The above provided examples, enumerations, and lists are not intended to limit the scope of the available fields and are intended to convey only the nature and structure that fields within medical data may be represented within a universal EMR. These fields of medical data may also identify concept candidates, such as: Diagnosis, Primary Diagnosis Site, Metastatic Diagnosis Site, Tumor Characterization, Standard Grade, Alternative Grade, Medications, Surgical Procedure, Smoking Status, Comorbidities, Adverse Events, Outcomes, Performance Scores, Radiotherapy Modality, Radiotherapy Units, Imaging Type, Gene Mention, Immunology Markers, TNM Status, and American Joint Committee on Cancer (AJCC) Stage.

Feature collections may include a diverse set of fields available within patient health records. Clinical information may be based upon fields which have been entered into an EMR or an EHR by a physician, nurse, or other medical professional or representative. Other clinical information may be curated from other sources, such as molecular fields from genetic sequencing reports. Sequencing may include next-generation sequencing (NGS) and may be long-read, short-read, or other forms of sequencing a patient's somatic and/or normal genome. A comprehensive collection of features in additional feature modules may combine a variety of features together across varying fields of medicine which may include diagnoses, responses to treatment regimens, genetic profiles, clinical and phenotypic characteristics, and/or other medical, geographic, demographic, clinical, molecular, or genetic features. For example, a subset of features may comprise molecular data features, such as features derived from an RNA feature module or a DNA feature module sequencing.

Features may be derived from information from additional medical or research based Omics fields including proteome, transcriptome, epigenome, metabolome, microbiome, and other multi-omic fields. Features derived from an organoid modeling lab may include the DNA and RNA sequencing information germane to each organoid and results from treatments applied to those organoids. Features derived from imaging data may further include reports associated with a stained slide, size of tumor, tumor size differentials over time including treatments during the period of change, as well as machine learning approaches for classifying PDL1 status, HLA status, or other characteristics from imaging data. Other features may include the additional derivative features sets from other machine learning approaches based at least in part on combinations of any new features and/or those listed above. For example, imaging results may need to be combined with MSI calculations derived from RNA expressions to determine additional further imaging features. In another example a machine learning model may generate a likelihood that a patient's cancer will metastasize to a particular organ or a patient's future probability of metastasis to yet another organ in the body. Other features that may be extracted from medical information may also be used. There are many thousands of features, and the above listing of types of features are merely representative and should not be construed as a complete listing of features.

Fuzzy matching is structured around the text concepts included in the above enumerated list or the UMLS, including metadata fields CUI (the UMLS unique ID) and AUI (dictionary-specific unique ID), so that an exhaustive search may be performed for all medical concepts. The dictionary search engine may also return metadata about the specific entry detected (e.g., universal ID assigned in the above enumerated list or the UMLS), which is useful for understanding Tylenol as a medical concept and not just the correct spelling of a drug. At the end of text normalization, some of the extracted candidates may have zero matches but others may have many matches. For example, there are many versions of Tylenol throughout the UMLS database because of the number of dictionaries represented therein. Fortunately, the CUI (the UMLS uuid) provides a generalization to join similar concepts, which reduces the number of matches from one for each potential database to the number of unique CUIs represented. Not all concepts can be simplified so succinctly, though. For example, "Tylenol" is a different concept than "Tylenol 50 mg", which is a dosage-specific version of "Tylenol". Any ambiguation from "Tylenol 50 mg" to "Tylenol" would effectively constitute a loss of information.

Fuzzy matching may also apply on a word-by-word basis rather than a letter-by-letter basis. For example, a concept candidate may include the phrase "needle biopsy." An entity matching search may identify entities linked to, for example an exact match ("needle biopsy"), a reordered match ("biopsy needle"), or phrase matches of "needle aspiration biopsy of lung" or "breast needle biopsy." Such entity matches may be derived using the same fuzzy matching operations above (deletion, insertion, transcription, etc.), but on the whole word rather than each individual character. Furthermore, in another embodiment, both fuzzy matching on a character-by-character basis and word-by-word basis may be applied concurrently to generate entity matches. When operating as an ontology-directed system that specializes in abstracting patient from fields from a particular ontology, dictionary lookups may be relaxed to allow for wider inferences. This may include allowing more wildcards into the phrase search or other adjustments to allow for broader lookups. When operating as a system having aspects that are ontology agnostic for performing recognition across multi-discipline medical records, dictionary lookups may be stricter, allowing less flexibility or wild characters for matching.

Various fields, e.g., UMLS_CUI, UMLS_AUI, Medication, and Active Ingredient may each be determined through the entity normalization process by exploring the links to each of the Entities. The other fields, e.g., dosage, dosage units, date/time administered, document, and page may not be determined through the normalization process. Instead, these other fields are provided to a Relational Extraction MLA for extracting this information from the surrounding context or document information (e.g., name, number of pages, etc.). For example, a document named Progress Note 01_01_01 may be presumed to have a date of Jan. 1, 2001. Other concept candidates from the document may be referenced to validate the date/time or select the date absent any other validating/corroborating information. For example, the time 11:35 am may have been provided as a concept candidate spatially near the "Tylenol 50 mg" concept candidate. The Relational Extraction MLA may then identify 11:35 am as the time the medication was administered based on the concept candidate time being the next concept candidate in the list, a spatial proximity of the concept candidate, a new application of NLP to the OCRed text string, or any combination thereof. Additionally, a page number may be identified, for example, in a document that has 5 pages by referencing the page number by performing an OCR of text at the bottom of the page or may be extrapolated by counting the number of pages before the page the concept candidate was extracted from. Once each field of the medical data is identified through either the normalization process or the structuring process and the relational extraction MLA, the patient/document may be ready to be classified according to each normalized and structured entity.

In some embodiments, the electronic document capture may reference the predefined model to identify the region of the electronic document capture containing key health information and extract the identified region for further processing. A region (such as a header, table, graphic, or chart) may be identified by utilizing a stored feature list for the document, or each page of the document, that identifies features present in the page along with their corresponding locations in the page. For example, the model may indicate that a page should expect to present a patient header, two tables, a chart, and a graph. A region mask may be applied to the capture to verify that any regions expected to be present are actually within the capture. A region to extract may be identified by the region mask and, upon verifying that the region is present, the region may be extracted. Text may be identified from the extracted region and provided to a natural language processing (NLP) algorithm to extract patient information, such as patient name, diagnosis, notable genetic mutations, or gene expression count information including count values representing the number of times the RNA sequence occurs in sequencing and/or deviation in gene expression counts compared to the gene expression counts of normal samples that may be labeled over or under expressed. More details for feature/region detection and extraction are discussed below with respect to tabular extraction.

Each field of the extracted region may have a plurality of enumerated values, or, if an enumerated list of values is unavailable may be limited to a certain type of value. For example, if the field relates to patient diagnosis, it may have a corresponding enumerated list of all diagnoses that may be provided in the report. If the field relates to a treatment, it may have all known treatments and further parse the field to identify and enumerate unknown treatments. Alternatively, if the field is a medication that the patient was prescribed, it may be limited to a type, so that data parsed will be checked against known medications and, if necessary, add the parsed text to a medications database as a new entry of type medications. The types of field, their enumerated values, or the classification associated with unknown values may be stored as part of the predefined model.

As an example, a natural language processing (NLP) algorithm may receive as an input, a region of an electronic document capture which has had optical character recognition (OCR) performed on the extracted region to identify if the text of the region corresponds to the value type expected in that extracted region where the mask was applied. For example, if the region was expected to provide treatment information, the NLP algorithm may attempt to classify the extracted text as treatment information based off of the NLP algorithm training data set. Furthermore, the patient information being extracted, such as patient name, diagnosis, treatment, or sequencing information, may be associated with a respective field in the application. For example, extracted patient information may then be populated into the mobile application for review by the user. The user may correct any errors by selecting the data field in the application corresponding to the information. These errors may be stored and/or sent to a training engine to improve upon the extraction algorithms and techniques. The training engine may generate a new extraction algorithm to use in future extractions based from detected errors. For text-based fields, a text editor/keyboard may be displayed for the user to provide text corrections. Additionally, suggested replacements may also appear in a dropdown list in addition to or in place of the text editor. For date-based fields, a calendar may be displayed to select the correct date, or for diagnosis, a dropdown featuring diagnoses supported by the document type, report type, or even the database may be enumerated for selection. The field types listed above are merely representative and are not intended to limit fields to the specific type of data associated in the above description, for example, date-based fields may also be populated using text input. The predefined model that is associated with each of the templates may contain reference fields to identify each of the fields that may be extracted to generate the extracted patient information. For example, a feature corresponding to the informational header may have a corresponding field in the predefined model which lists a location the patient name is expected, a location the patient date of birth is expected, and a location of the diagnosis (such as cancer type) is expected as described above.

In another example, an MLA may receive as an input, a region of the electronic document capture which has had optical character recognition (OCR) performed on the extracted region to identify if the text of the region corresponds to the value type expected in that extracted region where the mask was applied. For example, if the region was expected to provide treatment information, the MLA may attempt to classify the extracted text as treatment information based off of the MLA training set. Furthermore, the patient information being extracted, such as a patient name, diagnosis, treatment, or sequencing information, may be associated with a respective field in the application. Extracted patient information may then be populated into the mobile user interface for review by the user. Other information that may be extracted may include: gene(s) (such as TP53, NF1, or PDL1); gene expression count information (such as over/under expressed or count values representing the number of times the expression occurs in sequencing); respective gene variants (such as Q192 or E496); gene variant calls (such as "4724+1G>A", "Q192*", or "c.380C>A"); depth of sequencing (such as occurrences of chromosome hits per number of DNA reads); scope of sequencing (such as panel type: whole genome or targeted panels); proteomics (such as protein based assertions: counts and shapes); epigenetics; RNA expressions (such as over-expression or under-expression); organoids (such as chemical/medical responses organoids experienced in a lab setting); germline (such as mutations present in healthy cell DNA); immunotherapies (such as engineered immune receptors such as CAR-T, cancer vaccines, checkpoint blockades, etc.); and tumor-normal (such as a comparison of RNA and/or DNA sequencing results of tumor tissue with RNA and/or DNA sequencing results of a non-tumor sample, such as non-tumor tissue, blood, or saliva). Features of the electronic data capture may also be directed to clinical trials, for example, by listing details associated with the name of the clinical trial, geographic location of the facilities administering the trial, treatments associated with the trial, inclusion/exclusion criteria for patients who may participate in the trial, and other relevant information.

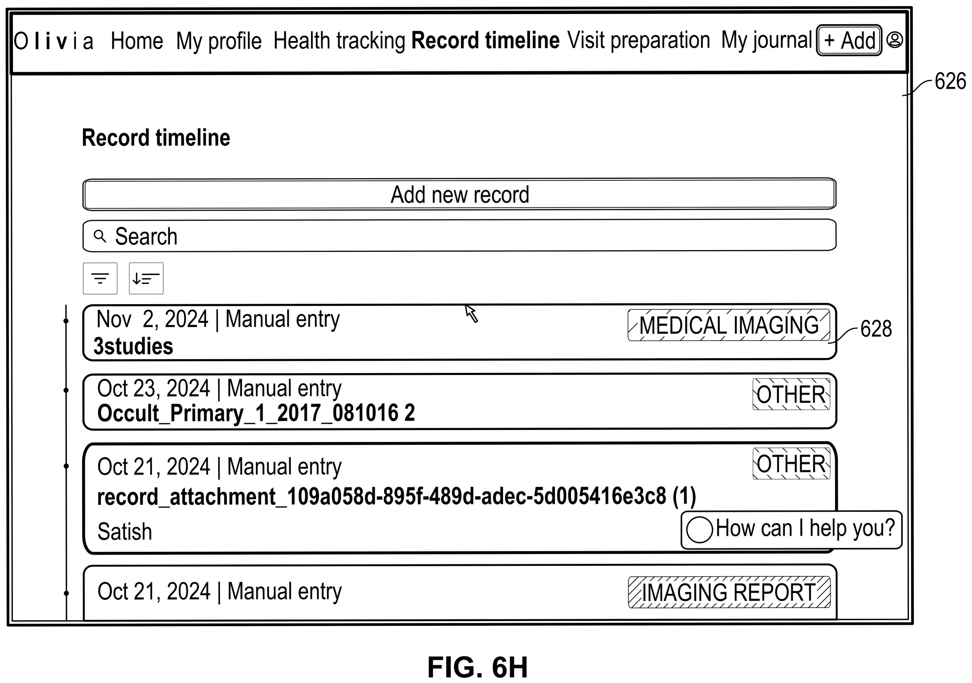

Figure 6H illustrates a user interface 626 with a record timeline for a user (e.g., a patient). The user interface 626 may be displayed in response to the set of files being imported (e.g., a transition from Figure 6G). The user interface 626 may be displayed in response to a user requesting to view the record timeline. The user interface 626 includes a timeline of actions associated with a user record (e.g., importing medical images and other medical documents). The user interface 626 includes a user interface element 628 for viewing details of the medical imaging file(s).

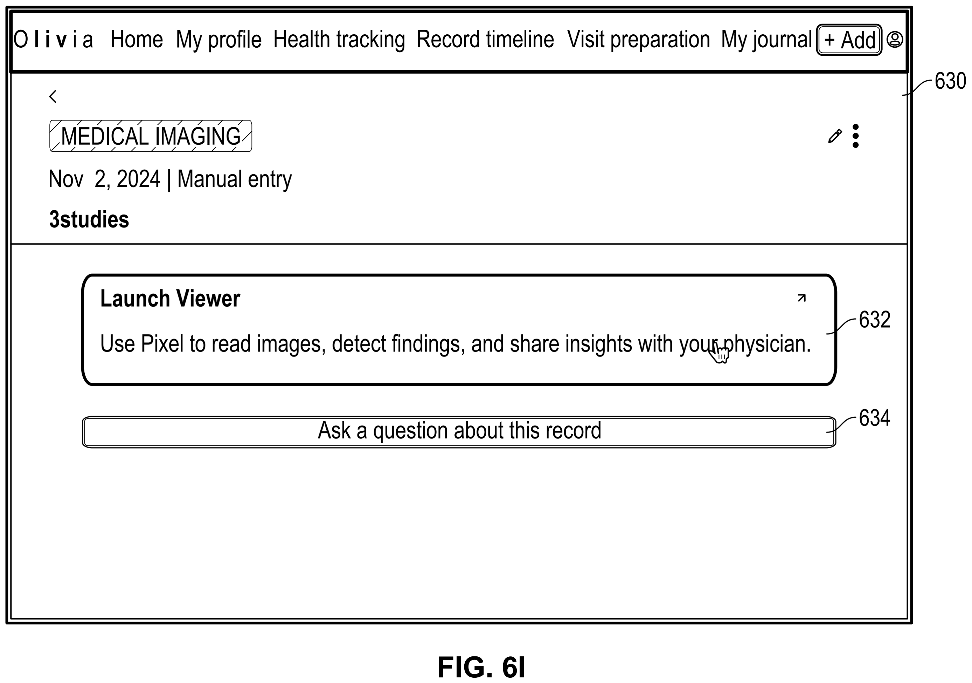

Figure 6I illustrates a user interface 630 being displayed, e.g., in response to an activation of the user interface element 628 in Figure 6H. The user interface 630 includes an option 632 (e.g., user interface element) to view the medical imaging files and an option 634 to ask questions about the medical imaging files.

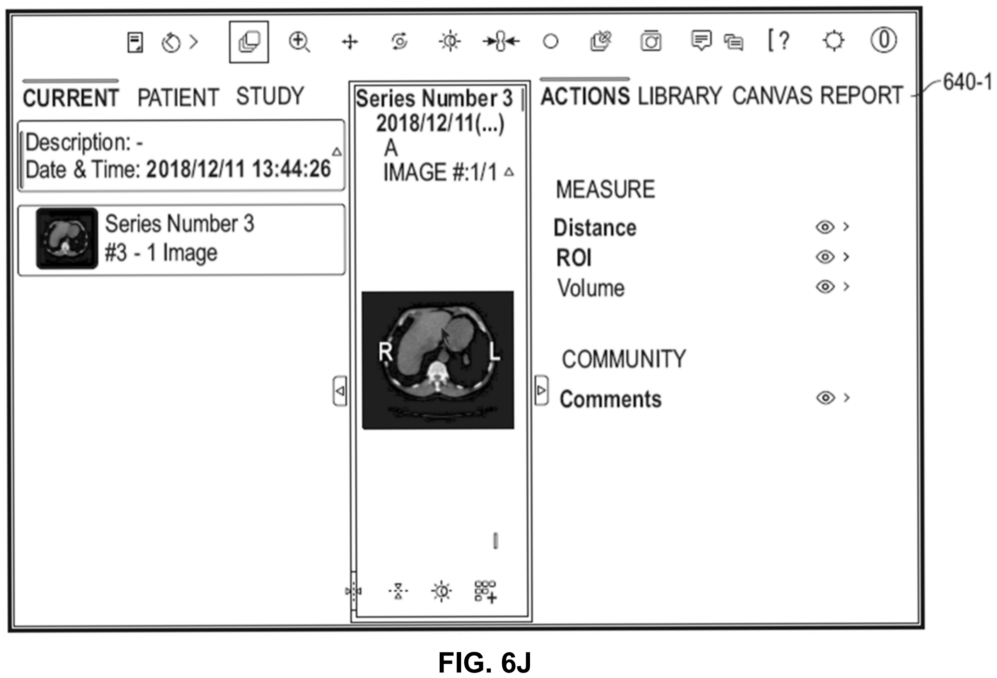

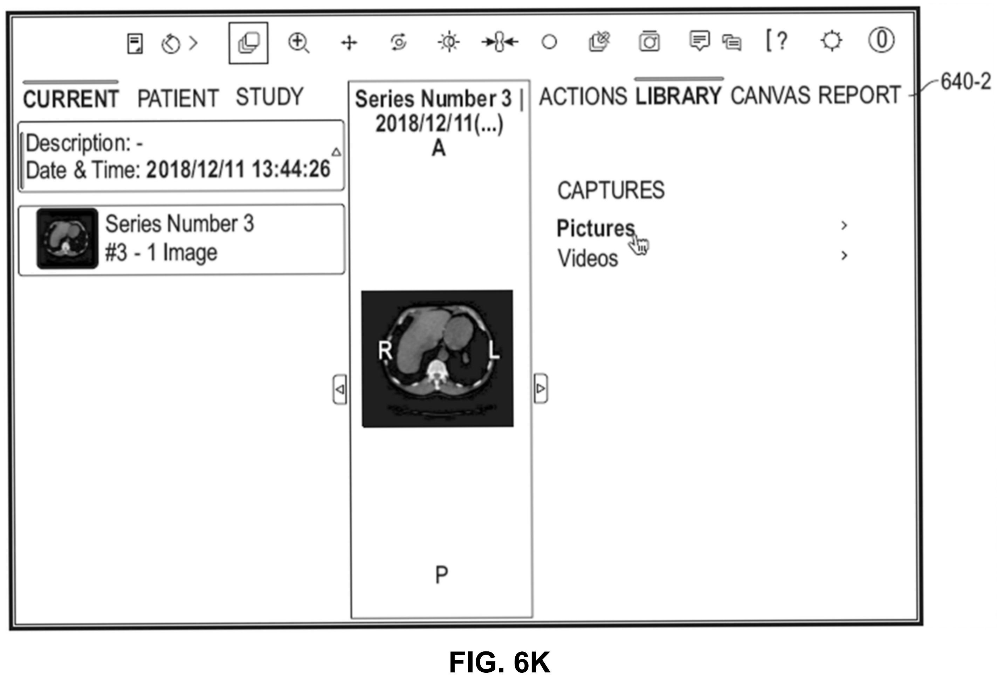

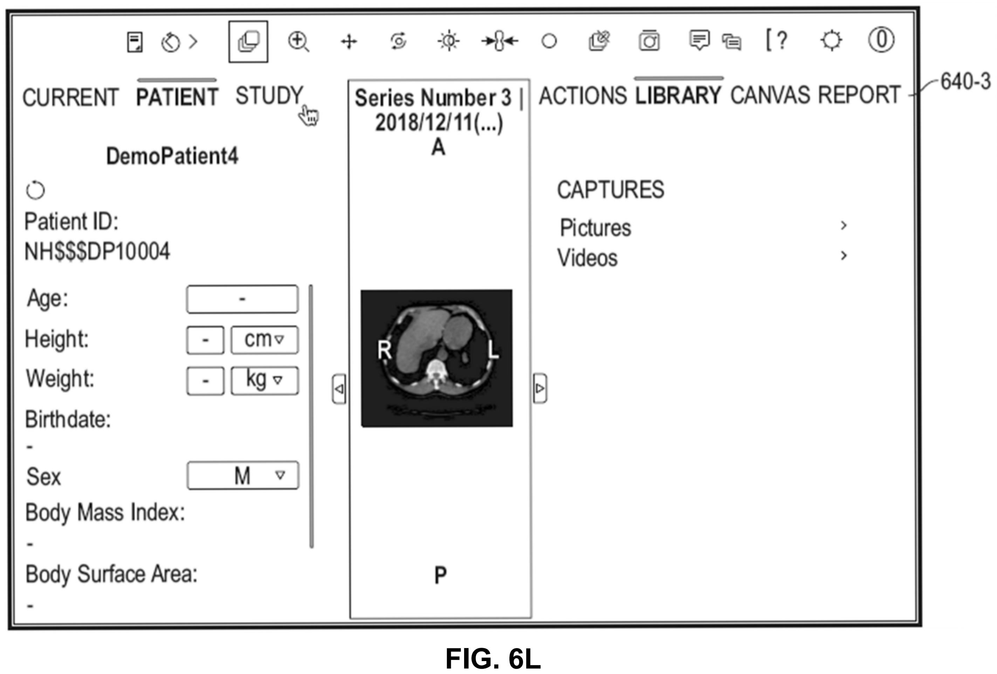

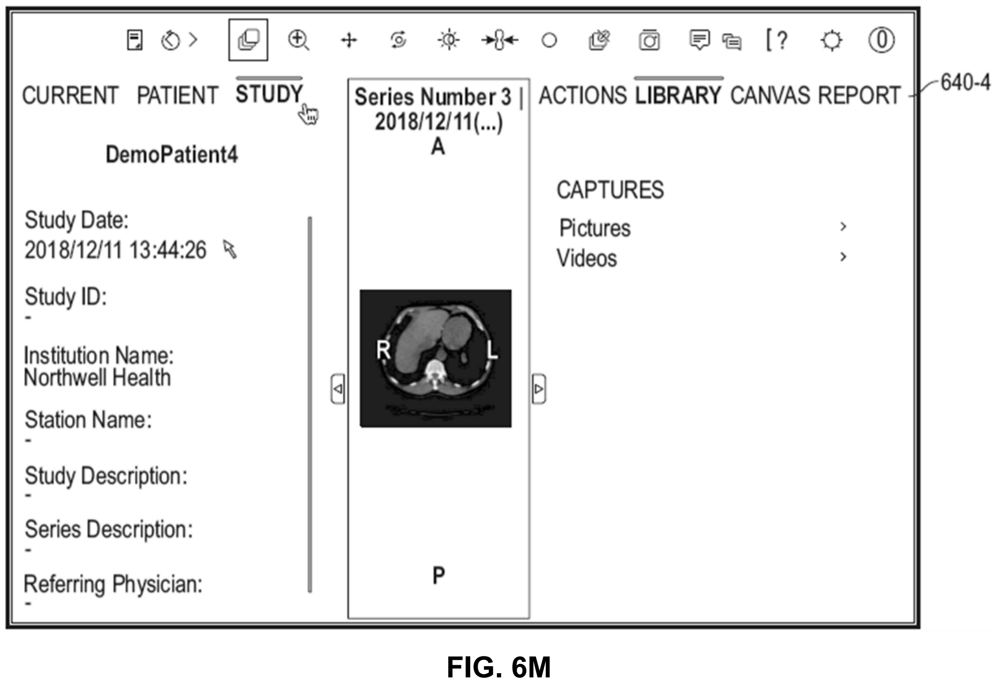

Figure 6J illustrates a user interface 640-1 being displayed, e.g., in response to an activation of the option 632 in Figure 6I. The user interface 640-1 includes the imported images along with patient and context data (and other types of metadata). In Figure 6J, an actions tab is shown within the user interface 640-1, with measurement and community action options, and a current tab is shown with information about the currently displayed image. Figure 6K illustrates a user interface 640-2 being displayed, e.g., the user interface 640-1 with the library tab being activated instead of the actions tab. The library tab includes an index of pictures and/or videos included in the medical imaging files. Figure 6L illustrates a user interface 640-3 being displayed e.g., the user interface 640-1 with the patient tab being activated instead of the current tab. The patient tab in Figure 6L includes fields with information about the patient. In some embodiments, the fields are interactive, and the user may enter/update patient information, e.g., which is stored to metadata associated with the image and/or stored in a health profile for the user. Figure 6M illustrates a user interface 640-4 being displayed e.g., the user interface 640-1 with the study tab being activated instead of the current tab. The study tab in Figure 6M includes fields with information about the study corresponding to image. In some embodiments, the fields are interactive, and the user may enter/update study information.

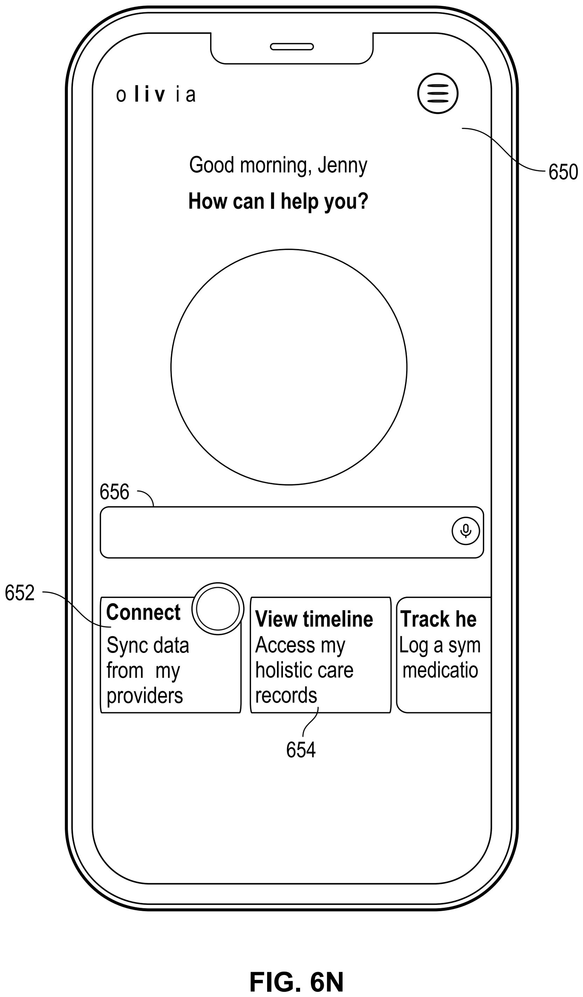

Figure 6N illustrates an example user interface 650 corresponding to a digital assistant platform, e.g., employing a digital assistant to interact with a user. For example, the user interface 650 may be used on small screen devices and the user interface 600 may be used on larger screen devices. In some embodiments, the digital assistant is an AI digital assistant composed of a plurality of task-specific orchestrations (e.g., one or more of the agents shown in Figure 5B). The user interface 650 includes a user interface element 652 to connect the digital assistant platform with a healthcare provider, a user interface element 654 to view a health timeline for the user (e.g., the timeline shown in Figure 6H), and a user interface element 656 to send queries/prompts to the digital assistant.

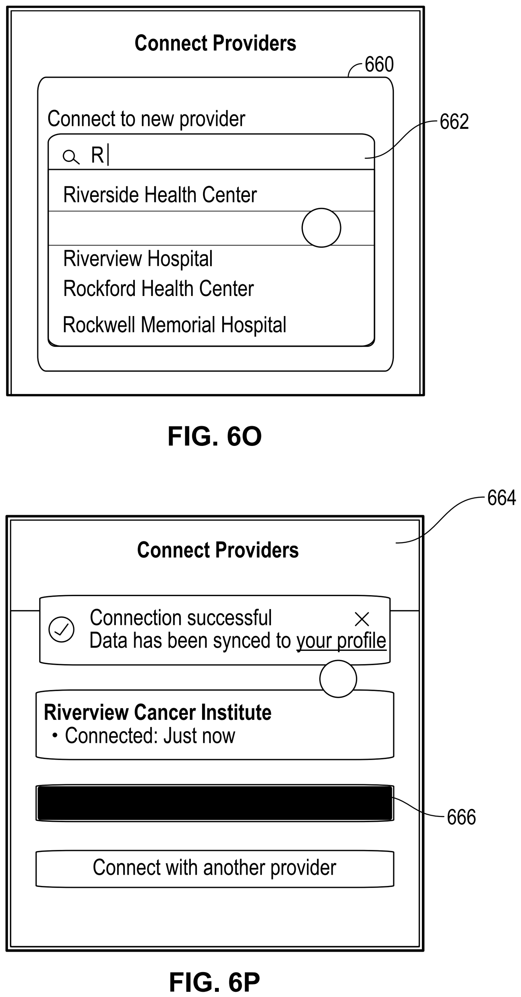

Figure 6O illustrates a user interface (e.g., a menu) 660 being displayed, e.g., in response to an activation of the user interface element 652. The user interface 660 includes a field 662 to input a name of a healthcare provider and a list of potential providers (e.g., identified based on the current input into the field 662). Figure 6P illustrates a user interface 664 being displayed, e.g., in response to selection of a healthcare provider from the list shown in Figure 6O. The user interface 660 indicates that a healthcare provider has been connected (e.g., communicatively coupled to the digital assistant platform) and includes a user interface element 666 to proceed with the connected healthcare provider.

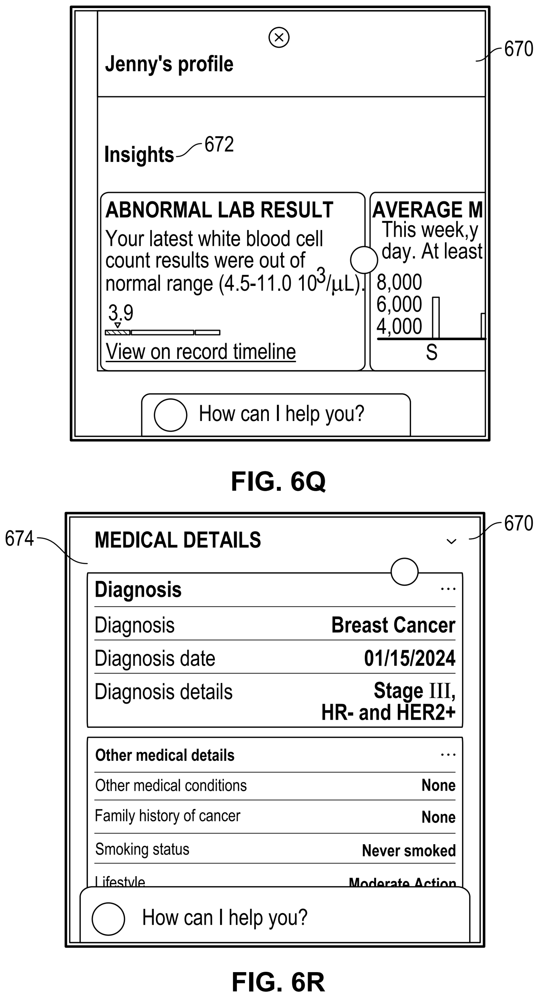

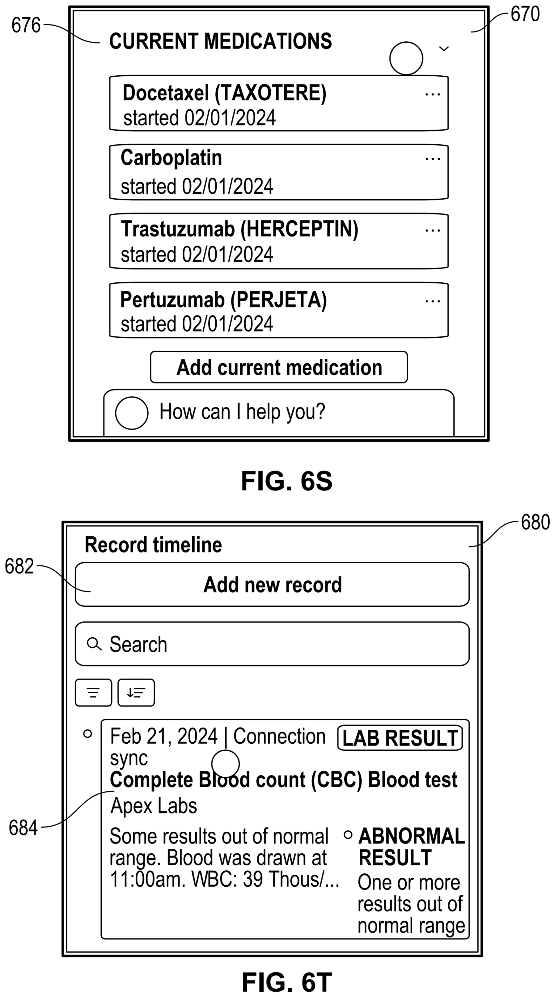

Figure 6Q illustrates a portion of a user interface 670 being displayed, e.g., in response to a request to view a user profile or in response to an activation of the user interface element 666. The user interface 670 includes profile information for the user (e.g., a profile generated based on data from a variety of data sources). In some embodiments, the profile information includes information obtained from the user (e.g., by entering data into fields and/or uploading files) and/or information obtained by communicatively coupling to healthcare provider(s) (e.g., as illustrated in Figures 6O-6P). In the example in Figure 6Q, the portion of the user interface 670 includes insights 672 generated by the platform based on the information of the user profile. Figure 6R illustrates a lower portion of the user interface 670 (e.g., in response to a user scrolling downward from Figure 6Q) that includes medical details 674 (e.g., a summary of medical information available in the digital assistant platform). Figure 6S illustrates a second lower portion of the user interface 670 (e.g., in response to a user scrolling downward from Figure 6R) that includes medication details 676 indicating current medications prescribed to the user.

Figure 6T illustrates an example user interface 680 corresponding to a record timeline for a user (e.g., similar to the user interface 626 in Figure 6H). For example, the user interface 680 may be used on small screen devices (e.g., mobile devices) and the user interface 626 may be used on larger screen devices. The user interface 680 includes a user interface element 682 to import a new record (and add the new record to the timeline), and a timeline item 686 (e.g., corresponding to previously imported information).

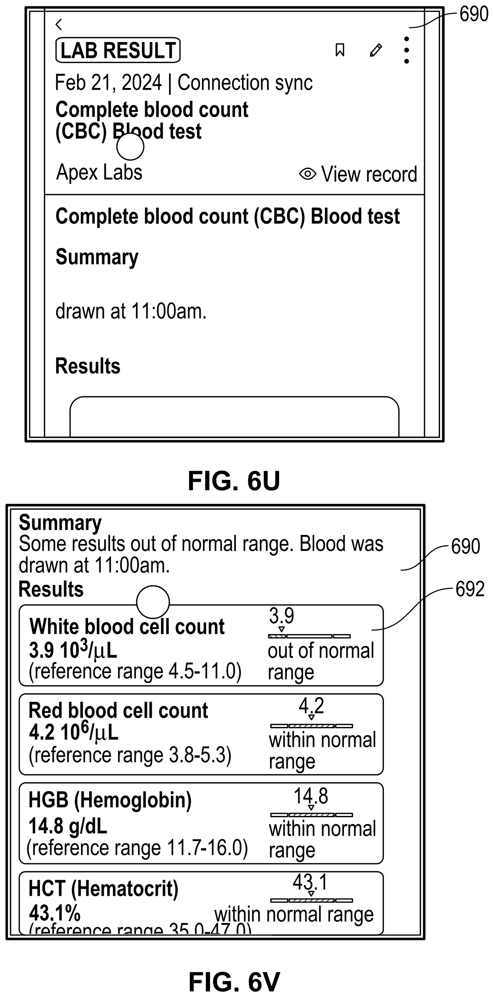

Figure 6U illustrates a portion of a user interface 690 being displayed, e.g., in response to selection of the timeline item 686 in Figure 6T. The user interface 690 includes details about the timeline item 686 (e.g., blood test results), such as a summary of the test results. Figure 6V illustrates a lower portion of the user interface 690 (e.g., in response to a user scrolling downward from Figure 6U) that includes test result details 692. In some embodiments, the test result details 692 include data visualizations generated by the digital assistant platform (e.g., to facilitate conceptualization of the test results).

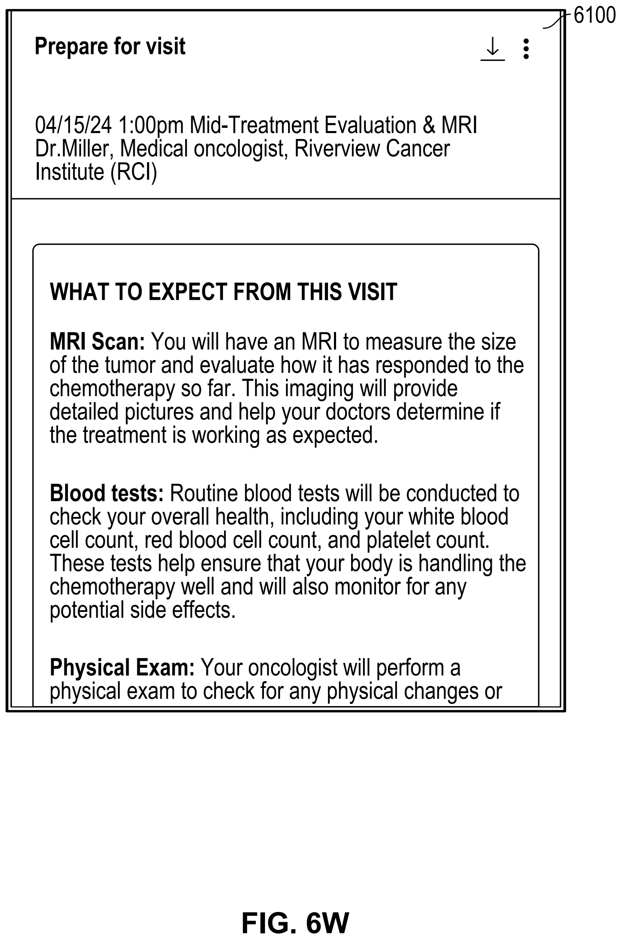

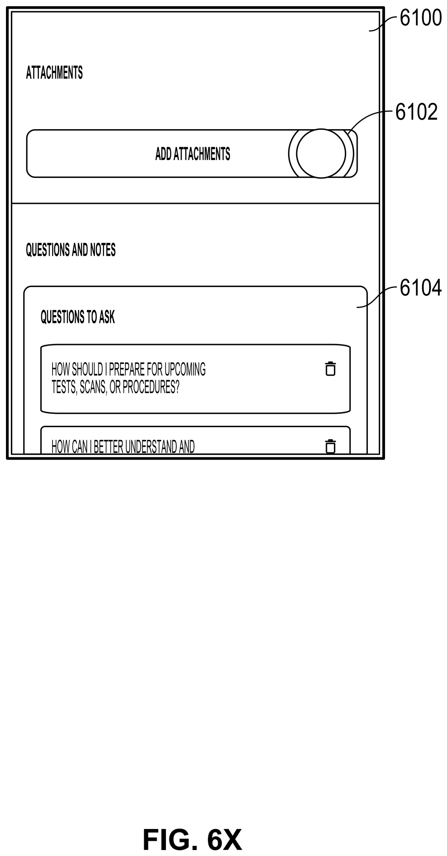

Figure 6W illustrates a portion of a user interface 6100 configured to prepare a user for an upcoming event (e.g., a scheduled medical appointment). The user interface 6100 in Figure 6W includes information regarding what to expect at the upcoming event. Figure 6X illustrates a lower portion of the user interface 6100 (e.g., in response to a user scrolling downward from Figure 6W) that includes a user interface element 6102 for importing attachments related to the upcoming event and a section 6104 including elements with questions and notes.

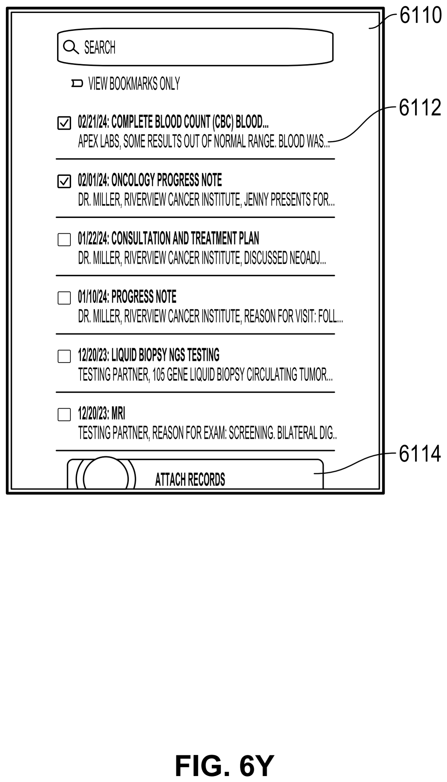

Figure 6Y illustrates a user interface 6110 for associating files/documents to an upcoming event, e.g., in response to a selection of the user interface element 6102 in Figure 6W. The user interface 6110 includes a listing 6112 of documents that may be associated with the upcoming event, a user interface element for searching for appropriate documents, and a user interface element 6114 to associate selected documents with the upcoming event. In some embodiments, documents (e.g., medical documents such as test results, progress notes, treatment plans, and medical images) associated with the user profile may be attached to the upcoming event.

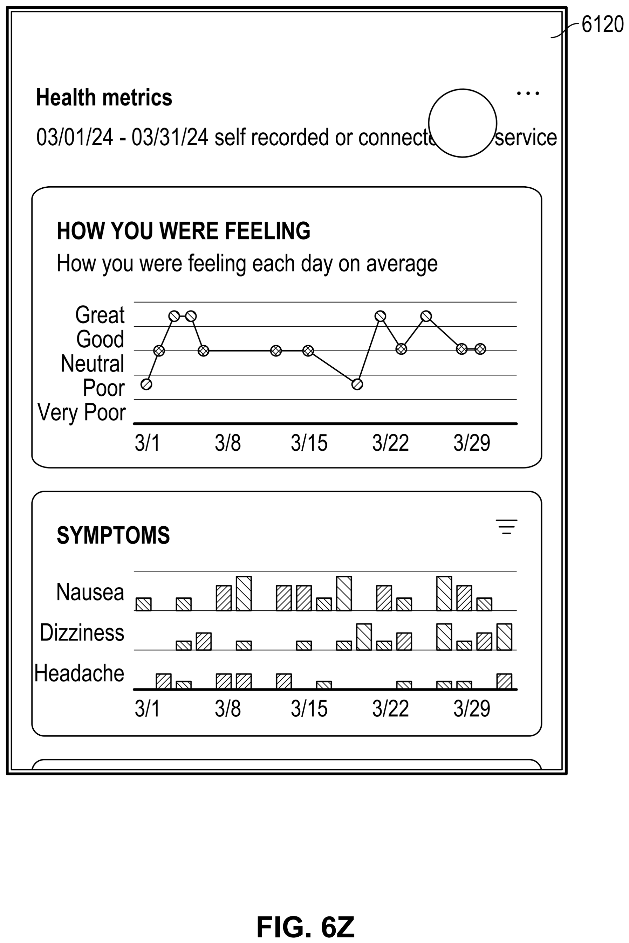

Figure 6Z illustrates a user interface 6120 indicating health metrics for the user. In some embodiments, the digital assistant platform generates data visualizations (e.g., to facilitate conceptualization) based on data input/imported into the platform.

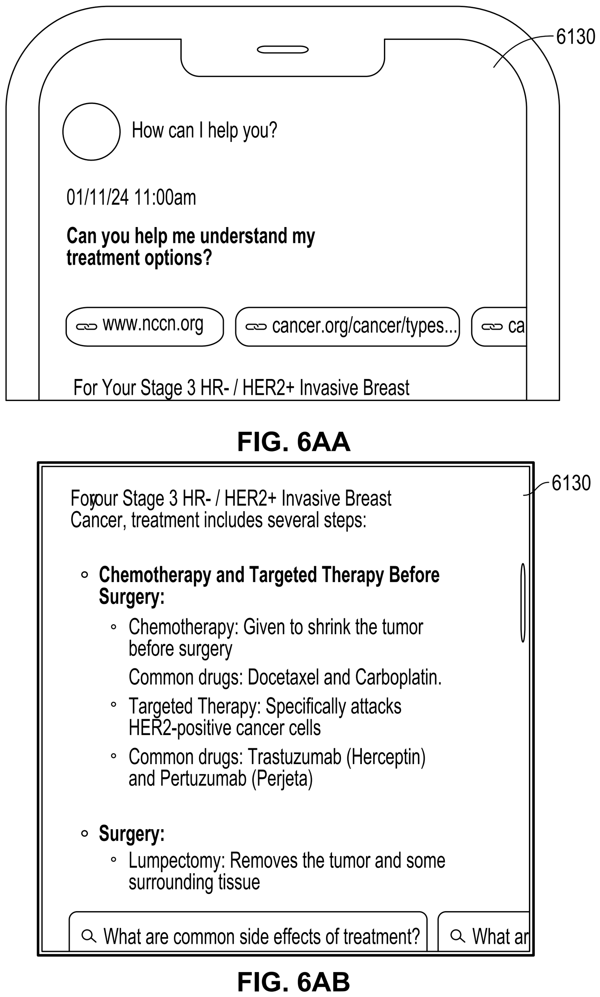

Figure 6AA illustrates a portion of a user interface 6130 indicating an example response to a user query about treatment options. For example, the user interface 6130 may be displayed in response to a user query input into the user interface element 656 in Figure 6N. The user interface 6130 in Figure 6AA includes a query response (e.g., a bulleted list) along with links to resources with additional information. Figure 6AB illustrates a lower portion of the user interface 6130 (e.g., in response to a user scrolling downward from Figure 6AA) that includes the query response and suggested follow up questions.

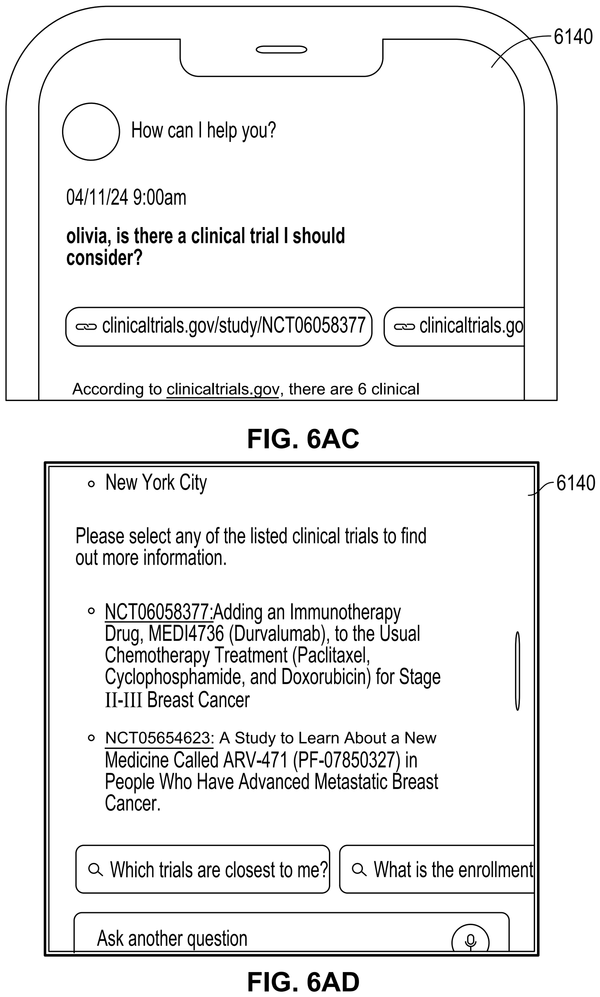

Figure 6AC illustrates a portion of a user interface 6140 indicating an example response to a user query about clinical trials. For example, the user interface 6140 may be displayed in response to a user query input into the user interface element 656 in Figure 6N. The user interface 6140 in Figure 6AC includes a query response (e.g., a bulleted list) along with links to resources with additional information. Figure 6AD illustrates a lower portion of the user interface 6140 (e.g., in response to a user scrolling downward from Figure 6AC) that includes the query response and suggested follow up questions.

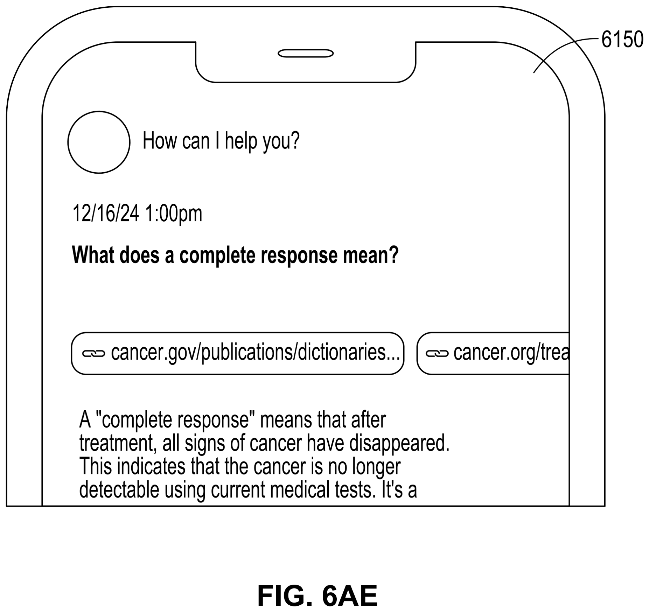

Figure 6AE illustrates user interface 6150 indicating an example response to a user query about a complete response. For example, the user interface 6150 may be displayed in response to a user query input into the user interface element 656 in Figure 6N. The user interface 6150 in Figure 6AE includes a query response along with links to resources with additional information.

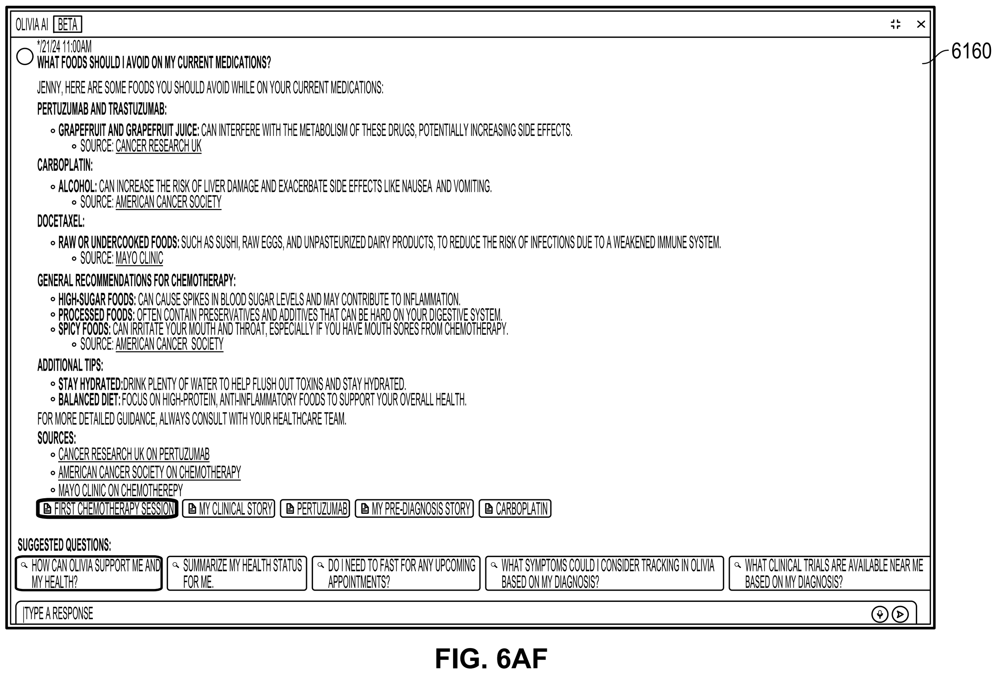

Figure 6AF illustrates user interface 6160 indicating an example response to a user query about a complete response. For example, the user interface 6160 may be displayed in response to a user query input into the user interface 600 in Figure 6A. The user interface 6160 in Figure 6AF includes a query response (e.g., including a bulleted list) along with links to resources with additional information and suggested subsequent queries.

Figures 9A-9C illustrate an example architecture 950 for an AI assistant in accordance with some embodiments. For example, the architecture 950 may be used to instantiate the bureaucrat component described previously with respect to Figure 5C. Figure 9A shows an input message component 952 coupled to a model component 954 configured to determine if the input message is a follow-up message. The outputs of the model component 954 and the input message component 952 are provided to a template component 956. The output of the template component 956 may include a question output and a follow-up question output. The follow-up question output may be provided to a template component 958 (e.g., to reformat the follow-up question information) and a model component 962 (e.g., configured to answer follow-up questions specifically). The question output may be provided to a model component 960 (e.g., configured to determine whether the input is a greetings, phatic expression, or query). Figure 9A also shows an input component 964 for obtaining user identifier information and an input component 966 for obtaining patient identifier information.

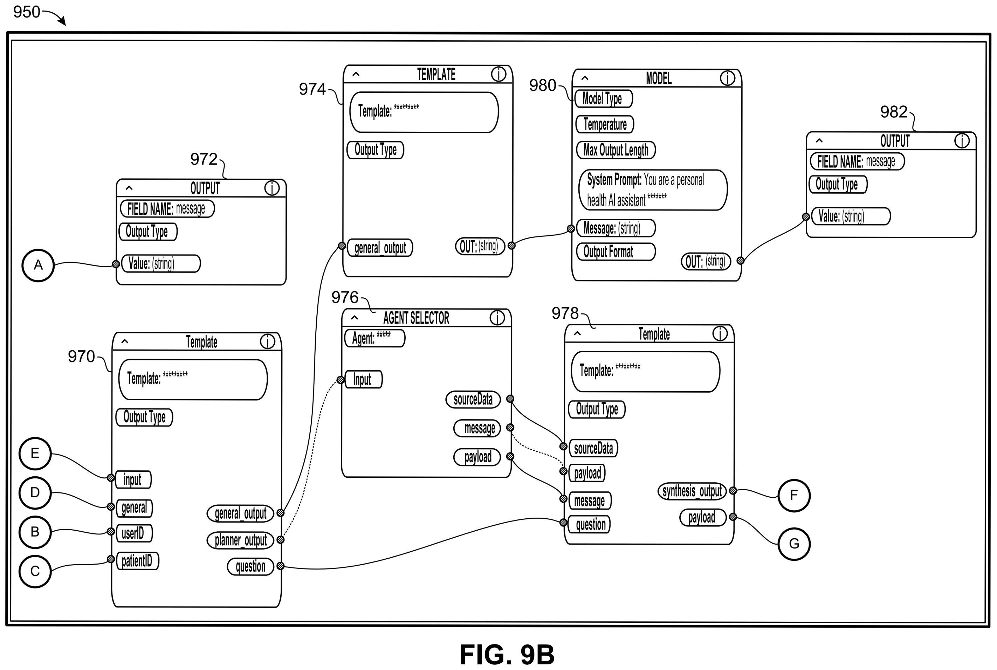

Figure 9B shows the output of the model component 962 being provided to an output component 972 configured to generate an output to be provided to the user who sent the input message. Figure 9B also shows a template component 970 receiving the outputs from the input components 964 and 966 as well as the template component 956 and the model component 960. General outputs from the template component 970 may be provided to a template component 974 for reformatting before being provided to a model component 980 (e.g., a model prompted to provide general assistance as a personal health assistant). The output from the model component 980 is provided to an output component 982. The template component 970 may provide a planner output that is used to determine an appropriate agent for answering the input message (e.g., as described previously with respect to Figure 5C). An agent selector component 976 (e.g., corresponding to the categorization orchestration in Figure 5C) may receive the planner output and provide a corresponding message with corresponding source data and payload. The outputs of the agent selector component 976 (e.g., outputs of the planner agent) may be provided to a template component 978 to generate a synthesis output and corresponding payload.

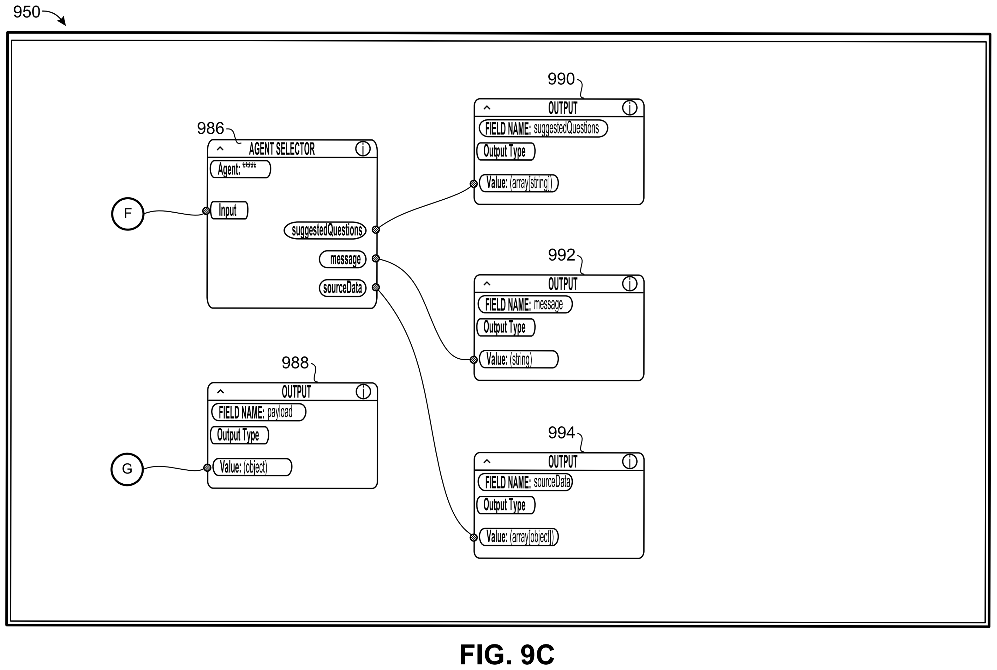

Figure 9C shows the payload output from the template component 978 being provided to an output component 988 and the synthesis output from the template component 978 being provided to an agent selector component 986 (e.g., corresponding to the synthesis orchestration in Figure 5C). The agent selector component 986 (e.g., indicating a synthesis agent) may provide a response message, suggested follow-up questions, and corresponding source data. Each output may be provided to a corresponding output component, e.g., the suggested follow-up questions to an output component 990, the message to an output component 992, and the source data to an output component 994.

Each template component may include a template for reformatting input(s) to a specified output type. For example, a template component may combine a user message with various pieces of context data. As a specific example, a query regarding whether a clinical trial is appropriate for a user may be combined with information about the user's age, location, and conditions so that the model may make an informed decision. Each model component may include a model type selected from a set of model types (e.g., model types configured to handle personal health information), a temperature setting that controls how random the output of the model may be, a maximum output length setting (e.g., 1024 characters, 512 characters, or other suitable length), and/or a prompt setting for providing a system prompt (e.g., designating rules and/or boundaries for the model) to the model. Each agent selector component may be configured to provide input information to a separate agent (orchestration) indicated in the settings of the agent selector component. The AI assistant architecture illustrated in Figures 9A-9C is merely an example, and in some embodiments, an AI assistant architecture (e.g., a bureaucrat orchestration) includes a subset or superset of the components shown in Figures 9A-9C. For example, the template component 956 may be combined with the template component 958.

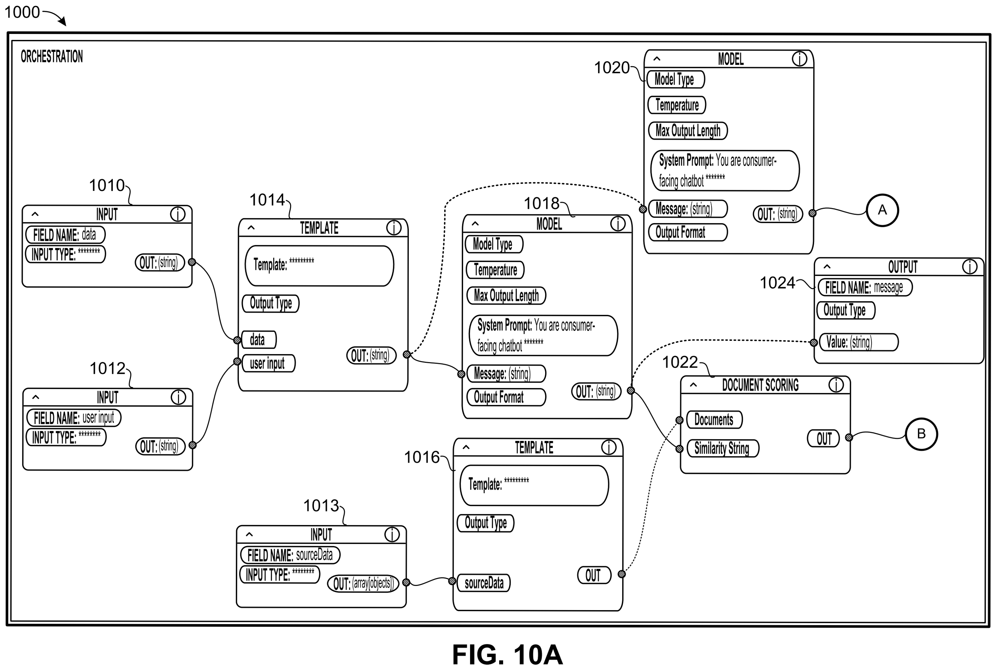

Figures 10A-10B illustrate an example architecture 1000 for a synthesis orchestration (e.g., the synthesis orchestration illustrated in Figure 5C) in accordance with some embodiments. Figure 10A shows an input component 1010 configured to obtain data (e.g., corresponding to the synthesis output of the template component 978) and an input component 1012 configured to obtain a user input (e.g., a user message/query). The outputs of the input components 1010 and 1012 may be provided to a template component 1014. The output of the template component 1014 may be provided to a model component 1018 and a model component 1020. The model component 1020 may be configured to generate a response message based on outputs from other agents/models. The model component 1020 may be configured to ensure that the response message is consistent with prior responses and/or the information about the user. An input component 1013 may be configured to obtain source data (e.g., source documents). The output of the input component 1013 may be provided to a template component 1016 and the output of the model component 1018 and the template component 1016 may be provided to a document scoring component 1022. The document scoring component 1022 may be configured to compare the output of the model component 1018 with the source documents and rank the source documents based on relevance to the output (e.g., and identify the top 10, 5, 2, or 1 documents). Additionally, the output of the model 1018 may be provided to a user via an output component 1024. The source documents may come from multiple sources, such as imported documents, external documents obtained from an approved datasource (e.g., a medical database, a research database, or a patient database), and general external documents. In some embodiments, the source data is presented (e.g., displayed/outputted) to the user along with an indication of the corresponding datasource (e.g., with a link to the document and/or datasource).

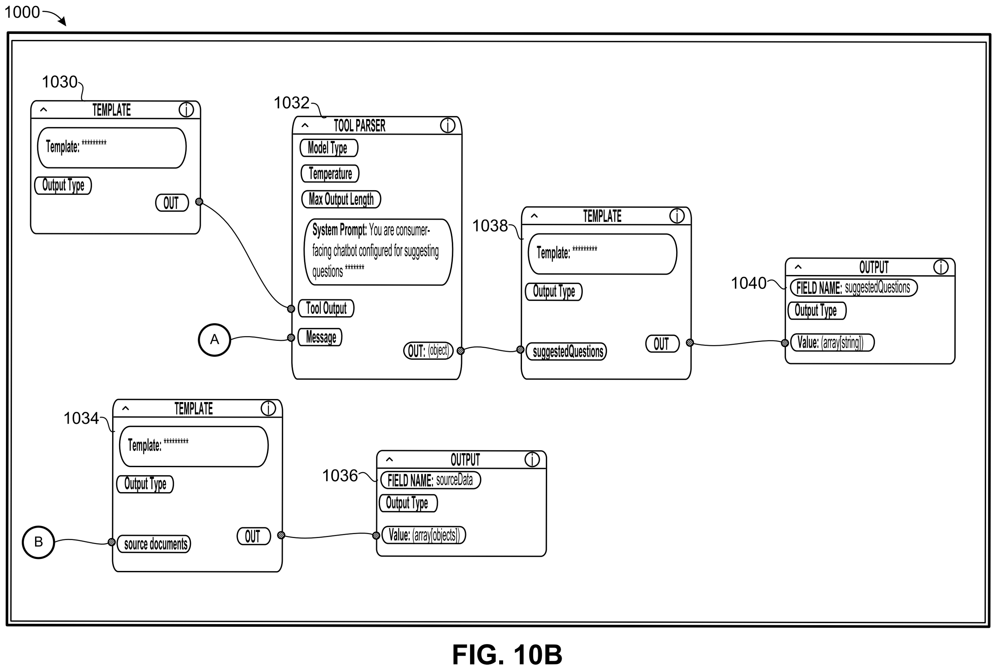

Figure 10B shows the output of the model component 1020 being provided to a tool parser component 1032 along with a tool template component 1030. The tool parsing component 1032 may be configured to identify potential follow-up questions for a user based on the user query and/or corresponding response. In some embodiments, the tool parsing component 1032 is configured to identify a set of potential follow-up questions (e.g., 10, 5, 2, or 1 most relevant follow-up questions). The output of the tool parser component 1032 may be coupled to a template component 1038, which in turn may be coupled to an output component 1040 to return suggested follow-up questions to a user. The output of the document scoring component 1022 may be coupled to a template component 1034, which in turn may be coupled to an output component 1036 to return source document information to a user.

Similar to the example architectures for the bureaucrat orchestration (Figures 9A-9C) and the synthesis orchestration (Figures 10A-10B), an architecture for a planner agent (e.g., the categorization orchestration shown in Figure 5C) may include the same types of building blocks (input components, model components, template components, agent selector components, and output components). The planner component may also include other component types, such as a HTTP call component configured to execute HTTP calls. As described previously, the planner agent may be configured to classify inputs into a set of categories, such as a user-related query, a clinic trial query, a medical research query, a medical advice query, a general medical query, a general non-medical query, a customer support query, or an action query.

In some embodiments, the planner agent is configured to determine what type of data is needed to answer the question (e.g., based on a categorization of the question). In some embodiments, when the question is not related to a preset set of categories, the planner agent is configured to respond that it is not permitted to answer the question. In some embodiments, the planner agent includes a separate flow for user-related inquiries, action inquiries, general medical inquiries, general non-medical inquires, and customer support inquiries. For example, for an action inquiry, the planner agent passes information for the action to be performed by a different agent. As another example, for general medical inquires, the planner agent passes information to a medical information agent to provide a response. As another example, the planner agent passes information for customer support inquiries to a customer support agent (e.g., an agent trained on the capabilities of the AI assistant architecture) to provide a response. As another example, for user-related inquiries, the planner agent may use the user identifier and/or the patient identifier to obtain user-specific and/or patient-specific information (e.g., with a call, such as an HTTP call, to a user database and/or a patient database) to use to generate a response to the user-related inquiry. For user-related inquiries, the planner agent may determine whether the inquiry is related to clinical trials, research, or other user-specific matters (and optionally has a different flow for each). As an example, for general inquiries about clinical trials, the planner agent may obtain context information for the user (e.g., user demographics, location, diagnoses, etc.) and provide the context information to a model/agent configured to provide clinical trials responses (e.g., a clinical trial agent). The user context information may be obtained from patient summary data for the user. For a user-specific inquiry, the planner agent may determine whether the inquiry is a question about a condition of the user (e.g., passed to a medical agent), a health tracking inquiry (e.g., passed to a health tracking agent), or a patient summary inquiry (e.g., passed to patient summary agent).

In accordance with some embodiments, Figures 9A-9C and 10A-10B correspond to a user interface in which a user may add, delete, and/or modify the connections, components, and/or configurations of the respective orchestrations. For example, an agent-builder application may include various user interface elements for causing operations to modify respective orchestrations associated with the user of the agent-builder application. In accordance with some embodiments, a user is permitted access to the agent-builder application by providing user credentials, e.g., from the client device 102 to the server system 106. The user interface may include a form-builder user interface element for interacting with (e.g., instantiating and/or configuring) an agent module (e.g., an agent module 226 or 316) in accordance with some embodiments. In some embodiments, the user interface includes global user interface elements that are present within different respective user interfaces of the agent-builder application, as described herein. For example, the user interface includes respective user interface elements for accessing different user interfaces of the agent-builder application (e.g., a user interface element for accessing a home user interface, a user interface element for accessing an agent-builder user interface, a user interface element for accessing a data viewing user interface, and a user interface element for viewing a list of task-specific orchestrations (e.g., task-specific agents) that are available to the user accessing the agent-builder application. For example, the global user interface elements may include a prompt user interface element for initiating a chat session with a respective agent module of the agent-builder application.

The user interface may include a plurality of user interface elements for modifying an orchestration (e.g., a task-specific orchestration, which may comprise an agent module and/or an agent architecture) in accordance with some embodiments. For example, the user interface may include a user interface element for naming the orchestration, and a user interface element for providing a description of the orchestration. In some embodiments, other users having access to the data associated with the orchestration may access and/or implement the orchestration by selecting it from an agent library (e.g., the agent library 250). In accordance with some embodiments, the user interface also includes a template-selector section for interacting with a plurality of user interface elements corresponding to different default orchestrations that the user can select to provide an initial node architecture 254 to the orchestration (e.g., a user interface element for creating a task-specific orchestration for interacting with a general-purpose machine-learning model, a user interface element for interacting with a task-specific orchestration that includes a machine-learning model (e.g., a general-purpose machine-learning model and/or a task-specific machine learning model) that has been trained with specific data (e.g., from a data collection that is continuously updated in real-time), and a user interface element for interacting with a task-specific orchestration that was previously created within the task-specific orchestration creator application.

The orchestration (e.g., task-specific agent) may be instantiated in accordance with the user providing an input directed to the respective user interface element for interacting with an orchestration that uses data provided by the user (e.g., a medical document, live collection data). In some embodiments, an orchestration (agent) is instantiated based on a time (e.g., at a certain date and/or time), based on an event (e.g., in response to a triggering event), and/or based on a user action. In some embodiments, the orchestration includes one or more agent-level configurations (e.g., agent attributes and/or agent settings) and one or more block-level configurations (e.g., node-level attributes and/or model settings). When the orchestration is instantiated based on the user's input, user-specific data may be provided to the orchestration. In some embodiments, based on the orchestration being instantiated by the user input directed to the user interface element, a respective machine-learning model of the task-specific agent is trained (e.g., automatically, without further input provided by the user) on clinician-specific patient data (e.g., precision medicine) based on data associated with the respective user accessing the agent-builder (orchestration) application. Figures 9A-9C and 10A-10B may correspond to a workflow editor user interface that is displayed in accordance with an orchestration being instantiated (e.g., a workflow view for a particular agent/orchestration). In some embodiments, the workflow UI is an interactive UI (e.g., a drag-and-drop representation) in which a user may select an output and then select an input to couple the input to the output (or vice versa). In accordance with some embodiments, each input and output has a corresponding data type (e.g., indicated by a color and/or label). In some embodiments, the system provides suggested building blocks (e.g., agent modules, models, tools, and/or other types of building blocks) based on user prompts. In some embodiments, the system provides a list of available building blocks, and the user may drag and drop the building blocks into the workflow representation to add them to the agent module.

As described above, the building blocks may include data building blocks, operator building blocks, and/or tool building blocks. Non-limiting examples of data building blocks include an agent listing block (e.g., obtains a listing of available agents), an input block (e.g., accepts a value from a user), a message block (e.g., returns a recent message (and optionally associated metadata) from a conversation), an output block (e.g., returns a response such as a message or document), a history block (e.g., returns a message history), a retrieval bock (e.g., retrieves data, such as documents, from a database or collection), and a semantic block (e.g., identifies semantically similar documents and/or text). Non-limiting examples of operator blocks include a storage block (e.g., configured to store bits of data and/or set common data values with various types), an array block (e.g., configured to transform (e.g., combine) inputs into arrays), a map block (e.g., configured to execute a sub-assembly for inputs in an array and return an array of results), a JSON block (e.g., configured to convert input text to an object via JSON parsing, and optionally validate against a provided schema), an XML block (configured to convert input text to an object via XML parsing, and optionally validate), a status block (e.g., configured provide information about execution status), a template block (e.g., configured to output text in accordance with a given template), and a tool block (e.g., configured to wrap an assembly consumable by another block). Non-limiting examples of tool blocks include an agent tool block (e.g., configured to interface with an agent module), a similarity block (e.g., configured to provide a similarity score for documents), a web block (e.g., configured to operate as an HTTP interface), and a model-tool interface block (e.g., configured to interface between a model and a tool (e.g., ask a model to use a tool)).

Turning now to example flows and embodiments.

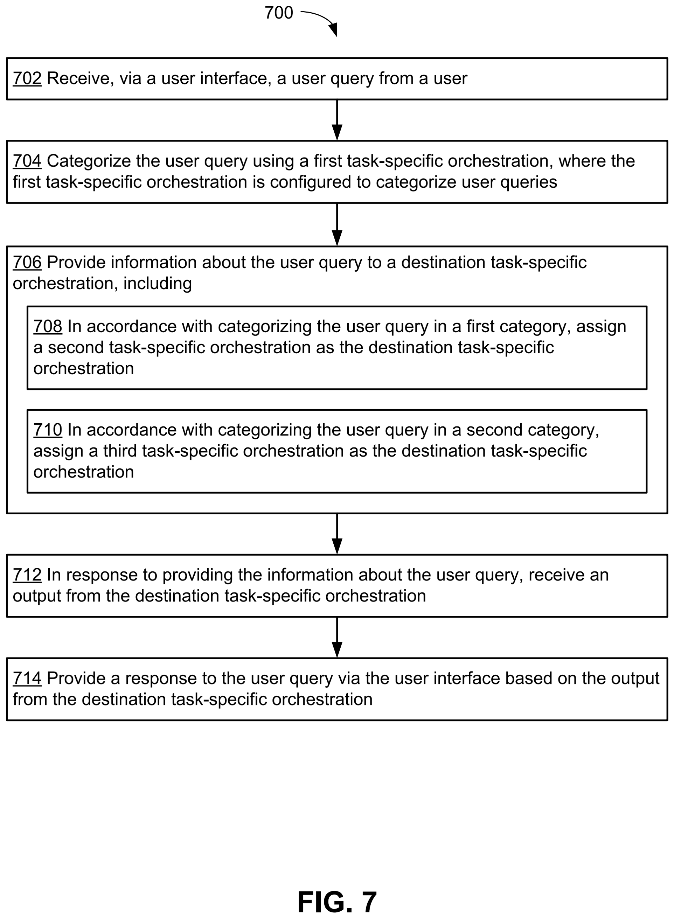

Figure 7 is a flow diagram illustrating a method 700 of responding to user queries in accordance with some embodiments. The method 700 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors. At least some of the operations shown in Figure 7 correspond to instructions stored in a computer memory or a computer-readable storage medium. In some embodiments, the computing system is the platform 100, the client device(s) 102, and/or the server system 106.

(A1) In one aspect, some embodiments include the method 700 being performed at a computing system. For example, the method 700 may be performed at an application of the client device 102 associated with the platform 100. The computing system receives (702), via a user interface (e.g., the user interface 600 or 650), a user query from a user; categorizes (704) the user query using a first task-specific orchestration (e.g., the categorization agent 552), where the first task-specific orchestration is configured to categorize user queries; provides (706) information about the user query to a destination task-specific orchestration (e.g., one of the destination agents in Figure 5B), including, in accordance with categorizing the user query in a first category, assigning (708) a second task-specific orchestration as the destination task-specific orchestration; and, in accordance with categorizing the user query in a second category, assigning (710) a third task-specific orchestration as the destination task-specific orchestration; in response to providing the information about the user query, receives (712) an output from the destination task-specific orchestration; and provides (714) a response to the user query via the user interface (e.g., the user interface 6150 in Figure 6AE) based on the output from the destination task-specific orchestration. As an example, the user query may comprise audio input and/or text input from the user.

(A2) In some embodiments of A1, the user query comprises a request to log one or more symptoms, and wherein the response to the user query indicates that the one or more symptoms have been added to a record for the user. For example, the user query may include "I'm experiencing headaches and nausea today" or "Log my pain level as 7 out of 10." In some embodiments, the symptoms are categorized by type, such as pain symptoms, gastrointestinal symptoms, neurological symptoms, or respiratory symptoms. In some embodiments, the symptoms are associated with severity levels, timestamps, and contextual information such as medication timing or activity levels. In some embodiments, the response includes a confirmation message such as "Your headache and nausea symptoms have been recorded for today" along with suggestions for related tracking, such as "Would you like to log any potential triggers?" In some embodiments, the logged symptoms are automatically integrated with existing health patterns (e.g., and trigger alerts if concerning trends are detected).

(A3) In some embodiments of A1, the user query comprises a request to log intake of medication, and wherein the response to the user query indicates that the intake of medication has been added to a record for the user. For example, the user query may include "I took my morning metformin" or "Log 10mg of lisinopril at 8 AM." In some embodiments, the medication logging includes dosage information, timing, and/or method of administration (e.g., oral, injection, topical). In some embodiments, the system automatically tracks medication adherence patterns and provides reminders for missed doses. In some embodiments, the response includes confirmation details such as "Your metformin dose has been logged for 8:15 AM today" and may include adherence statistics like "You've taken 95% of your prescribed doses this week." In some embodiments, the logged medication data is cross-referenced with symptom logs to identify potential correlations or side effects. In some embodiments, the system provides warnings for potential drug interactions when multiple medications are logged.

(A4) In some embodiments of A1, the user query comprises a request to generate a user profile for the user. For example, the user query may include "Create my health profile" or "Generate a comprehensive medical summary for me." In some embodiments, in response to the request to generate the user profile, the destination task-specific orchestration obtains health/medical information for the user from a set of one or more databases and uses the obtained information to generate the user profile. In some embodiments, data from multiple sources is compared and used to generate the profile , such as electronic health records from different healthcare providers, laboratory results from various testing facilities, and pharmacy records. In some embodiments, inconsistencies in the data are indicated to the user to review/correct, such as conflicting medication lists or duplicate diagnoses with different dates. In some embodiments, data missing from the user profile is indicated to the user so that the user may fill in the missing data, such as family medical history, allergies, or recent symptoms not captured in formal medical records. In some embodiments, the generated profile includes sections for demographics, medical history, current medications, allergies, recent test results, and/or care team information. In some embodiments, the profile is formatted for sharing with healthcare providers or for personal reference during medical appointments.

(A5) In some embodiments of A1, the user query comprises a request to update a user profile for the user, and wherein the response to the user query indicates information from the user profile. For example, the user query may include "Update my profile with my new diagnosis" or "Add my recent blood pressure readings to my profile." The response may indicate what information is in the user profile before and/or after the update requested in the user query, such as "Your profile has been updated to include Type 2 diabetes diagnosis from 12/15/2023. Your current conditions now include: hypertension, Type 2 diabetes, and seasonal allergies." In some embodiments, the update request includes specific data fields to modify, such as contact information, emergency contacts, insurance details, or medical conditions. In some embodiments, the response includes a summary of changes made and requests confirmation for updates. In some embodiments, the system maintains a version history of profile changes and allows users to revert recent modifications if desired.

(A6) In some embodiments of A1, the user query relates to a future event, and wherein the response to the user query comprises information about the future event. For example, the user query may include "What should I expect at my cardiology appointment next week?" or "Help me prepare for my MRI scan tomorrow." For example, the query relates to an upcoming medical appointment and the response indicates relevant information for the appointment, key questions, anticipated procedures, and/or recommended documents. In some embodiments, the information about the future event includes recommended tasks to perform in advance of the future event, such as fasting requirements, medication adjustments, or completing pre-appointment questionnaires. In some embodiments, the information about the future event includes information about what will likely occur during the future event, such as "During your echocardiogram, you'll lie on an examination table while a technician uses an ultrasound probe to create images of your heart." In some embodiments, the response includes logistical information such as appointment location, parking instructions, estimated duration, and what to bring. In some embodiments, the system generates personalized preparation checklists based on the specific type of appointment and the user's medical history. In some embodiments, the response includes relevant questions the user might want to ask their healthcare provider during the appointment.

(A7) In some embodiments of A1, the user query comprises a request to send information to a third party. For example, the user query may include "Send my recent lab results to Dr. Smith" or "Share my medication list with my new cardiologist." For example, the user query may include a request to send one or more documents (e.g., medical documents, such as test results and/or diagnoses) to a third party (e.g., a healthcare provider, an insurance provider, a family member, or other third party). In some embodiments, the response to the user query comprises an indication of the information, an indication of the third party, and a request for confirmation., such as "I'm ready to send your CBC results from 11/20/2023 to Dr. Sarah Johnson at Metro Cardiology. Should I proceed?" In some embodiments, the response to the user query indicates that the information has been sent to the third party, such as "Your lab results have been successfully sent to Dr. Johnson's office via secure message." In some embodiments, the request includes contact information (e.g., a phone number, email address, or mailing address) for the third party, and the contact information is used to send the information to the third party. In some embodiments, the system maintains a log of all information sharing activities for privacy and security auditing. In some embodiments, the sharing process includes encryption and secure transmission protocols to protect patient health information. In some embodiments, the system provides options for different sharing methods, such as secure email, direct EHR integration, or encrypted file transfer.

(A8) In some embodiments of A1, the user query comprises a request to generate a summary of one or more documents, and wherein the response to the user query comprises the summary. For example, the request may be a request to summarize a portion of the user's medical record, such as "Summarize my lab results from the past six months" or "Create a summary of my cardiology visits." As another example, the request may be a request to summarize medication and/or treatment documentation, such as "Summarize my current treatment plan" or "Provide an overview of my medication history." In some embodiments, the summary includes key findings, trends, and recommendations extracted from the documents. In some embodiments, the summary is generated in different formats, such as a bulleted list, a narrative summary, or a structured report (e.g., in accordance with a user preference or request). In some embodiments, the destination task-specific orchestration is configured to identify the most relevant information from multiple documents and consolidate it into a coherent summary. In some embodiments, the summary includes timestamps and source references for traceability. In some embodiments, the system generates both a brief summary and a detailed summary, allowing the user to choose the level of detail desired.

(A9) In some embodiments of A1, the user query comprises a request to schedule an appointment. For example, the user query may include "Schedule a follow-up appointment with Dr. Smith" or "Book my annual physical exam." In some embodiments, the response to the user query comprises an option to schedule an appointment, such as presenting available time slots from the healthcare provider's calendar. In some embodiments, the response to the user query comprises an indication of the appointment time and place and a request for confirmation, such as "I've found an available slot on Tuesday, March 15th at 2:00 PM at Metro Medical Center. Would you like me to book this appointment?" In some embodiments, the response to the user query comprises display of an interactive user interface to facilitate appointment scheduling, including calendar views, provider selection, and appointment type options. In some embodiments, the request is to schedule an appointment with a third party, and the response indicates availability information obtained from the third party (e.g., obtained by the destination task-specific orchestration). In some embodiments, the destination task-specific orchestration comprises a communication module configured to communicate with a third-party system to schedule the appointment. In some embodiments, the destination task-specific orchestration is configured to interact with an external portal (e.g., via an API) to obtain scheduling information and/or schedule the appointment. In some embodiments, the system automatically suggests optimal appointment times based on the user's health history and treatment schedule. In some embodiments, the system provides appointment preparation information, such as fasting requirements or documents to bring. In some embodiments, the system sends confirmation notifications and reminders to the user after scheduling the appointment.

(A10) In some embodiments of A1, the user query comprises a request to provide an insight for a set of one or more documents, and wherein the destination task-specific orchestration is configured to use health information of the user as context information for generating the insight. For example, the health information of the user may be obtained from an electronic health record for the user. For example, the user query may include "What insights can you provide about my recent blood work?" or "Analyze my imaging results in the context of my medical history." In some embodiments, the insight includes trend analysis, such as identifying patterns in lab values over time or correlations between symptoms and treatments. In some embodiments, the insight includes risk assessment based on the user's medical history and current health status. In some embodiments, the destination task-specific orchestration compares the user's data against population norms or clinical guidelines to generate personalized insights. In some embodiments, the insight includes recommendations for follow-up actions, such as scheduling additional tests or consulting with specialists. In some embodiments, the system generates insights by combining multiple data sources, such as lab results, imaging studies, and clinical notes. In some embodiments, the insight includes visualization of health trends through charts or graphs to facilitate understanding.

(A11) In some embodiments of A1, the user query comprises a request to generate a document, and wherein the response to the user query comprises the generated document. For example, the document may be a medical form, an insurance form, a letter, a summary, a cover sheet, or the like. For example, the user query may include "Generate a medical history summary for my new doctor" or "Create an insurance pre-authorization form for my upcoming procedure." In some embodiments, the request is a request to populate a document (e.g., fill in a form) and the response provides the populated document. In some embodiments, the generated document includes patient demographic information, medical history, current medications, and/or relevant test results automatically populated from the user's health profile. In some embodiments, the system generates documents in multiple formats, such as PDF, Word, or structured data formats. In some embodiments, the generated document includes digital signatures or authentication markers for official use. In some embodiments, the system generates specialized documents such as disability forms, FMLA paperwork, or medical clearance letters. In some embodiments, the document generation includes compliance checking to ensure required fields are completed and information is accurate. In some embodiments, the system provides templates for different types of medical documents that can be customized based on the user's specific needs.

(A12) In some embodiments of A1, the user query comprises a request to view health data, and wherein the response to the user query comprises indication of the health data. For example, the request may be to view a test result, and the response includes the test result information (e.g., an image of the test result document and/or a summary of the test result document). As another example, the request may be to view a medical image (e.g., an ultrasound image, an x-ray image, or the like) and the response includes the medical image. The health data may include visit summaries, scans, molecular tests, and/or DICOM images such as CT scans, MRIs, PET scans. For example, the user query may include "Show me my latest cholesterol results" or "Display my chest X-ray from last week." In some embodiments, the response includes interactive viewing capabilities, such as zooming, panning, or adjusting contrast for medical images. In some embodiments, the health data is presented with contextual information, such as normal ranges for lab values or annotations explaining medical terminology. In some embodiments, the system provides comparison views showing current results alongside historical data to identify trends. In some embodiments, the response includes data visualization tools, such as graphs or charts, to help users understand their health metrics over time. In some embodiments, the system provides filtered views of health data based on date ranges, data types, or specific conditions. In some embodiments, the health data viewing includes privacy controls that allow users to selectively share specific data elements with healthcare providers or family members.

(A13) In some embodiments of A1, the user query comprises a health update, and wherein the response to the user query comprises indication that the health update is added to a record for the user. For example, the health update may include mood information, symptom information, medication information, and the like. For example, the user query may include "Update my record to show I'm feeling anxious today" or "Add that I took my blood pressure medication this morning." In some embodiments, the response indicates that the health update is being added to an EHR for the user. In some embodiments, the response indicates that the health update is being added to a health profile for the user. In some embodiments, the health update includes quantitative data, such as vital signs, pain levels on a scale, or medication dosages. In some embodiments, the health update includes qualitative information, such as descriptions of symptoms, side effects, or general well-being. In some embodiments, the system automatically timestamps health updates and associates them with relevant medical conditions or treatment plans. In some embodiments, the response includes confirmation of the specific information that was recorded, such as "I've recorded that you experienced a headache with severity level 6 at 2:30 PM today." In some embodiments, the system provides suggestions for related information to track, such as "Would you also like to log any potential triggers for your headache?" In some embodiments, the health updates are automatically categorized and integrated with existing health patterns to identify trends or concerning changes.

(A14) In some embodiments of any of A1-A13, the first category and the second category are selected from a group comprising: a medication category, a care-coordination category, a report-generation category, a retrieval category, a health-profile category, a visit-preparation category, and a health-tracking category. For example, the medication category may include queries about drug interactions, dosage information, side effects, or medication adherence tracking. The care-coordination category may encompass queries about scheduling appointments, communicating with healthcare providers, or managing referrals between specialists. The report-generation category may include requests to create medical summaries, insurance forms, or treatment timelines. The retrieval category may involve queries to access specific test results, medical images, or historical health records. The health-profile category may include requests to update personal medical information, add new diagnoses, or modify demographic data. The visit-preparation category may encompass queries about preparing for upcoming appointments, gathering relevant documents, or formulating questions for healthcare providers. The health-tracking category may include logging symptoms, recording vital signs, or monitoring medication compliance over time.

(A15) In some embodiments of any of A1-A14, the user query is received from a patient. In some embodiments, the user query comprises a natural language input. For example, the patient may input queries such as "What are the side effects of my blood pressure medication?" or "When is my next cardiology appointment?" In some embodiments, the user query is received from a healthcare provider, such as a physician, nurse, or medical assistant. In some embodiments, the user query is received from a caregiver or family member authorized to access the patient's medical information. In some embodiments, the natural language input includes conversational phrases, medical terminology, or colloquial expressions. In some embodiments, the user query is received via voice input that is converted to text using speech recognition technology. In some embodiments, the user query includes multiple questions or requests within a single input, such as "Show me my recent lab results and schedule a follow-up appointment with Dr. Smith." In some embodiments, different portions of the query (e.g., different questions or requests) are sent to different task-specific orchestrations.

(A16) In some embodiments of any of A1-A15, the response to the user query comprises a natural language response. For example, the response to the user query may be provided in a conversational tone. In some embodiments, the natural language response is tailored to the user's level of medical knowledge, providing simplified explanations for patients and more technical details for healthcare providers. In some embodiments, the response includes empathetic language when addressing sensitive medical topics, such as "I understand this diagnosis may be concerning. Here's what you need to know..." In some embodiments, the response is formatted as a structured narrative that flows logically from general information to specific details. In some embodiments, the response includes transitional phrases and connective language to improve readability, such as "Additionally," "Furthermore," or "In contrast." In some embodiments, the response adapts its tone based on the urgency or severity of the medical information being conveyed. In some embodiments, the response includes personalized elements, such as addressing the user by name or referencing their specific medical conditions.

(A17) In some embodiments of any of A1-A16, the first task-specific orchestration comprises a first machine-learning (ML) model, and wherein the second task-specific orchestration comprises a second ML model. In some embodiments, the first and second ML models are different types of models. In some embodiments, the first and second ML models are a same type of model. For example, the first ML model may be a transformer-based language model optimized for text classification, while the second ML model may be a neural network specialized for medical image analysis. In some embodiments, the first ML model is a large language model (LLM) such as GPT-4 or PaLM-2, while the second ML model is a domain-specific model trained on medical literature. In some embodiments, both ML models are variants of the same base architecture but fine-tuned on different datasets, such as one trained on clinical trial data and another trained on patient care guidelines. In some embodiments, the first ML model operates on structured data while the second ML model processes unstructured text. In some embodiments, the ML models have different parameter counts, with the first model having millions of parameters and the second model having billions of parameters. In some embodiments, the ML models utilize different training methodologies, such as supervised learning versus reinforcement learning from human feedback.

(A18) In some embodiments of any of A1-A17, in accordance with categorizing the user query in a third category and a determination that the third category does not have a corresponding task-specific orchestration, the system generates a new task-specific orchestration and using the new task-specific orchestration as the destination task-specific orchestration. For example, if a user query relates to a newly emerging medical field such as personalized genomics and no existing orchestration is configured to handle such queries, the system may automatically create a genomics-specific orchestration. In some embodiments, the new task-specific orchestration is generated by adapting an existing orchestration template and training it on relevant domain-specific data. In some embodiments, the system identifies the closest existing orchestration and creates a hybrid orchestration that combines capabilities from multiple existing orchestrations. In some embodiments, the new orchestration is created using transfer learning techniques, where a pre-trained model is fine-tuned on data specific to the third category. In some embodiments, the system maintains a repository of orchestration building blocks that can be dynamically assembled to create new orchestrations. In some embodiments, the generation of the new orchestration triggers a notification to system administrators for review and approval before deployment. In some embodiments, the new orchestration undergoes automated testing against a validation dataset before being made available for user queries.

(A19) In some embodiments of any of A1-A18, the second task-specific orchestration comprises a clinical trials orchestration, a customer support orchestration, a medical publication orchestration, or a general query orchestration. For example, the clinical trials orchestration may be specifically trained on clinical trial databases and may include capabilities to match patient profiles with trial inclusion criteria. The customer support orchestration may be configured to handle questions about system functionality, account management, or technical troubleshooting. The medical publication orchestration may have access to medical databases and may be optimized for literature searches and evidence synthesis. The general query orchestration may serve as a fallback option for queries that don't fit into specific categories and may provide general medical information or health education content. In some embodiments, the clinical trials orchestration includes geographic filtering capabilities to identify trials available in the patient's location. In some embodiments, the medical publication orchestration includes citation analysis and impact factor weighting to prioritize high-quality research sources. In some embodiments, the customer support orchestration includes escalation pathways to human support agents for complex technical issues.

(A20) In some embodiments of any of A1-A19, the response comprises a bulleted list. For example, when a user asks about medication side effects, the response may present each side effect as a separate bullet point with associated frequency information. In some embodiments, the bulleted list is hierarchically organized with main points and sub-points, such as organizing side effects by severity level or body system affected. In some embodiments, the bulleted list includes numerical or alphabetical ordering to indicate priority or sequence. In some embodiments, the response combines bulleted lists with narrative text, where bullet points highlight key information and paragraphs provide detailed explanations. In some embodiments, the bulleted list includes interactive elements, such as expandable sections that provide additional detail when selected. In some embodiments, the bullets are replaced with icons or symbols relevant to the medical content, such as warning symbols for serious side effects or checkmarks for recommended actions. In some embodiments, the list is formatted with different indentation levels to show relationships between related concepts.

(A21) In some embodiments of any of A1-A20, the response includes one or more relevant medical disclaimers. For example, the response may include disclaimers such as "This information is for educational purposes only and should not replace professional medical advice" or "Always consult with your healthcare provider before making changes to your treatment plan." In some embodiments, the medical disclaimers are automatically selected based on the type of medical information provided, with more prominent disclaimers for medication-related advice or diagnostic information. In some embodiments, the disclaimers include emergency contact information or instructions to seek immediate medical attention for urgent symptoms. In some embodiments, the disclaimers are personalized based on the user's medical history, such as including specific warnings for patients with known allergies or chronic conditions. In some embodiments, the disclaimers include references to relevant medical guidelines or regulatory authorities, such as FDA warnings or clinical practice guidelines. In some embodiments, the disclaimers are presented in a visually distinct format, such as highlighted text boxes or warning banners, to ensure user attention. In some embodiments, the system requires user acknowledgment of critical disclaimers before displaying potentially sensitive medical information.

(A22) In some embodiments of any of A1-A21, the system analyzes the output from the destination task-specific orchestration; and identifies one or more disclaimers relevant to the output, where the response to the user query includes the one or more disclaimers. For example, if the output contains information about prescription medications, the system may automatically include disclaimers about drug interactions and the importance of consulting with a pharmacist. In some embodiments, the analysis includes natural language processing to identify medical terms, drug names, or diagnostic information that trigger specific disclaimer requirements. In some embodiments, the system maintains a database of disclaimer templates mapped to medical concepts, conditions, or treatment categories. In some embodiments, the disclaimer identification process considers the user's demographic information, such as age or pregnancy status, to include relevant warnings. In some embodiments, the system uses machine learning models trained on medical liability and safety guidelines to predict which disclaimers are most appropriate for specific content. In some embodiments, the analysis includes severity scoring to determine whether standard disclaimers are sufficient or if enhanced warnings are needed. In some embodiments, the system cross-references the output against known contraindications or safety alerts from medical databases to ensure comprehensive disclaimer coverage.

(A23) In some embodiments of any of A1-A22, the response includes one or more links to source documents and/or resources. For example, the response may include links to peer-reviewed research articles, clinical practice guidelines, or patient education materials from reputable medical organizations. In some embodiments, the links are categorized by type, such as "Research Studies," "Clinical Guidelines," or "Patient Resources," to help users identify the most relevant information for their needs. In some embodiments, the source documents include metadata such as publication date, journal impact factor, or evidence quality ratings to help users assess credibility. In some embodiments, the links direct users to specific sections or pages within larger documents, rather than generic homepage links. In some embodiments, the system provides brief summaries or abstracts of linked resources to help users determine relevance before clicking. In some embodiments, the links include both primary sources (such as original research) and secondary sources (such as systematic reviews or meta-analyses) to provide comprehensive evidence. In some embodiments, the system tracks link engagement to improve future source recommendations and identify the most valuable resources for different types of queries. In some embodiments, the links are dynamically updated to ensure they remain active and point to the most current versions of documents.

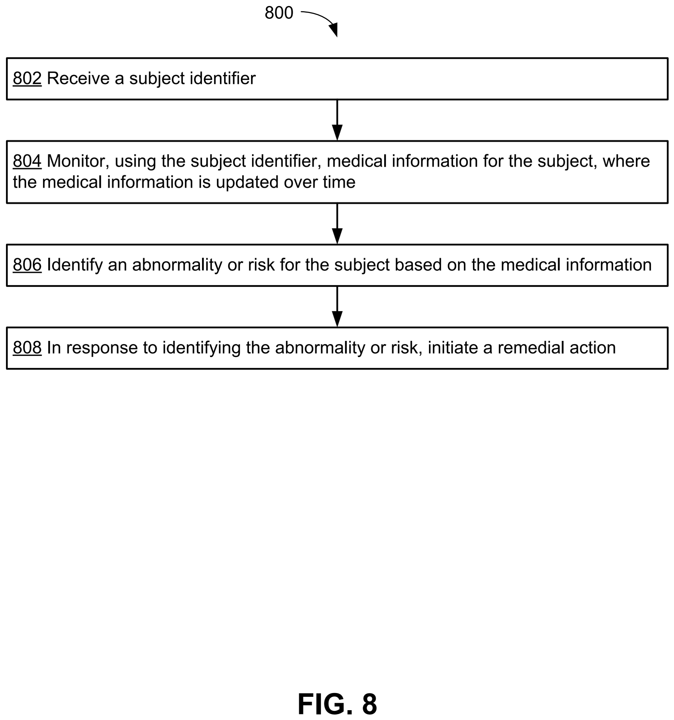

Figure 8 is a flow diagram illustrating a method 800 of identifying and responding to abnormalities and risks in accordance with some embodiments. The method 800 is performed at a computing system (e.g., a client device, server system, and/or service platform) having one or more processors (e.g., the CPUs 202 and/or 302) and memory (e.g., the memory 218 and/or 310). In some embodiments, the memory stores one or more programs configured for execution by the one or more processors. At least some of the operations shown in Figure 8 correspond to instructions stored in a computer memory or a computer-readable storage medium. In some embodiments, the computing system is the platform 100, the client device(s) 102, and/or the server system 106.

(B1) In one aspect, some embodiments include the method 800 being performed at a computing system. For example, the method 800 may be performed at an application of the client device 102 associated with the platform 100. The computing system receives (802) a subject identifier; monitors (804), using the subject identifier, medical information for the subject, where the medical information is updated over time; identifies (806) an abnormality or risk for the subject based on the medical information; and, in response to identifying the abnormality or risk, initiates (808) a remedial action. In some embodiments, the medical information is stored in one or more medical databases (e.g., the databases 400). In some embodiments, the medical information is obtained from two or more sources (e.g., databases managed by different parties).

(B2) In some embodiments of B1, initiating the remedial action comprises providing a notification to the subject, the second notification indicating the abnormality or risk. For example, the notification may be sent via email, text message, push notification through a mobile application, or automated phone call. In some embodiments, the notification includes severity indicators, such as "urgent," "moderate concern," or "routine follow-up required." In some embodiments, the notification is personalized based on the subject's communication preferences and medical history. For example, a notification about elevated blood pressure readings may include contextual information about the subject's hypertension management plan. In some embodiments, the notification includes educational content relevant to the identified abnormality, such as explanatory materials about what elevated glucose levels mean for diabetic patients. In some embodiments, the notification is delivered through multiple channels simultaneously to ensure receipt, such as both email and SMS. In some embodiments, the system tracks notification delivery and provides confirmation when the subject has acknowledged receipt of the alert.

(B3) In some embodiments of B1 or B2, initiating the remedial action comprises providing a notification to a healthcare provider, the second notification indicating the abnormality or risk. For example, the healthcare provider may be the subject's primary care physician, a specialist relevant to the identified condition, or an on-call medical professional. In some embodiments, the notification to the healthcare provider includes comprehensive context information, such as the subject's recent medical history, current medications, and trending health metrics. In some embodiments, the notification is integrated directly into the healthcare provider's electronic health record system or clinical workflow platform. For example, an alert about abnormal cardiac rhythm patterns may be automatically routed to the subject's cardiologist with attached ECG data and trend analysis. In some embodiments, the notification includes risk stratification information to help the healthcare provider prioritize their response, such as "high priority - immediate attention required" or "moderate priority - review within 24 hours." In some embodiments, the system maintains a hierarchy of healthcare providers and escalates notifications if the primary provider does not respond within a predetermined timeframe. In some embodiments, the notification includes suggested clinical actions or protocols based on established medical guidelines for the identified abnormality.

(B4) In some embodiments of any of B1-B3, initiating the remedial action comprises: identifying an action the subject can perform to address the abnormality or risk; and providing an indication of the action to the subject. For example, the action may be a test the subject can perform, a meeting/appointment the subject can schedule, and/or a medication the subject can take. In some embodiments, the identified action includes self-monitoring activities, such as taking daily blood pressure readings, monitoring blood glucose levels, or tracking symptoms in a health journal. In some embodiments, the action comprises lifestyle modifications, such as dietary changes, exercise recommendations, or stress management techniques. For example, if elevated cholesterol levels are detected, the system may recommend specific dietary modifications and suggest scheduling a consultation with a nutritionist. In some embodiments, the action includes scheduling specific types of medical appointments, such as follow-up laboratory tests, imaging studies, or specialist consultations. In some embodiments, the system provides step-by-step instructions for performing the recommended action, such as detailed guidance on how to properly measure blood pressure at home. In some embodiments, the action includes medication adherence reminders or adjustments, such as taking prescribed medications at specific times or contacting a physician about potential dosage modifications. In some embodiments, the system provides multiple alternative actions ranked by priority or effectiveness, allowing the subject to choose the most appropriate option for their circumstances. In some embodiments, the recommended actions are personalized based on the subject's medical history, current health status, and previously successful interventions.

(B5) In some embodiments of any of B1-B4, the abnormality or risk is identified using a task-specific orchestration. For example, the task-specific orchestration may include an ML model such as an LLM configured to analyze the medical information. In some embodiments, the task-specific orchestration comprises multiple specialized models, each trained on specific types of medical data, such as laboratory results, imaging data, or clinical notes. In some embodiments, the task-specific orchestration includes a cardiovascular risk assessment model that analyzes ECG patterns, blood pressure trends, and lipid profiles to identify potential cardiac abnormalities. In some embodiments, the orchestration comprises a diabetes monitoring model that evaluates glucose levels, HbA1c trends, and medication adherence patterns to detect diabetic complications. In some embodiments, the task-specific orchestration includes ensemble methods that combine outputs from multiple models to improve accuracy and reduce false positives. For example, a cancer screening orchestration may combine imaging analysis models with biomarker evaluation models and clinical history assessment models. In some embodiments, the orchestration includes temporal analysis capabilities that identify abnormal trends over time rather than just point-in-time abnormalities. In some embodiments, the task-specific orchestration is continuously updated and retrained on new medical data to improve its predictive accuracy. In some embodiments, different orchestrations are used for different medical specialties, such as oncology-specific orchestrations, endocrinology-specific orchestrations, or mental health-specific orchestrations.

(B6) In some embodiments of any of B1-B5, the medical information is monitored using a task-specific orchestration. For example, when the medical information is updated a notification may be pushed to the task-specific orchestration. As another example, the task-specific orchestration may periodically pull updates. In some embodiments, the monitoring orchestration operates in real-time, continuously analyzing incoming data streams from wearable devices, remote monitoring equipment, or electronic health record systems. In some embodiments, the monitoring frequency is adjustable based on the subject's risk profile, with high-risk patients monitored more frequently than low-risk patients. For example, a patient with unstable diabetes may have their glucose data monitored every few minutes, while a stable patient may be monitored daily. In some embodiments, the monitoring orchestration uses event-driven architecture where specific triggers, such as new laboratory results or medication changes, automatically initiate monitoring processes. In some embodiments, the orchestration employs batch processing for non-urgent monitoring tasks and real-time processing for critical health indicators. In some embodiments, the monitoring system includes data validation and quality checks to ensure the accuracy of incoming medical information before analysis. In some embodiments, the orchestration maintains historical baselines for each subject and compares new data against personalized normal ranges rather than population averages. In some embodiments, the monitoring orchestration includes failsafe mechanisms that alert system administrators if monitoring processes fail or if data feeds are interrupted. In some embodiments, the system provides different monitoring protocols for different types of medical conditions, such as intensive monitoring for acute conditions and routine monitoring for chronic conditions.

Although Figures 7 and 8 illustrates a number of logical stages in a particular order, stages which are not order dependent may be reordered and other stages may be combined or broken out. Some reordering or other groupings not specifically mentioned will be apparent to those of ordinary skill in the art, so the ordering and groupings presented herein are not exhaustive. Moreover, it should be recognized that the stages could be implemented in hardware, firmware, software, or any combination thereof.

In another aspect, some embodiments include a non-transitory computer-readable storage medium storing one or more sets of instructions for execution by control circuitry of a computing system, the one or more sets of instructions including instructions for performing one or more of the methods described herein (e.g., A1-A23 and B1-B6 above).

Various types of models and algorithms may be used with the agents and components disclosed herein. In some embodiments, a model is a supervised machine learning algorithm. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, Gradient Boosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (e.g., a deep-and-wide sample-level classifier).

In some embodiments, a model is, or includes, a neural network (e.g., a convolutional neural network and/or a residual neural network). Neural network algorithms, also known as artificial neural networks (ANNs), include convolutional and/or residual neural network algorithms (deep learning algorithms). Neural networks can be machine learning algorithms that may be trained to map an input data set to an output data set, where the neural network comprises an interconnected group of network nodes organized into multiple layers of network nodes. For example, the neural network architecture may comprise at least an input layer, one or more hidden layers, and an output layer. The neural network may comprise any total number of layers, and any number of hidden layers, where the hidden layers function as trainable feature extractors that allow mapping of a set of input data to an output value or set of output values. As used herein, a deep learning algorithm can be a neural network comprising a plurality of hidden layers, e.g., two or more hidden layers. Each layer of the neural network can comprise a number of network nodes (also sometimes referred to as neurons). A network node can receive input that comes either directly from the input data or the output of network nodes in previous layers, and perform a specific operation, e.g., a summation operation. In some embodiments, a connection from an input to a network node is associated with a parameter (e.g., a weight and/or weighting factor). In some embodiments, a network node sums up the products of all pairs of inputs, xᵢ, and their associated parameters. In some embodiments, the weighted sum is offset with a bias, b. In some embodiments, the output of a network node is gated using a threshold or activation function, f, which may be a linear or non-linear function. The activation function may be, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

The weighting factors, bias values, and threshold values, or other computational parameters of the neural network, may be "taught" or "learned" in a training phase using one or more sets of training data. For example, the parameters may be trained using the input data from a training data set and a gradient descent or backward propagation method so that the output value(s) that the ANN computes are consistent with the examples included in the training data set. The parameters may be obtained from a back propagation neural network training process.

As an example, a variety of neural networks may be suitable for use in analyzing an image of an eye of a subject. Examples can include, but are not limited to, feedforward neural networks, radial basis function networks, recurrent neural networks, residual neural networks, convolutional neural networks, residual convolutional neural networks, and the like, or any combination thereof. In some embodiments, a machine-learning model uses a pre-trained and/or transfer-learned ANN or deep learning architecture. Convolutional and/or residual neural networks can be used for analyzing an image of a subject in accordance with the present disclosure. Some embodiments use generative models, such as generative adversarial networks (GANs) and hidden Markov models. In a GAN, two neural networks compete against each other, with one generating samples and the other evaluating whether they are real or generated. A hidden Markov model is a generative model that has been successful in various sequence labeling tasks such as chunking, named entity recognition, POS tagging, and speech recognition.

A deep neural network model may include an input layer, a plurality of individually parameterized (e.g., weighted) convolutional layers, and an output scorer. The parameters (e.g., weights) of each of the convolutional layers as well as the input layer contribute to the plurality of parameters (e.g., weights) associated with the deep neural network model. In some embodiments, at least 100 parameters, at least 1000 parameters, at least 2000 parameters or at least 5000 parameters are associated with the deep neural network model. As such, deep neural network models require a computer to be used because they cannot be mentally solved. In other words, given an input to the model, the model output needs to be determined using a computer rather than mentally in such embodiments. See, for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference.

Neural network algorithms, including convolutional neural network algorithms, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety. Additional example neural networks suitable for use as models are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety.

In some embodiments, a model is, or includes, a support vector machine (SVM). SVM algorithms suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space can correspond to a non-linear decision boundary in the input space. In some embodiments, the plurality of parameters (e.g., weights) associated with the SVM define the hyper-plane. In some embodiments, the hyper-plane is defined by at least 10, at least 20, at least 50, or at least 100 parameters and the SVM model requires a computer to calculate because it cannot be mentally solved.

In some embodiments, a model is, or includes, a Naive Bayes algorithm. Naïve Bayes models suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference. A Naive Bayes model is any model in a family of "probabilistic models" based on applying Bayes' theorem with strong (naïve) independence assumptions between the features. In some embodiments, they are coupled with Kernel density estimation. See, for example, Hastie et al., 2001, The elements of statistical learning : data mining, inference, and prediction, eds. Tibshirani and Friedman, Springer, New York, which is hereby incorporated by reference.

In some embodiments, a model is, or includes, a Boltzmann machine. A Boltzmann machine comprises a set of binary units that are connected through weighted connections. Boltzmann Machines may use directionless unsupervised generative deep learning network for recommended systems.

In some embodiments, a model is, or includes, a nearest neighbor algorithm. Nearest neighbor models can be memory-based and include no model to be fit. For nearest neighbors, given a query point x0 (a test subject), the k training points x(r), r, ... , k (here the training subjects) closest in distance to x0 are identified and then the point x0 is classified using the k nearest neighbors. Here, the distance to these neighbors is a function of the abundance values of the discriminating gene set. In some embodiments, Euclidean distance in feature space is used to determine distance as Typically, when the nearest neighbor algorithm is used, the abundance data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York, each of which is hereby incorporated by reference.

As an example, a k-nearest neighbor model is a non-parametric machine learning method in which the input includes the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. See, Duda et al., 2001, Pattern Classification, Second Edition, John Wiley & Sons, which is hereby incorporated by reference. In some embodiments, the number of distance calculations needed to solve the k-nearest neighbor model is such that a computer is used to solve the model for a given input because it cannot be mentally performed.

In some embodiments, a model is, or includes, a decision tree. Decision trees suitable for use as models are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety. In some embodiments, the decision tree model includes at least 10, at least 20, at least 50, or at least 100 parameters (e.g., weights and/or decisions) and requires a computer to calculate because it cannot be mentally solved.

In some embodiments, a model uses a regression algorithm. A regression algorithm can be any type of regression. For example, the regression algorithm may be logistic regression. In some embodiments, the regression algorithm is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multicategory responses is used as the model. Logistic regression algorithms are disclosed in Agresti, An Introduction to Categorical Data Analysis, 1996, Chapter 5, pp. 103-144, John Wiley & Son, New York, which is hereby incorporated by reference. In some embodiments, the model makes use of a regression model disclosed in Hastie et al., 2001, The Elements of Statistical Learning, Springer-Verlag, New York. In some embodiments, the logistic regression model includes at least 10, at least 20, at least 50, at least 100, or at least 1000 parameters (e.g., weights) and requires a computer to calculate because it cannot be mentally solved.

Linear discriminant analysis (LDA), normal discriminant analysis (NDA), or discriminant function analysis can be a generalization of Fisher's linear discriminant, a method used in statistics, pattern recognition, and machine learning to find a linear combination of features that characterizes or separates two or more classes of objects or events. The resulting combination can be used as a model (e.g., a linear model) in some embodiments of the present disclosure.

In some embodiments, a model is a mixture model, such as that described in McLachlan et al., Bioinformatics 18(3):413-422, 2002. In some embodiments, in particular, those embodiments including a temporal component, a model is a hidden Markov model such as described by Schliep et al., 2003, Bioinformatics 19(1):i255-i263.

In some embodiments, a model is an unsupervised clustering model. In some embodiments, a model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety. The clustering problem can be described as one of finding natural groupings in a dataset. To identify natural groupings, two issues can be addressed. First, a way to measure similarity (or dissimilarity) between two samples can be determined. This metric (e.g., similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in the training set. If distance is a good measure of similarity, then the distance between reference entities in the same cluster can be significantly less than the distance between the reference entities in different clusters. However, clustering may not use a distance metric. For example, a nonmetric similarity function s(x, x') can be used to compare two vectors x and x'. s(x, x') can be a symmetric function whose value is large when x and x' are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering (e.g., with no preconceived number of clusters and/or no predetermination of cluster assignments).

In some embodiments, an ensemble (e.g., two or more) of models is used. In some embodiments, a boosting technique such as AdaBoost is used in conjunction with many other types of learning algorithms to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, etc. In some embodiments, the plurality of outputs is combined using a voting method. In some embodiments, a respective model in the ensemble of models is weighted or unweighted.

In some embodiments, a model is a reinforcement learning model. In some embodiments, the reinforcement learning system comprises four main elements - an agent, a policy, a reward signal, and a value function, where the behavior of the agent is defined in terms of the policy. In some embodiments, the reinforcement learning system comprises a learning algorithm. In some implementations, the learning algorithm is an on-policy learning algorithm or an off-policy learning algorithm. On-Policy learning algorithms evaluate and improve the same policy which is being used to select the agent's actions. Off-Policy learning algorithms evaluate and improve policies that are different from the policy being used for action selection. Reinforcement learning is further described, for example, in Sutton RS, Barto AG, "Reinforcement learning: an introduction," IEEE Transactions on Neural Networks. 1998;9(5):1054-1054, which is hereby incorporated herein by reference in its entirety.

In some embodiments, a model is, or includes, an autoencoder. An autoencoder is a type of generative model used for unsupervised learning that learns a latent representation of the image and uses that to reconstruct the image. The autoencoder may be a variational autoencoder (VAE) that learns to generate new data samples that are similar to a training dataset.

In some embodiments, a model is, or includes, a transformer model. As described previously, a transformer model is a neural network that learns context and thus meaning by tracking relationships in sequential data like the words in this sentence. Transformer models are used to generate images and audio as well as text.

In some embodiments, a model is, or includes, a diffusion model. A diffusion model generates data points that are similar to the data points on which the model has been trained. In some embodiments, a model is, or includes, a probabilistic generative model, such as a Bayesian network in which the joint distribution between all of the model variables can be expressed as a function of their parents.

As used herein, the term "instruction" refers to an order given to a computer processor by a computer program. On a digital computer, in some embodiments, each instruction is a sequence of 0's and 1's that describes a physical operation the computer is to perform. Such instructions can include data transfer instructions and data manipulation instructions. In some embodiments, each instruction is a type of instruction in an instruction set that is recognized by a particular processor type used to carry out the instructions. Examples of instruction sets include, but are not limited to, Reduced Instruction Set Computer (RISC), Complex Instruction Set Computer (CISC), Minimal Instruction Set Computers (MISC), Very Long Instruction Word (VLIW), Explicitly Parallel Instruction Computing (EPIC), and One Instruction Set Computer (OISC).

As used herein, the term "parameter" refers to any coefficient or, similarly, any value of an internal or external element (e.g., a weight and/or a hyperparameter) in an algorithm, model, regressor, and/or classifier that can affect (e.g., modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (e.g., a feature) to an algorithm, model, regressor, and/or classifier. As a nonlimiting example, in some embodiments, a parameter is used to increase or decrease the influence of a node (e.g., of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (e.g., by error minimization and/or backpropagation methods). In some embodiments, an algorithm, model, regressor, and/or classifier of the present disclosure includes a plurality of parameters. As such, the algorithms, models, regressors, and/or classifiers of the present disclosure cannot be mentally performed. In some embodiments, the algorithms, models, regressors, and/or classifier of the present disclosure operate in a k-dimensional space, where k is a positive integer of 5 or greater (e.g., 5, 6, 7, 8, 9, 10, etc.). As such, the algorithms, models, regressors, and/or classifiers of the present disclosure cannot be mentally performed.

In some embodiments, the methods described herein include inputting information into a model comprising a plurality of parameters, where the model applies the plurality parameters to the information through a plurality of instructions to generate an output from the model.

In some embodiments, an algorithm, model, regressor, and/or classifier of the present disclosure comprises a plurality of parameters. In some embodiments the plurality of parameters is n parameters, where: n ≥ 2; n ≥ 5; n ? 10; n ≥ 25; n ≥ 40; n ≥ 50; n ≥ 75; n ≥ 100; n ≥ 125; n ≥ 150; n ≥ 200; n ≥ 225; n ≥ 250; n ≥ 350; n ≥ 500; n ≥ 600; n ≥ 750; n ≥ 1,000; n ≥ 2,000; n ≥ 4,000; n ≥ 5,000; n ≥ 7,500; n ≥ 10,000; n ≥ 20,000; n ≥ 40,000; n ≥ 75,000; n ≥ 100,000; n ≥ 200,000; n ≥ 500,000, n ≥ 1 x 106, n ≥ 5 x 106, or n ≥ 1 x 107. In some embodiments n is between 10,000 and 1 x 107, between 100,000 and 5 x 106, or between 500,000 and 1 x 106. In some embodiments, the plurality of parameters is at least 1000 parameters, at least 5000 parameters, at least 10,000 parameters is at least 50,000 parameters, at least 100,000 parameters, at least 250,000 parameters, at least 500,000 parameters, at least 1 million parameters, at least 5 million parameters, at least 10 million parameters, at least 25 million parameters, at least 50 million parameters, at least 100 million parameters, at least 250 million parameters, at least 500 million parameters, at least 1 billion parameters, or more parameters.

In some embodiments, the plurality of instructions is at least 1000 instructions, at least 5000 instructions, at least 10,000 instructions is at least 50,000 instructions, at least 100,000 instructions, at least 250,000 instructions, at least 500,000 instructions, at least 1 million instructions, at least 5 million instructions, at least 10 million instructions, at least 25 million instructions, at least 50 million instructions, at least 100 million instructions, at least 250 million instructions, at least 500 million instructions, at least 1 billion instructions, or more instructions.

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "set" refers to a group of one or more objects. As used herein, the terms "request," "prompt," and "query" are used interchangeable unless expressly stated otherwise. As used herein, the term "model" refers to a machine learning model or algorithm. In some embodiments, the model is a task-specific model (e.g., a task-specific machine-learning model). As used herein, the term "task-specific" refers to a component that is specifically configured to perform a single task or a subset of tasks (e.g., a single class of tasks). In some embodiments, the model is an unsupervised learning algorithm. One example of an unsupervised learning algorithm is cluster analysis.

As used herein, the term "if" can be construed to mean "when" or "upon" or "in response to determining" or "in accordance with a determination" or "in response to detecting" that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for purposes of explanation, has been described with reference to specific embodiments. However, the illustrative discussions above are not intended to be exhaustive or limit the claims to the precise forms disclosed. Many modifications and variations are possible in view of the above teachings. The embodiments were chosen and described in order to best explain principles of operation and practical applications, to thereby enable others skilled in the art.
The following items constitute embodiments of the invention:
1. A method of routing queries, comprising:
   receiving, via a user interface of a computing system, a user query from a user;
   automatically categorizing the user query using a first task-specific orchestration executed by one or more processors, wherein the first task-specific orchestration comprises a first machine-learning (ML) model configured to categorize user queries;
   providing information about the user query to a destination task-specific orchestration selected from a plurality of task-specific orchestration, including:
      in accordance with categorizing the user query in a first category, automatically assigning a second task-specific orchestration as the destination task-specific orchestration; and
      in accordance with categorizing the user query in a second category, automatically assigning a third task-specific orchestration as the destination task-specific orchestration;
   in response to providing the information about the user query, receiving an output from the destination task-specific orchestration, wherein the output is generated by processing the user query using an ML model specific to the first category or the second category; and
   automatically generating and providing a response to the user query via the user interface based on the output from the destination task-specific orchestration.
2. The method of item 1, wherein the first category and the second category are selected from a group comprising: a medication category, a care-coordination category, a report-generation category, a retrieval category, a health-profile category, a visit-preparation category, and a health-tracking category.
3. The method of item 1, wherein the user query is received from a patient.
4. The method of item 1, wherein the response to the user query comprises a natural language response.
5. The method of item 1, wherein the second task-specific orchestration comprises a second ML model.
6. The method of item 1, further comprising, in accordance with categorizing the user query in a third category and a determination that the third category does not have a corresponding task-specific orchestration, generating a new task-specific orchestration and using the new task-specific orchestration as the destination task-specific orchestration.
7. The method of item 1, wherein the second task-specific orchestration comprises a clinical trials orchestration, a customer support orchestration, a medical publication orchestration, or a general query orchestration.
8. The method of item 1, wherein the response comprises a bulleted list.
9. The method of item 1, wherein the response includes one or more relevant medical disclaimers.
10. The method of item 1, further comprising:
   analyzing the output from the destination task-specific orchestration; and
   identifying one or more disclaimers relevant to the output, wherein the response to the user query includes the one or more disclaimers.
11. The method of item 1, wherein the response includes one or more links to source documents and/or resources.
12. The method of item 1, further comprising:
   receiving a subject identifier;
   monitoring, using the subject identifier, medical information for a subject, wherein the medical information is updated over time;
   identifying an abnormality or risk for the subject based on the medical information; and
   in response to identifying the abnormality or risk, initiating a remedial action.
13. The method of item 12, wherein initiating the remedial action comprises providing a notification to the subject, the notification indicating the abnormality or risk.
14. The method of item 12, wherein initiating the remedial action comprises providing a notification to a healthcare provider, the notification indicating the abnormality or risk.
15. The method of item 12, wherein initiating the remedial action comprises:
   identifying an action the subject can perform to address the abnormality or risk; and
   providing an indication of the action to the subject.
16. The method of item 12, wherein the abnormality or risk is identified using a task-specific orchestration.
17. The method of item 12, wherein the medical information is monitored using a task-specific orchestration.
18. An artificial intelligence (AI) assistant system, comprising:
   a first orchestration configured to interface with a user and distinguish answers that require context information from answers that do not require the context information;
   a second orchestration configured to categorize the answers that require context information and provide the respective answers to a task-specific orchestration associated with the categorization; and
   a third orchestration configured to consolidate and evaluate outputs from other orchestrations and generate a response for the user with an appropriate tone.
19. The AI assistant system of item 18, wherein each orchestration is composed of one or more machine-learning models.
20. The Al assistant system of item 19, wherein each orchestration is further composed of one or more of: a set of input blocks, a set of template blocks, and a set of output blocks.

## Claims

1. A method of routing queries, comprising:
receiving, via a user interface of a computing system, a user query from a user;
automatically categorizing the user query using a first task-specific orchestration executed by one or more processors, wherein the first task-specific orchestration comprises a first machine-learning (ML) model configured to categorize user queries;
providing information about the user query to a destination task-specific orchestration selected from a plurality of task-specific orchestration, including:
in accordance with categorizing the user query in a first category, automatically assigning a second task-specific orchestration as the destination task-specific orchestration; and
in accordance with categorizing the user query in a second category, automatically assigning a third task-specific orchestration as the destination task-specific orchestration;
in response to providing the information about the user query, receiving an output from the destination task-specific orchestration, wherein the output is generated by processing the user query using an ML model specific to the first category or the second category; and
automatically generating and providing a response to the user query via the user interface based on the output from the destination task-specific orchestration.

2. The method of claim 1, wherein the first category and the second category are selected from a group comprising: a medication category, a care-coordination category, a report-generation category, a retrieval category, a health-profile category, a visit-preparation category, and a health-tracking category.

3. The method of claim 1, wherein the second task-specific orchestration comprises a second ML model.

4. The method of claim 1, further comprising, in accordance with categorizing the user query in a third category and a determination that the third category does not have a corresponding task-specific orchestration, generating a new task-specific orchestration and using the new task-specific orchestration as the destination task-specific orchestration.

5. The method of claim 1, wherein the second task-specific orchestration comprises a clinical trials orchestration, a customer support orchestration, a medical publication orchestration, or a general query orchestration.

6. The method of claim 1, further comprising:
analyzing the output from the destination task-specific orchestration; and
identifying one or more disclaimers relevant to the output, wherein the response to the user query includes the one or more disclaimers.

7. The method of claim 1, wherein the response includes one or more links to source documents and/or resources.

8. The method of claim 1, further comprising:
receiving a subject identifier;
monitoring, using the subject identifier, medical information for a subject, wherein the medical information is updated over time;
identifying an abnormality or risk for the subject based on the medical information; and
in response to identifying the abnormality or risk, initiating a remedial action.

9. The method of claim 8, wherein initiating the remedial action comprises providing a notification to the subject, the notification indicating the abnormality or risk.

10. The method of claim 8, wherein initiating the remedial action comprises providing a notification to a healthcare provider, the notification indicating the abnormality or risk.

11. The method of claim 8, wherein initiating the remedial action comprises:
identifying an action the subject can perform to address the abnormality or risk; and
providing an indication of the action to the subject.

12. The method of claim 8, wherein the abnormality or risk is identified using a task-specific orchestration.

13. The method of claim 8, wherein the medical information is monitored using a task-specific orchestration.

14. A computing system, comprising:
one or more processors;
memory; and
one or more programs, wherein the one or more programs are stored in the memory and configured to be executed by the one or more processors, the one or more programs including instructions to perform or cause performance of the methods of any one of claims 1-13.

15. A computer-readable storage medium including executable instructions that, when executed by one or more processors, cause the one or more processors to perform or cause performance of the methods of any one of claims 1-13.
